(19) Europäisches Patentamt
European Patent Office
Office européen des brevets

(11) EP 1 362 848 A1

(12) **EUROPÄISCHE PATENTANMELDUNG**

(43) Veröffentlichungstag: **19.11.2003 Patentblatt 2003/47**

(51) Int Cl.[7]: **C07C 401/00**

(21) Anmeldenummer: **03090212.6**

(22) Anmeldetag: **24.07.2000**

(84) Benannte Vertragsstaaten:
**AT BE CH CY DE DK ES FI FR GB GR IE IT LI LU MC NL PT SE**
Benannte Erstreckungsstaaten:
**LT LV RO SI**

(30) Priorität: **23.07.1999 DE 19935771**

(62) Dokumentnummer(n) der früheren Anmeldung(en) nach Art. 76 EPÜ:
**00962278.8 / 1 210 327**

(71) Anmelder: **Schering Aktiengesellschaft 13353 Berlin (DE)**

(72) Erfinder:
- **Steinmeyer, Andreas 13581 Berlin (DE)**
- **Schwarz, Katica 10585 Berlin (DE)**
- **Giesen, Claudia 13587 Berlin (DE)**
- **Haberey, Martin 12169 Berlin (DE)**
- **Fähnrich, Marianne 10997 Berlin (DE)**

Bemerkungen:
Diese Anmeldung ist am 14 - 07 - 2003 als Teilanmeldung zu der unter INID-Kode 62 erwähnten Anmeldung eingereicht worden.

(54) **Verfahren zur Herstellung von Vitamin D Derivaten**

(57) Die Erfindung betrifft ein Verfahren zur Herstellung von Vitamin D-Derivaten der allgemeinen Formel IIa

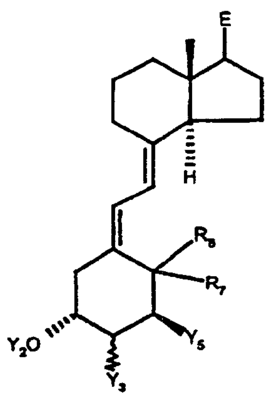

**IIa**

worin
E eine beliebige Seitenkette bedeutet,
$R_7$, $R_8$, $Y_2$, $Y_3$ und $Y_5$ die in der Beschreibung angegebenen Bedenutungen haben.

EP 1 362 848 A1

## Beschreibung

Die Erfindung betrifft ein Verfahren zur Herstellung von Vitamin D Derivaten.

## Stand der Technik

Die natürlichen Vitamine $D_2$ und $D_3$ sind an sich biologisch inaktiv und werden erst nach Hydroxylierung am C-Atom 25 in der Leber und am C-Atom 1 in der Niere in biologisch aktive Metaboliten [$1\alpha$,25-Dihydroxyvitamin $D_3$ (Calcitriol) bzw. -$D_2$] umgewandelt. Die Wirkung der aktiven Metaboliten besteht in der Regulation der Calciumund Phosphatkonzentration im Serum; sie wirken einem Absinken der Calciumkonzentration im Serum entgegen, indem sie die Calciumabsorption im Darm erhöhen und unter bestimmten Umständen die Calciummobilisation aus dem Knochen fördern. Abbildung 1 zeigt die Struktur einiger bekannter Vitamin D-Derivate:

| | |
|---|---|
| | **Ergocalciferol**: $R^a$=$R^b$=H, $R^c$=$CH_3$,<br>Doppelbindung C-22/23<br>**Vitamin $D_2$**<br><br>**Cholecalciferol:** $R^a$=$R^b$=$R^c$=H,<br>**Vitamin $D_3$**<br><br>**25-Hydroxycholecalciferol:** $R^a$=$R^c$=H, $R^b$=OH,<br>**1a-Hydroxycholecalciferol:** $R^a$=OH, $R^b$=,$R^c$=H, |

| | |
|---|---|
| 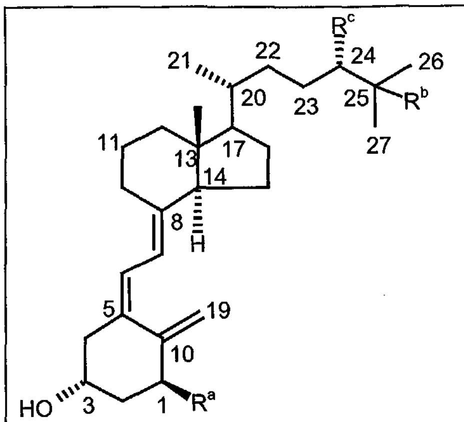 | **1a, 25-Dihydroxycholecalciferol:** $R^a$=$R^b$=OH, $R^c$=H |

Abbildung I

Neben ihrer ausgeprägten Wirkung auf den Calcium- und Phosphatstoffwechsel besitzen die aktiven Metaboliten von Vitamin $D_2$ und Vitamin $D_3$ und seine synthetischen Abkömmlinge eine proliferationshemmende und differenzierungsstimulierende Wirkung auf Tumorzellen und normale Zellen, wie zum Beispiel Hautzellen. Weiterhin wurde eine ausgeprägte Wirkung auf Zellen des Immunsystems (Hemmung der Proliferation und Interleukin-2-Synthese von Lymphocyten, Steigerung der Cytotoxizität und Phagocytose in vitro von Monocyten) gefunden, die sich in einer immunmodulatorischen Wirkung äußert. Schließlich wird infolge einer fördernden Wirkung auf knochenbildende Zellen eine vermehrte Knochenbildung bei normalen und osteoporotischen Ratten gefunden [R. Bouillon et al. "Short term course of 1,25-$(OH)_2D_3$ stimulates osteoblasts but not osteodasts" . *Calc. Tissue Int.* **49,** 168 (1991)] Alle Wirkungen werden durch Bindung an den Vitamin D-Rezeptor vermittelt. Infolge der Bindung wird die Aktivität von spezifischen Genen reguliert.

Bei Anwendung der biologisch aktiven Metaboliten von Vitamin $D_2$ und $D_3$ wird eine toxische Wirkung auf den Calciumstoffwechsel hervorgerufen (Hypercalcämie).

Durch strukturelle Manipulationen der Seitenkette können therapeutisch nutzbare Wirkqualitäten von der unerwünschten hypercalcämischen Aktivität abgetrennt werden. Eine geeignete Strukturvariante ist die Einführung einer 24-Hydroxy-Gruppe.

In 24-Stellung hydroxylierte 1α-Cholecalciferole gehen bereits aus der DE 25 26 981 hervor. Sie besitzen eine geringere Toxizität als das entsprechende nicht-hydroxylierte 1α-Cholecalciferol. Darüberhinaus sind 24-Hydroxy-Derivate in folgenden Patentanmeldungen beschrieben:

DE 39 33 034, DE 40 03 854 , DE 40 34 730 (?), EP 0 421 561 , EP 0 441 467 , WO 87/00834 , WO 91/12238 .

Schließlich werden in der WO 94/07853 an C-24 hydroxylierte 25-Carbonsäure-Derivate von Calcitriol beschrieben, die ein günstigeres Wirkspektrum als Calcitriol aufweisen. Entsprechendes gilt auch für neue Vitamin D-Derivate mit anderen Substituenten an C-25 (WO 97/00242). Während die Fähigkeit zur Auslösung einer Hypercalcämie deutlich abgeschwächt ist, bleiben die proliferationshemmenden und differenzierungsstimuliernden Wirkungen erhalten. In der Regel führt die Einführung der 24-Hydroxylgruppe jedoch zur metabolischen Destabilisierung der Derivate. Aus diesem Grunde sind diese Verbindungen nur bedingt zur systemischen Applikation geeignet.

Es besteht daher Bedarf an neuen Vitamin D-Derivaten, die ein ähnlich günstiges oder verbessertes Wirkspektrum, wie die im Stand der Technik beschriebenen Verbindungen (insbesondere WO 94/07853 und WO 97/00242) aufweisen, aber durch eine höhere metabolische Stabilität besser zur systemischen Applikation geeignet sind.

Der vorliegenden Patentanmeldung liegt daher die Aufgabe zugrunde, derartige Vitamin D-Derivate zur Verfügung zu stellen. Die Lösung dieser Aufgabe erfolgt durch die in den Patentansprüchen offenbarten Verbindungen.

Die vorliegende Erfindung betrifft daher Vitamin D-Derivate der allgemeinen Formel I,

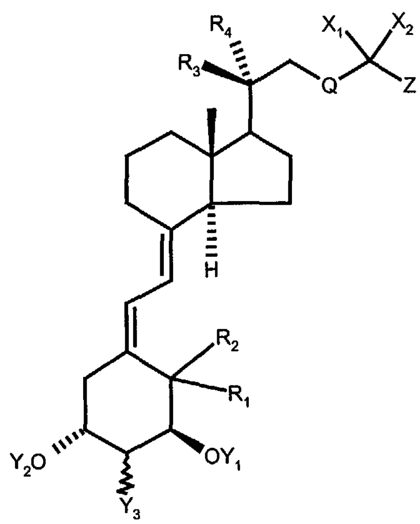

I

worin

$Y_1$ und $Y_2$ unabhängig voneinander je ein Wasserstoffatom oder eine Gruppe -C(O)$R_5$ bedeuten,

und $Y_3$ ein Wasserstoffatom oder eine Hydroxygruppe, ein Halogenatom, eine Gruppe -OC(O)$R_5$ oder eine -$OR_5$-Gruppe bedeutet, wobei

$R_5$ für einen aromatischen Rest mit 5 bis 12-C-Atomen steht, oder für einen aliphatischen, geradkettigen oder verzweigten, gesättigten oder ungesättigten $C_1$-$C_{12}$ Alkylrest, der gegebenenfalls un-

terbrochen ist von 1-2 Sauerstoffatomen, 1-2 Schwefelatomen und/oder 1-2 NH-Gruppen und/oder gegebenenfalls substituiert ist durch 1-2 Hydroxygruppen, 1-2 Aminogruppen, 1-2 SH-Gruppen, 1-2 COOH-Gruppen und/oder 1-2 Phenylgruppen,

und die Gruppe $Y_3$ sowohl in der 2α- als auch der epimeren 2β-Situation vorliegen kann,

$R_1$ und $R_2$ je ein Wasserstoffatom oder gemeinsam eine exocyclische Methylengruppe bedeuten,

$R_3$ und $R_4$ unabhängig voneinander ein Wasserstoffatom, ein Fluor-, Chlor- oder Bromatom, eine Alkylgruppe mit 1 bis 4 Kohlenstoffatomen, gemeinsam eine Methylengruppe oder gemeinsam mit dem quartären Kohlenstoffatom 20 einen 3-7-gliedrigen, gesättigten oder ungesättigten carbocyclischen Ring bedeuten,

Q eine geradkettige Alkylengruppe mit 1 bis 5 Kohlenstoffatomen bedeutet,

$X_1$ und $X_2$ gemeinsam ein doppelt gebundenes Ketosauerstoffatom oder unabhängig voneinander ein Wasserstoffatom, eine Hydroxygruppe, eine -OC(O)$R_5$-Gruppe, ein Fluor-, Chlor- oder Bromatom bedeuten, wobei $X_1$ und $X_2$ nicht gleichzeitig je eine Hydroxygruppe oder je eine -OC(O)$R_5$-Gruppe sein dürfen,

Z einen carbocylischen oder heterocyclischen gegebenenfalls aromatischen oder heteroaromatischen Ring mit 5- oder 6-Ringliedern oder ein kondensiertes Ringsystem bestehend aus einem 5- und einem 6-gliedrigen Ring oder zwei 6-gliedrigen Ringen, die substituiert sein können durch ein oder mehrere Fluor-, Chlor-, Brom- oder Iodatome, eine oder mehrere Hydroxygruppen, eine oder mehrere COOR$_6$-Gruppen, eine oder mehrere $C_1$-$C_5$-Alkylgruppen, die ihrerseits substituiert sein können durch ein oder mehrere Fluor-, Chlor-, Brom- oder Iod- atome, $C_1$-$C_6$-Alkoxygruppen und/oder COOR$_6$-Gruppen, bedeutet,
wobei

$R_6$ für eine $C_1$-$C_6$-Alkylgruppe, eine Benzylgruppe oder eine Phenylgruppe steht,

sowie alle möglichen Epimeren oder Diastereomeren und deren Gemische.

Die Erfindung betrifft ferner ein Verfahren zur Herstellung der erfindungsgemäßen Verbindungen, Zwischenprodukte des Herstellungsverfahrens sowie die Verwendung der erfindungsgemäßen Verbindungen zur Herstellung von Arzneimitteln.

Besonders vorteilhafte Ausführungsformen der Erfindung sind Gegenstand der Unteransprüche.

Die Gruppe —C(O)$R_5$ , die für $Y_1$ und $Y_2$ definiert ist, kann 1 bis 13 Kohlenstoffatome tragen und leitet sich insbesondere von gesättigten Carbonsäuren ab. Die Reste können cyclisch, acyclisch, geradkettig oder verzweigt, gesättigt oder ungesättigt, carbocyclisch oder heterocyclisch, sein. Bevorzugt leiten sich die Reste von $C_1$-$C_9$-Carbonsäuren ab. Beispielsweise seien Ameisensäure, Essigsäure, Propionsäure, Butansäure, Pentansäure, Pivalinsäure genannt. Besonders bevorzugt können die Gruppen $Y_1$ und $Y_2$ unabhängig voneinander je ein Wasserstoffatom oder eine Acetyl-, Propionyl- oder Pivaloyl-Gruppe bedeuten.

Diese Erläuterung findet auch für die Gruppe -OC(O)$R_5$ Anwendung, die für die Reste $Y_3$, $X_1$ und $X_2$ definiert ist.

Die Gruppe $Y_3$ kann ein Wasserstoffatom, ein Fluor-, Chlor- oder Bromatom oder eine Hydroxygruppe, eine OR$_5$-Gruppe oder eine -OC(O)$R_5$-Gruppe bedeuten.

Die Alkoxygruppe $Y_3$ kann geradkettig oder verzweigt, bevorzugt unsubstituiert und ohne Unterbrechung von Heteroatomen z.B. Methoxy, Ethoxy, Propoxy, Isopropoxy, Butoxy, Isobutoxy, t-.Butoxy bedeuten. Die bevorzugte Kettenlänge ist $C_1$-$C_9$.

Die Gruppen $R_3$ und $R_4$ können unabhängig voneinander je ein Fluor-, Chlor- oder Bromatom, eine Alkylgruppe mit 1 bis 4 Kohlenstoffatomen (Methyl-, Ethyl,- n-Propyl-, i-Propyl, n-Butyl-, i-Butyl-, t-Butyl-), gemeinsam eine Methylengruppe oder gemeinsam mit dem quartären Kohlenstoffatom 20 einen 3-7-gliedrigen, gesättigten oder ungesättigten carbocyclischen Ring bedeuten.

Für $R_3$ und $R_4$ gelten die folgenden bevorzugten Kombinationen: $R_3$ = H, $R_4$ = Methyl oder $R_3$ = Methyl, $R_4$ = H; $R_3$ = $R_4$ = Methyl; $R_3$ und $R_4$ bilden zusammen eine Methylengruppe oder gemeinsam mit dem tertiären Kohlenstoffatom 20 einen Cyclopropylring.

Der optionale Rest $R_5$ aus dem für $Y_3$, $X_1$ und $X_2$ definierten Rest -OC(O)$R_5$ ist ein organischer Rest mit 1 bis 12 C-Atomen, der sich von den entsprechend um ein Kohlenstoffatom längeren Carbonsäuren ableitet. Diese Reste können gesättigt oder ungesättigt, verzweigt oder unverzweigt, gesättigt oder ungesättigt, acyclisch, carbocyclisch oder heterocyclisch sein. Beispiele für den Rest $R_5$ sind Methyl-, Ethyl-, Propyl-, i-Propyl-, Butyl- oder Phenylgruppen.

Möglich sind aber auch die Reste der natürlich vorkommenden Aminosäuren.

Der bevorzugte Rest $R_5$ leitet sich von $C_1$- bis $C_9$-, insbesondere $C_2$- bis $C_5$-Alkancarbonsäuren wie beispielsweise Essigsäure, Propionsäure, Buttersäure oder Pivaloylsäure ab. Unter den aromatischen Gruppen sind die Phenylgruppe und substituierte Phenylgruppen bevorzugt.

Die Alkylgruppe $R_6$ kann geradkettig oder verzweigt, gesättigt oder ungesättigt sein und z.B. Methyl, Ethyl, Propyl, Butyl, Isobutyl, tert.Butyl, Pentyl, Isopentyl, Neopentyl, Hexyl bedeuten.

Die Benzylgruppe und die Phenylgruppe $R_6$ kann unsubstituiert oder auch durch ein oder mehrere Halogenatom(e), Hydroxygruppe(n), $C_1$-$C_4$-Alkoxygruppe(n), $CF_3$-Gruppe(n) oder Aminogruppe(n) substituiert sein. Bevorzugt wird die unsubstituierte Benzyl- und Phenylgruppe.

Q soll bevorzugt eine Methylen-, Ethylen- oder Propylengruppe bedeuten.

$X_1$ und $X_2$ sollen bevorzugt gemeinsam eine Carbonylgruppe
oder $X_1$ eine Hydroxylgruppe oder ein Fluoratom und $X_2$ ein Wasserstoffatom
oder $X_1$ ein Wasserstoffatom und $X_2$ eine Hydroxylgruppe oder ein Fluoratom
oder $X_1$ -OC(O)$R_6$-Gruppe und $X_2$ ein Wasserstoffatom
oder $X_1$ ein Wasserstoffatom und $X_2$ eine -OC(O)$R_6$-Gruppe bedeuten.

Die beiden Fälle, in denen $X_1$=$X_2$=OH oder $X_1$=$X_2$=O-C(O)$R_6$ bedeuten, wurden ausgeschlossen, da sie chemisch nicht sinnvoll sind.

Z soll bevorzugt einen Phenylring bedeuten, der in ortho-, meta- oder para-Stellung mit einer oder mehreren Methoxy-, Ethoxy-, Propoxy-, Hydroxy, Fluor-, Chlor-, Brom-, Methyl-, Ethyl-, Propyl- oder Trifluormethylgruppen substituiert ist oder Z soll bevorzugt ein heterocyclisches System wie z.B. einen Furan-, Thiophen-, Pyrazol, Pyrrol-, Oxazol-, Thiazol-, Imidazolring, der an einer oder mehreren beliebigen Position(en) Methyl-, Ethyl- oder Propylgruppen, die ihrerseits durch Halogen der Hydroxygruppen substituiert sein können, tragen kann und über jedes beliebige C-Atom mit dem Stammsystem verknüpft sein kann, bedeuten oder Z soll bevorzugt ein heterocyclisches kondensiertes System wie z.B. ein Benzofuran, Benzothiophen, Benzimidazol, Benzoxazol, Benzothiazol, Indol-System bedeuten, das an jeder beliebigen Position Methyl-, Ethyloder Propylgruppen, die ihrerseits durch Halogen der Hydroxygruppen substituiert sein können, tragen kann und über jedes beliebige C-Atom mit dem Stammsystem verknüpft sein kann.

Wird der Begriff Halogen verwendet, so ist damit Fluor, Chlor, Brom oder Iod im Zusammenhang mit Substitutionsmustern von Resten und bevorzugt Brom oder Iod als Abgangsgruppe im Verfahren gemeint.

Von den erfindungsgemäßen Verbindungen der allgemeinen Formel I sind die folgenden Verbindungen ganz besonders bevorzugt:

(7*E*)-(1*R*,3*R*,24a*R*)-24a-(Oxazol-4-yl)-24a-homo-19-nor-9,10-secochola-5,7-dien-1,3,24a-triol
(7*E*)-(1*R*,3*R*,24a*S*)-24a-(Oxazol-4-yl)-24a-homo-19-nor-9,10-secochola-5,7-dien-1,3,24a-triol
(5*Z*,7*E*)-(1*S*,3*R*,24a*R*)-24a-(Oxazol-4-yl)-24a-homo-9,10-secochola-5,7,10(19)-trien-1,3,24a-triol
(5*Z*,7*E*)-(1*S*,3*R*,24a*S*)-24a-(Oxazol-4-yl)-24a-homo-9,10-secochola-5,7,10(19)-trien-1,3,24a-triol (7*E*)-(1*R*,3*R*,24a*R*)-24a-(Thiazol-2-yl)-24a-homo-19-nor-9,10-secochola-5,7-dien-1,3,24a-triol
(7*E*)-(1*R*,3*R*,24a*S*)-24a-(Thiazol-2-yl)-24a-homo-19-nor-9,10-secochola-5,7-dien-1,3,24a-triol
(7*E*)-(1*R*,3*R*)-1,3-Dihydroxy-24a-(thiazol-2-yl-)-24-a-homo-19-nor-9,10-secochola-5,7-dien-24a-on
(5*Z*,7*E*)-(1*S*,3*R*,24a*R*)-24a-(Thiazol-2-yl)-24a-homo-9,10-secochola-5,7,10(19)-trien-1,3,24a-triol
(5*Z*,7*E*)-(1*S*,3*R*,24a*S*)-24a-(Thiazol-2-yl)-24a-homo-9,10-secochola-5,7,10(19)-trien-1,3,24a-triol (5*Z*,7*E*)-(1*S*,3*R*)-1,3-Dihydroxy-24a-(thiazol-2-yl)-24a-homo-9,10-secochola-5,7,10(19)-trien-24a-on
(7*E*)-(1*R*,3*R*,24a*R*)-24a-(4-Methylthiazol-2-yl)-24a-homo-19-nor-9, 10-secochola-5,7-dien-1,3,24a-triol
(7*E*)-(1*R*,3*R*,24a*S*)-24a-(4-Methylthiazol-2-yl)-24a-homo-19-nor-9,10-secochola-5,7-dien-1,3,24a-triol
(7*E*)-(1*R*,3*R*)-1,3-Dihydroxy-24a-(4-methylthiazol-2-yl)-24a-homo-19-nor-9,10-secochola-5,7-dien-24a-on
(5*Z*,7*E*)-(1*S*,3*R*,24a*R*)-24a-(4-Methylthiazol-2-yl)-24a-homo-9,10-secochola-5,7,10(19)-trien-1,3,24a-triol
(5*Z*,7*E*)-(1*S*,3*R*,24a*S*)-24a-(4-Methylthiazol-2-yl)-24a-homo-9,10-secochola-5,7,10(19)-trien-1,3,24a-triol
(5*Z*,7*E*)-(1*S*,3*R*)-1,3-Dihydroxy-24a-(4-methylthiazol-2-yl)-24a-homo-9,10-secochola-5,7,10(19)-trien-24a-on
(7*E*)-(1*R*,3*R*,24a*R*)-24a-(Thien-2-yl)-24a-homo-19-nor-9,10-secochola-5,7-dien-1,3,24atriol
(7*E*)-(1*R*,3*R*,24a*S*)-24a-(Thien-2-yl)-24a-homo-19-nor-9,10-secochola-5,7-dien-1,3,24atriol
(7*E*)-(1*R*,3*R*)-1,3-Dihydroxy-24a-(thien-2-yl)-24a-homo-19-nor-9,10-secochola-5,7-dien-24a-on
(5*Z*,7*E*)-(1*S*,3*R*,24a*R*)-24a-(Thien-2-yl)-24a-homo-9,10-secochola-5,7,10( 19)-trien1,3,24a-triol (5*Z*,7*E*)-(1*S*,3*R*,24a*S*)-24a-(Thien-2-yl)-24a-homo-9,10-secochola-5,7,10(19)-trien-1,3,24a-triol (7*E*-(1*R*,2*S*,3*R*,24a*R*)-24a-Thien-2-yl-24a-homo-19-nor-9,10-secochola-5,7-dien-1,2,3,24-tetrol(7*E*)-(1*R*,2*S*,3*R*,24a*S*)-24a-Thien-2-yl-24ahomo-19-nor-9,10-secochola-5,7-dien-1,2,3,24-tetrol(7*E*)-(1*R*,2*S*,3*R*)-24a-Thien-2-yl-1,2,3-trihydroxy-24a-homo-9,10-secochola-5,7-dien-24a-on
(7*E*)-(1*R*,3*R*,24a*R*)-24a-(4-Methylthien-2-yl)-24a-homo-19-nor-9,10-secochola-5,7-dien-1,3,24a-triol
(7*E*)-(1*R*,3*R*,24a*S*)-24a-(4-Methylthien-2-yl)-24a-homo-19-nor-9,10-secochola-5,7-dien-1,3,24a-triol
(7*E*)-(1*R*,3*R*)-1,3-Dihydroxy-24a-(4-methylthien-2-yl)-24a-homo-19-nor-9,10-secochola-5,7-dien-24a-on

(5*Z*,7*E*)-(1*S*,3*R*,24a*R*)-24a-(4-Methylthien-2-yl)-24a-homo-9,10-secochola-5,7,10(19)-trien-1,3,24a-triol
(5*Z*,7*E*)-(1*S*,3*R*,24a*S*)-24a-(4-Methylthien-2-yl)-24a-homo-9,10-secochola-5,7,10(19)-trien-1,3,24a-triol
(5*Z*,7*E*)-(1*S*,3*R*)-1,3-Dihydroxy-24a-(4-methylthien-2-yl)-24a-homo-9,10-secochola-5,7,10(19)-trien-24a-on
(7*E*)-(1*R*,2*S*,3*R*,24a*R*)-24a-(4-Methylthien-2-yl)-24a-homo-19-nor-9,10-secochola-5,7-dien-1,2,3,24-tetrol
(7*E*)-(1*R*,2*S*,3*R*,24a*S*)-24a-(4-Methylthien-2-yl)-24a-homo-19-nor-9,10-secochola-5,7-dien-1,2,3,24-tetrol
(7*E*)-(1*R*,2*S*,3*R*)-24a-(4-Methylthien-2-yl)-1,2,3-trihydroxy-24a-homo-19-nor-9,10-secochola-5,7-dien-24a-on
(7*E*)-(1*R*,3*R*,24a*R*)-24a-(5-Ethylthien-2-yl)-24a-homo-19-nor-9,10-secochola-5,7-dien-1,3,24a-triol
(7*E*)-(1*R*,3*R*,24a*S*)-24a-(5-Ethylthien-2-yl)-24a-homo- 19-nor-9,10-secochola-5,7-dien-1,3,24a-triol
(7*E*)-(1*R*,3*R*,24a*R*)-24a-[5-(2-Hydroxyethyl)-4-methylthiazol-2-yl]-24a-homo-19-nor-9,10-secochola-5,7-dien-1,3,24a-triol
(7*E*)-(1*R*,3*R*,24a*S*)-24a-[5-(2-Hydroxyethyl)-4-methylthiazol-2-yl]-24a-homo-19-nor-9,10-secochola-5,7-dien-1,3,24a-triol
(7*E*)-(1*R*,3*R*)-1,3-Dihydroxy-24a-[5-(2-hydroxyethyl)-4-methylthiazo-2-yl]-24a-homo-19-nor-9,10-secochola-5,7-dien-24a-on
(7*E*)-(1*R*,3*R*,24a*R*)-24a-(Benzothiazol-2-yl)-24a-homo-19-nor-9,10-secochola-5,7-dien-1,3,24a-triol
(7*E*)-(1*R*,3*R*,24a*S*)-24a-(Benzothiazol-2-yl)-24a-homo-19-nor-9,10-secochola-5,7-dien-1,3,24a-triol
(7*E*)-(1*R*,3*R*)-24a-(Benzothiazol-2-yl)-1,3-dihydroxy-24a-homo-19-nor-9,10-secochola-5,7-dien-24a-on
(7*E*)-(1*R*,3R24a*R*)-24a-(Benzofuran-2-yl)-24a-homo-19-nor-9,10-secochola-5,7-dien-1,3,24a-triol (7*E*)-(1*R*,3*R*,24a*S*)-24a-(Benzofuran-2-yl)-24a-homo-19-nor-9,10-secochola-5,7-dien-1,3,24a-triol
(7*E*)-(1*R*,3*R*)-24a-(Benzofuran-2-yl)-1,3-dihydroxy-24a-homo-19-nor-9,10-secochola-5,7-dien-24a-on
(7*E*)-(1*R*,3*R*,24a*R*)-24a-(Benzothiophen-2-yl)-24a-homo-19-nor-9,10-secochola-5,7-dien-1,3,24a-triol
(7*E*)-(1*R*,3*R*,24a*S*)-24a-(Benzothiophen-2-yl)-24a-homo-19-nor-9,10-secochola-5,7-dien-1,3,24a-triol
(7*E*)-(1*R*,3*R*)-24a-(Benzothiophen-2-yl)-1,3-dihydroxy-24a-homo-19-nor-9,10-secochola-5,7-dien-24a-on
(7*E*)-(1*R*,3*R*,24a*R*)-24a-(1-Methylbenzimidazol-2-yl)-19-nor-9,10-secochola-5,7-dien-1,3,24a-triol
(7*E*)-(1*R*,3*R*,24a*S*)-24a-(1-Methylbenzimidazol-2-yl)-19-nor-9,10-secochola-5,7-dien-1,3 ,24a-triol
(7*E*)-(1*R*,3*R*)-1,3-Dihydroxy-24a-(1-methylbenzimidazol-2-yl)-24a-homo-19-nor-9,10-secochola-5,7-dien-24a-on
(7*E*)-(1*R*,3*R*)-1-(1,3-Dihydroxy-24a-homo-19-nor-9,10-secochola-5,7-dien-24a-yl)-3-[(4-methoxyphenyl)methoxy]-1*H* pyrazol-4-carbonsäureethylester
(7*E*)-(1*R*,3*R*)-1-(1,3-Dihydroxy-24a-homo-19-nor-9,10-secochola-5, 7-dien-24a-yl)-3-hydroxy-1*H*-pyrazol-4-carbonsäureethylester
(7*E*)-(1*R*,3*R*,24a*R*)-24a-(4-Methylphenyl)-24a-homo-19-nor-9,10-secochola-5,7-dien-1,3,24a-triol (7*E*)-(1*R*,3*R*,24a*S*)-24a-(4-Methylphenyl)-24a-homo-19-nor-9,10-secochola-5,7-dien-1,3,24a-triol
(7*E*)-(1*R*,3*R*)-1,3-Dihydroxy-24a-(4-methylphenyl)-24a-homo-19-nor-9,10-secochola-5,7-dien-24a-on
(7*E*)-(1*R*,2*R*,3*R*,24a*R*)-24a-(4-Methylphenyl)-24a-homo-19-nor-9,10-secochola-5,7-dien-1,2,3,24a-tetrol
(7*E*)-(1*R*,R*S*,3*R*,24a*S*)-24a-(4-Methylphenyl)-24a-homo-19-nor-9,10-secochola-5,7-dien-1,2,3,24a-tetrol
(7*E*)-(1*R*,3*R*,24a*R*)-24a-(4-Trifluormethylphenyl)-19-nor-9,10-secochola-5,7-dien-1,3,24a-triol (7*E*)-(1*R*,3*R*,24a*S*)-24a-(4-Trifluormethylphenyl)-19-nor-9,10-secochola-5,7-dien- 1,3,24a-triol (7*E*)-(1*R*,3*R*)-1,3-Dihydroxy-24a-(4-trifluormethylphenyl)-24ahomo-19-nor-9,10-secochola-5,7-dien-24a-on
(7*E*)-(1*R*,3*R*,24a*R*)-24a-(4-Methoxyphenyl)-24a-homo-19-nor-9,10-secochola-5,7-dien-1,3,24a-triol (7*E*)-(1*R*,3*R*,24a*S*)-24a-(4-Methoxyphenyl)-24a-homo-19-nor-9,10-secochola-5,7-dien-1,3,24a-triol
(7*E*)-(1*R*,3*R*)-1,3-Dihydroxy-24a-(4-methoxyphenyl)-24a-homo-19-nor-9,10-secochola-5,7-dien-24a-on
(7*E*)-(1*R*,3*R*,20*S*,24a*R*)-24a-(Thiazol-2-yl)-24a-homo-19-nor-9,10-secochola-5,7-dien-1,3,24a-triol
(7*E*)-(1*R*,3*R*,20*S*,24a*S*)-24a-(Thiazol-2-yl)-24-a-homo-19-nor-9,10-secochola-5,7-dien-1,3,24a-triol
(7*E*)-(1*R*,3*R*,20*S*)-1,3-Dihydroxy-24a-(thiazol-2-yl)-24-a-homo-19-nor-9,10-secochola-5,7-dien-24a-on
(5*Z*,7*E*)-(1*S*,3*R*,20*S*,24a*R*)-24a-(Thiazol-2-yl)-24a-homo-9,10-secochola-5,7,10(19)-trien-1,3,24a-triol
(5*Z*,7*E*)-(1*S*,3*R*,20*S*,24a*S*)-24a-(Thiazol-2-yl)-24a-homo-9,10-secochola-5,7,10(19)-trien- 1,3,24a-triol
(5*Z*,7*E*)-(1*S*,3*R*,20*S*)-1,3-Dihydroxy-24a-(thiazol-2-yl)-24a-homo-9,10-secochola-5,7,10(19)-trien-24a-on
(5*Z*,7*E*)-(1*S*,3*R*,24*S*)-24-(Thiazol-2-yl)-9,10-secochola-5,7,10(19)-trien-1,3,24-triol
(5*Z*,7*E*)-(1*S*,3*R*,24*R*)-24-(Thiazol-2-yl)-9,10-secochola-5,7,10(19)-trien-1,3,24-triol
(5*Z*,7*E*)-(1*S*,3*R*)-1,3-Dihydroxy-24-(thiazol-2-yl)-9,10-secochola-5,7,10(19)-trien-24-on (7*E*)-(1*R*,3*R*,20*S*,24a*R*)-24a-(Oxazol-4-yl)-24a-homo-19-nor-9,10-secochola-5,7-dien-1,3,24a-triol
(7*E*)-(1*R*,3*R*,20*S*,24a*S*)-24a-(Oxazol-4-yl)-24-a-homo-19-nor-9,10-secochola-5,7-dien-1,3,24a-triol (7*E*)-(1*R*,3*R*,20*S*)-1,3-Dihydroxy-24a-(oxazol-4-yl)-24-a-homo-19-nor-9,10-secochola-5,7-dien-24a-on
(5*Z*,7*E*)-(1*S*,3*R*,20*S*,24a*R*)-24a-(Oxazol-4-yl)-24a-homo-9,10-secochola-5,7,10(19)-trien-1,3,24a-triol
(5*Z*,7*E*)-(1*S*,3*R*,20*S*,24a*S*)-24a-(Oxazol-4-yl)-24a-homo-9,10-secochola-5,7,10(19)-trien-1,3,24a-triol
(5*Z*,7*E*)-(1*S*,3*R*,20*S*)-1,3-Dihydroxy-24a-(oxazol-4-yl)-24a-homo-9,10-secochola-5,7,10(19)-trien-24a-on
(5*Z*,7*E*)-(1*S*,3*R*,24*R*)-24-(Oxazol-4-yl)-9,10-secochola-5,7,10(19)-trien-1,3,24a-triol
(5*Z*,7*E*)-(1*S*,3*R*,24*S*)-24-(Oxazol-4-yl)-9,10-secochola-5,7,10(19)-trien-1,3,24a-triol

(5*Z*,7*E*)-(1*S*,3*R*)-1,3-Dihydroxy-24-(oxazol-4-yl)-9,10-secochola-5,7,10(19)-trien-24-on (7*E*)-(1*R*,3*R*,20*S*,24a*R*)-24a-(4-Methylthiazol-2-yl)-24a-homo-19-nor-9,10-secochola-5,7-dien- 1,3,24a-triol
(7*E*)-(1*R*,3*R*,20*S*,24a*S*)-24a-(4-Methylthiazol-2-yl)-24-a-homo-19-nor-9,10-secochola-5,7-dien-1,3,24a-triol
(7*E*)-(1*R*,3*R*,20*S*)-1,3-Dihydroxy-24a-(4-methylthiazol-2-yl)-24-a-homo-19-nor-9,10-secochola-5,7-dien-24a-on
(5*Z*,7*E*)-(1*S*,3*R*,20*S*,24a*R*)-24a-(4-Methylthiazol-2-yl)-24a-homo-9,10-secochola-5,7,10(19)-trien- 1,3,24a-triol
(5*Z*,7*E*)-(1*S*,3*R*,20*S*,24a*S*)-24a-(4-Methylthiazol-2-yl)-24a-homo-9,10-secochola-5,7,10(19)-trien-1,3,24a-triol
(5*Z*,7*E*)-(1*S*,3*R*,20*S*)-1,3-Dihydroxy-24a-(4-methylthiazol-2-yl)-24a-homo-9,10-secochola-5,7,10(19)-trien-24a-on
(5*Z*,7*E*)-(1*S*,3*R*,24*R*)-24-(4-Methylthiazol-2-yl)-9,10-secochola-5,7,10( 19)-trien-1,3,24atriol
(5*Z*,7*E*)-(1*S*,3*R*,24*S*)-24-(4-Methylthiazol-2-yl)-9,10-secochola-5,7,10(19)-trien-1,3,24atriol
(5*Z*,7*E*)-(1*S*,3*R*)-1,3-Dihydroxy-24-(4-methylthiazol-2-yl)-9,10-secochola-5,7,10(19)-trien-24-on
(7*E*)-(1*R*,3*R*,20*S*,24a*R*)-24a-(4-Methylthien-2-yl)-24a-homo-19-nor-9,10-secochola-5,7-dien-1,3,24a-triol
(7*E*)-(1*R*,3*R*,20*S*,24a*S*)-24a-(4-Methylthien-2-yl)-24-a-homo-19-nor-9,10-secochola-5,7-dien-1,3,24a-triol
(7*E*)-(1*R*,3*R*,20*S*)-1,3-Dihydroxy-24a-(4-methylthien-2-yl)-24-a-homo-19-nor-9,10-secochola-5,7-dien-24a-on
(5*Z*,7*E*)-(1*S*,3*R*,20*S*,24a*R*)-24a-(4-Methylthien-2-yl)-24a-homo-9,10-secochola-5,7,10(19)-trien-1,3,24a-triol
(5*Z*,7*E*)-(1*S*,3*R*,20*S*,24a*S*)-24a-(4-Methylthien-2-yl)-24a-homo-9,10-secochola-5,7,10(19)-trien- 1,3,24a-triol
(5*Z*,7*E*)-(1*S*,3*R*,20*S*)-1,3-Dihydroxy-24a-(4-methylthien-2-yl)-24a-homo-9,10-secochola-5,7,10(19)-trien-24a-on
(5*Z*,7*E*)-(1*S*,3*R*,24*R*)-24-(4-Methylthien-2-yl)-9,10-secochola-5,7,10(19)-trien-1,3,24atriol
(5*Z*,7*E*)-(1*S*,3*R*,24*S*)-24-(4-Methylthien-2-yl)-9,10-secochola-5,7,10(19)-trien-1,3,24atriol
(5*Z*,7*E*)-(1*S*,3*R*)-1,3-Dihydroxy-24-(4-methylthien-2-yl)-9,10-secochola-5,7,10(19)-trien-24-on
(7*E*)-(1*R*,3*R*,20*S*,24a*R*)-24a-(Thien-2-yl)-24a-homo-19-nor-9,10-secochola-5,7-dien-1,3,24a-triol
(7*E*)-(1*R*,3*R*,20*S*,24a*S*)-24a-(Thien-2-yl)-24-a-homo-19-nor-9,10-secochola-5,7-dien-1,3,24a-triol
(7*E*)-(1*R*,3*R*,20*S*)-1,3-Dihydroxy-24a-(thien-2-yl)-24-a-homo-19-nor-9,10-secochola-5,7-dien-24a-on
(5*Z*,7*E*)-(1*S*,3*R*,20*S*,24a*R*)-24a-(Thien-2-yl)-24a-homo-9,10-secochola-5,7,10(19)-trien-1,3,24a-triol
(5*Z*,7*E*)-(1*S*,3*R*,20*S*,24a*S*)-24a-(Thien-2-yl)-24a-homo-9,10-secochola-5,7,10(19)-trien-1,3,24a-triol
(5*Z*,7*E*)-(1*S*,3*R*,20*S*)-1,3-Dihydroxy-24a-(thien-2-yl)-24a-homo-9,10-secochola-5,7,10(19)-trien-24a-on
(5*Z*,7*E*)-(1*S*,3*R*,24*R*)-24-(Thien-2-yl)-9,10-secochola-5,7,10(19)-trien-1,3,24a-triol
(5*Z*,7*E*)-(1*S*,3*R*,24*S*)-24-(Thien-2-yl)-9,10-secochola-5,7,10(19)-trien-1,3,24a-triol
(5*Z*,7*E*)-(1*S*,3*R*)-1,3-Dihydroxy-24-(Thien-2-yl)-9,10-secochola-5,7,10(19)-trien-24-on
(5*Z*,7*E*)-(1*S*,3*R*,24*S*)-24-(4-Methylthiazol-2-yl)-9,10-secochola-5,7,10(19)-trien-1,3,24-triol
(5*Z*,7*E*)-(1*S*,3*R*,24*R*)-24-(4-Methylthiazol-2-yl)-9,10-secochola-5,7,10(19)-trien-1,3,24-triol
(5*Z*,7*E*)-(1*S*,3*R*)-1,3-Dihydroxy-24-(4-methylthiazol-2-yl)-9,10-secochola-5,7,10(19)-trien-24-on
(5*Z*,7*E*)-(1*S*,3*R*,24*S*)-24-(Thien-2-yl)-9,10-secochola-5,7,10(19)-trien-1,3,24-triol
(5*Z*,7*E*)-(1*S*,3*R*,24*R*)-24-(Thien-2-yl)-9,10-secochola-5,7,10(19)-trien-1,3,24-triol
(5*Z*,7*E*)-(1*S*,3*R*)-1,3-Dihydroxy-24-(thien-2-yl)-9,10-secochola-5,7,10(19)-trien-24-on
(5*Z*,7*E*)-(1*S*,3*R*,24*S*)-24-(4-Methylthien-2-yl)-9,10-secochola-5,7,10(19)-trien-1,3,24-triol
(5*Z*,7*E*)-(1*S*,3*R*,24*R*)-24-(4-Methylthien-2-yl)-9,10-secochola-5,7,10(19)-trien-1,3,24-triol
(5*Z*,7*E*)-(1*S*,3*R*)-1,3-Dihydroxy-24-(4-methylthien-2-yl)-9,10-secochola-5,7,10(19)-trien-24-on
(7*E*)-(1*R*,3*R*,24a*R*)-24a-Fluor-24a-(thiazol-2-yl)-24a-homo-19-nor-9,10-secochola-5,7-dien-1,3-diol
(7*E*)-(1*R*,3*R*,24a*S*)-24a-Fluor-24a-(thiazol-2-yl)-24a-homo-19-nor-9,10-secochola-5,7-dien-1,3-diol
(5*Z*,7*E*)-(1*S*,3*R*,24a*R*)-24a-Fluor-24a-(thiazol-2-yl)-24a-homo-9,10-secochola-5,7,10(19)-trien-1,3-diol
(5*Z*,7*E*)-(1*S*,3*R*,24a*S*)-24a-Fluor-24a-(thiazol-2-yl)-24a-homo-9,10-secochola-5,7,10(19)-trien-1,3-diol
(7*E*)-(1*R*,3*R*,24a*R*)-24a-(Acetyloxy)-24a-(thiazol-2-yl)-24a-homo-19-nor-9,10-secochola-5,7-dien-1,3,24a-triol
(7*E*)-(1*R*,3*R*,24a*S*)-24a-(Acetyloxy)-24a-(thiazol-2-yl)-24a-homo-19-nor-9,10-secochola-5,7-dien-1,3,24a-triol
(7*E*)-(1*R*,3*R*,24a*R*)-24a-(2,2-Dimethylpropanoyloxy)-24a-(thiazol-2-yl)-24a-homo-19-nor-9,10-secochola-5,7-dien-1,3,24a-triol
(7*E*)-(1*R*,3*R*,24a*S*)-24a-(2,2-Dimethylpropanoyloxy)-24a-(thiazol-2-yl)-24a-homo-19-nor-9,10-secochola-5,7-dien-1,3,24a-triol
(7*E*)-(1*R*,3*R*)-2-Brom-24a-(thiazol-2-yl)-24a-homo-19-nor-9,10-secochola-5,7-dien-1,3,24a-triol

Die erfindungsgemäßen Substanzen besitzen eine deutlich höhere metabolische Stabilität als die strukturell verwandten Verbindungen des Standes der Technik und eignen sich daher in besonderer Weise für systemische Applikationen.
Gegenüber den strukturell verwandten Verbindungen des Standes der Technik zeichnen sich einige der erfindungsgemäßen Substanzen ferner dadurch aus, daß sie eine starke Wirkung auf die Zelldifferenzierung zeigen, wobei die Wirkung auf den Calciumhaushalt nicht zunimmt.
Andere der erfindungsgemäßen Substanzen dagegen weisen ein antagonistisches oder partialagonistisches Wirkprofil auf, das neue Anwendungen ermöglicht.

**Bestimmung der biologischen Aktivität**

Die Vitamin D-Aktivität der erfindungsgemäßen Substanzen wird mittels des Calcitriol-Rezeptortests bestimmt. Er wird unter Verwendung eines Proteinextraktes aus dem Darm von jungen Schweinen durchgerührt. Rezeptorhaltiger Proteinextrakt wird mit $^3$H-Calcitriol (5 x $10^{-10}$ mol/l) in einem Reaktionsvolumen von 0,27 ml in Abwesenheit und in Anwesenheit der Prüfsubstanzen für zwei Stunden bei 4°C in einem Teströhrchen inkubiert. Zur Trennung von freiem und rezeptorgebundenem Calcitriol wird eine Charcoal-Dextran-Absorption durchgeführt. Dazu werden 250 µl einer Charcoal-Dextran-Suspension jedem Teströhrchen zugeführt und bei 4°C für 20 Minuten inkubiert. Anschließend werden die Proben bei 10 000 g 5 Minuten bei 4°C zentrifugiert. Der Überstand wird dekantiert und nach einstündiger Äquilibrierung in Picofluor 15 ™ in einem β-Zähler gemessen.
Die mit verschiedenen Konzentrationen der Prüfsubstanz sowie der Referenzsubstanz (unmarkiertes Calcitriol) bei konstanter Konzentration der Bezugssubstanz ($^3$H-Calcitriol) erhaltenen Kompetitionskurven werden in Beziehung zueinander gesetzt und ein Kompetitionsfaktor (KF) ermittelt.
Er ist definiert als Quotient aus den Konzentrationen der jeweiligen Prüfsubstanz und der Referenzsubstanz, die für 50%ige Kompetition erforderlich sind:

$$KF= \frac{\text{Konzentration Prüfsubstanz bei 50\% Kompetition}}{\text{Konzentration Referenzsubstanz bei 50\% Kompetition}}$$

Zur Bestimmung der akuten hypercalcämischen Wirkung verschiedener Calcitriolderivate wird nachfolgend beschriebener Test durchgeführt:
Die Wirkung von Kontrolle (Lösungsgrundlage), Referenzsubstanz (1,25-Dihydroxyvitamin $D_3$ = Calcitriol) und Testsubstanz wird jeweils nach einmaliger subcutaner Applikation in Gruppen von 10 gesunden männlichen Ratten (140-170 g) getestet. Die Ratten werden während der Versuchszeit in speziellen Käfigen zur Bestimmung der Exkretion von Wasser und Mineralstoffen gehalten. Der Harn wird in 2 Fraktionen (0-16 h und 16-22 h) gesammelt. Eine orale Calciumgabe (0,1 mM Calcium in 6,5% Alpha-Hydroxypropylcellulose, 5 ml/Tier) ersetzt zum Zeitpunkt 16 h die durch Futterentzug fehlende Calciumaufnahme. Zu Versuchsende werden die Tiere durch Dekapitieren getötet und für die Bestimmung der Serum-Calciumwerte entblutet. Für die primäre Screen-Untersuchung in vivo wird eine einzelne Standarddosis (200 µg/kg) getestet. Für ausgewählte Substanzen wird das Ergebnis durch Erstellung einer Dosis-Wirkungs-Beziehung abgesichert.
Eine hypercalcämische Wirkung zeigt sich in im Vergleich zur Kontrolle erhöhten Serum-Calciumspiegel-Werten.
Die Signifikanz auftretender Unterschiede zwischen Substanzgruppen und Kontrollen sowie zwischen Testsubstanz und Referenzsubstanz werden mit geeigneten statistischen Verfahren abgesichert. Das Ergebnis wird als Dosisrelation DR (DR = Faktor Testsubstanzdosis/ Referenzsubstanzdosis für vergleichbare Wirkungen) angegeben.
Die differenzierungsstimulierende Wirkung von Calcitriolanaloga wird ebenfalls quantitativ erfaßt.
Es ist literaturbekannt [D.J. Mangelsdorf et al., *J. Cell. Biol.* **98**, 391 (1984)], daß die Behandlung humaner Leukämiezellen (Promyelocytenzellinie HL 60) in vitro mit Calcitriol die Differenzierung der Zellen zu Makrophagen induziert.
HL 60-Zellen werden in Gewebekulturmedium (RPMI 10% fetales Kälberserum) bei 37°C in einer Atmosphäre 5% $CO_2$ in Luft kultiviert.
Zur Substanztestung werden die Zellen abzentrifugiert und 2,0 x $10^5$ Zellen/ml in phenolrotfreiem Gewebekulturmedium aufgenommen. Die Testsubstanzen werden in Ethanol gelöst und mit Gewebekulturmedium ohne Phenolrot auf die gewünschte Konzentration verdünnt. Die Verdünnungsstufen werden mit der Zellsuspension in einem Verhältnis von 1:10 gemischt und je 100 µl dieser mit Substanz versetzten Zellsuspension in eine Vertiefung einer 96-Loch-Platte pipettiert. Zur Kontrolle wird eine Zellsuspension analog mit dem Lösungsmittel versetzt.
Nach Inkubation über 96 Stunden bei 37°C in 5% $CO_2$ in Luft wird in jede Vertiefung der 96-Loch-Platte zu der Zellsuspension 100 µl einer NBT-TPA-Lösung (Nitroblautetrazolium (NBT), Endkonzentration im Ansatz 1 mg/ml, Tetradecanoylphorbolmyristat-13-acetat (TPA), Endkonzentration im Ansatz 2 x $10^{-7}$ mol/l pipettiert.
Durch Inkubation über 2 Stunden bei 37°C und 5% $CO_2$ in Luft wird infolge der intrazellulären Sauerstoffradikalfreisetzung, stimuliert durch TPA, in den zu Makrophagen differenzierten Zellen NBT zu unlöslichem Formazan reduziert.
Zur Beendigung der Reaktion werden die Vertiefungen der 96-Loch-Platte abgesaugt und die Zellen durch Zugabe von Methanol am Plattenboden fixiert und nach Fixation getrocknet. Zur Lösung der gebildeten intrazellulären Formazankristalle werden in jede Vertiefung 100 µl Kaliumhydroxid (2 mol/l) und 100 µl Dimethylsulfoxid pipettiert und 1 Minute ultrabeschallt. Die Konzentration von Formazan wird spektralphotometrisch bei 650 nm gemessen.
Als Maß für die Differenzierungsinduktion der HL 60-Zellen zu Makrophagen gilt die Konzentration an gebildetem Formazan. Das Ergebnis wird als Dosisrelation (DR = Faktor Testsubstanzdosis/Referenzsubstanzdosis für vergleichbare halbmaximale Wirkungen) angegeben.
Die Ergebnisse des Calcitriol-Rezeptortests sowie der Bestimmung der Dosisrelation der Differenzierungsinduktion von HL 60-Zellen und der Dosisrelation für Hypercalcämie sind nachfolgend zusammengefaßt:

Beispiele für Testsubstanzen:

(7*E*)-(1*R*,3*R*,24a*R*)-24a-(Oxazol-4-yl)-24a-homo-19-nor-9,10-secochola-5,7-dien-1,3,24a-triol **16a**
(5*Z*,7*E*)-(1*S*,3*R*,24a*R*)-24a-(Oxazol-4-yl)-24a-homo-9,10-secochola-5,7,10(19)-trien-1,3,24a-triol **20a**
(7*E*)-(1*R*,3*R*,24a*S*)-24a-(Thien-2-yl)-24a-homo-19-nor-9,10-secochola-5,7-dien-1,3,24atriol **55b**
(7*E*)-(1*R*,3*R*)-1,3-Dihydroxy-24a-(thien-2-yl)-24a-homo-19-nor-9,10-secochola-5,7-dien-24a-on **57**
(5*Z*,7*E*)-(1*S*,3*R*,24*R*)-24-(Thiazol-2-yl)-9,10-secochola-5,7,10(19)-trien-1,3,24a-triol **217a**

| | **KF** | **DR (HL 60)** | **DR (Ca)** |
|---|---|---|---|
| **16a** | 5 | 5 | >30 |
| **20a** | 3 | 2 | >50 |
| **55b** | 5 | 8 | >100 |
| **57** | 12 | 2 | >350 |
| **217a** | 7 | 100 | >300 |
| **Calcitriol** | 1 | 1 | 1 |

Die aufgeführten Verbindungen zeigen neben einer beträchtlichen Affinität zum Vitamin D-Rezeptor deutliche zelldifferenzierende Aktivität.

Die Induktion einer Hypercalcämie erfolgt dagegen durchweg erst bei sehr viel höheren Dosen als bei Calcitriol.

Durch die verminderte Eigenschaft, eine Hypercalcämie auszulösen sowie die hohe metabolische Stabilität, eignen sich die erfindungsgemäßen Substanzen in besonderer Weise zur Herstellung von Arzneimitteln für die Behandlung von Erkrankungen, die durch eine Hyperproliferation und fehlende Zelldifferenzierung gekennzeichnet sind. Dazu zählen zum Beispiel hyperproliferative Erkrankungen der Haut (Psoriasis, Pituriasis subia pilasis, Akne, Ichthyosis) und Pruritus sowie Tumorerkrankungen und Präkanzerosen (zum Beispiel Darmtumoren, Mammakarzinom, Lungentumoren, Prostatakarzinom, Leukämien, T-Zell-Lymphome, Melanome, Betazell Karzinom, Squamous Carcinoma, aktinische Keratosen, Cervixdysplasien, metastasierende Tumore jeglicher Art).

Auch zur Behandlung und Prophylaxe von Erkrankungen, die durch eine Störung des Gleichgewichts des Immunsystems gekennzeichnet sind, eignen sich die erfindungsgemäßen Substanzen. Hierzu zählen Ekzeme und Erkrankungen des atopischen Formenkreises und entzündliche Erkrankungen (rheumatoide Arthritis, Atemwegserkrankungen z.B. Asthma) sowie Autoimmunerkrankungen wie zum Beispiel Multiple Sklerose, Diabetes mellitus Typ I, Myasthenia gravis, Lupus erythematodes, Sklerodermie, bullöse Hauterkrankungen (Pemphigus, Pemphigoid), weiterhin Abstoßungsreaktionen bei autologen, allogenen oder xenogenen Transplantaten sowie AIDS. Bei all diesen Erkrankungen können die neuen Verbindung der allgemeinen Formel **I** auch vorteilhaft mit anderen immunsuppressiv wirksamen Stoffen wie Cyclosporin A, FK 506, Rapamycin und Anti-CD 4-Antikörpern kombiniert werden.

Ebenso sind die Substanzen geeignet zur Therapie von sekundärem Hyperparathyreoidismus und renaler Osteodystrophie infolge der Eigenschaft von Calcitriolen, die Parathormonsynthese zu senken.

Aufgrund der Präsenz des Vitamin D-Rezeptor in den insulinproduzierenden Zellen der Bauchspeicheldrüse eignen sich die Substanzen durch Erhöhung der Insulinsekretion zur Therapie des Diabetes mellitus Typ II.

Weiterhin wurde überraschenderweise gefunden, daß durch topische Applikation der erfindungsgemäßen Verbindungen auf die Haut von Mäusen, Ratten und Meerschweinchen eine vermehrte Hautrötung und Zunahme der Epidermisdicke induziert werden kann. Die Zunahme der Hautrötung wird anhand der Erhöhung des mit einem Farbmeßgerät quantifizierbaren Rotwertes der Hautoberfläche ermittelt. Der Rotwert ist nach dreimaliger Substanzapplikation (Dosis 0,003%) im Abstand von 24 Stunden typischerweise um das 1,5-fache erhöht. Die Zunahme der Epidermisdicke wird im histologischen Präparat quantifiziert. Sie ist typischerweise um das 2,5-fache erhöht. Die Anzahl der proliferierenden Epidermiszellen (Zellen in der S-Phase des Zellcyclus) wird durchflußcytometrisch ermittelt und ist typischerweise um den Faktor 6 erhöht.

Diese Eigenschaften der erfindungsgemäßen Derivate in der Vitamin D-Reihe läßt sie zum therapeutischen Einsatz bei atrophischer Haut, wie sie bei natürlicher Hautalterung infolge erhöhter Lichtexposition oder medikamentös induzierter Hautatrophie durch Behandlung mit Glucocorticoiden auftritt, geeignet erscheinen.

Darüber hinaus kann die Wundheilung durch topische Applikation mit den neuen Verbindungen beschleunigt werden.

In Zellpopulationen des Haarfollikels, die entscheidend zum Haarwachstum bzw. der Haarzyklusregulation beitragen, konnten Vitamin $D_3$-Rezeptorproteine nachgewiesen werden [W.E. Stumpf et al. *Cell Tissue Res.* **238**, 489 (1984); P. Milde et al., *J. Invest. Dermatol.* **97**, 230 (1991)]. Außerdem zeigen in vitro-Befunde an isolierten Haarfollikelkeratinozyten einen proliferationsinhibierenden und differenzierungsstimmulierenden Einfluß von 1,25-$(OH)_2D_3$.

Aus klinischen Beobachtungen ist bekannt, daß die Vitamin $D_3$-resistente Rachitis häufig mit einer Alopezie einhergeht, die sich im frühen Kindesalter ausprägt. Experimentelle Befunde zeigen, daß die Vitamin $D_3$-Bindungsstelle des VDR bei dieser Erkrankung mutiert, d. h. defekt ist [K. Kristjansson et al., *J Clin. Invest.* **92**, 12 (1993)]. Keratinozyten, die aus den Haarfollikeln dieser Patienten isoliert wurden, reagieren in vitro nicht auf die Zugabe von 1,25-$(OH)_2D_3$ [S. Arase et al., *J. Dermatol. Science* **2**, 353 1991)].

Aus diesen Befunden läßt sich eine entscheidende Rolle von 1,25-$(OH)_2D_3$ auf die Regulation des Haarwachstums ableiten.

Daher eignen sich diese Analoga besonders zur Herstellung von Arzneimitteln zur Behandlung von Erkrankungen, die mit einem gestörten Haarwachstum einhergehen (androgenetische Alopezie, Alopezia areata/totalis, chemotherapie-induzierte Alopezie), oder zur Unterstützung des physiologischen Haarwachstums ohne die Nebenwirkungen des Calcitriols (insbesondere Hypercalcämie) hervorzurufen.

Die senile und postmenopausale Osteoporose ist gekennzeichnet durch einen erhöhten Knochenumsatz mit einer insgesamt negativen Bilanz. Aufgrund des Knochenschwundes insbesondere von trabekulärem Knochen kommt es in verstärktem Maße zu Knochenbrüchen. Durch die fördernde Wirkung von Calcitriol sowohl auf die Anzahl als auch die Syntheseleistung von knochenneubildenden Zellen (Osteoblasten) eignen sich die erfindungsgemäßen Substanzen zur Therapie und Prophylaxe der senilen und postmenopausalen Osteoporose (EP 0 634 173 A1), der steroidinduzierten Osteoporose sowie zur beschleunigten Einheilung von Gelenkplastiken ohne die Nebenwirkungen des Calcitriols (insbesondere Hypercalcämie) hervorzurufen. Für die Therapie der verschiedenen Formen der Osteoporose können sie vorteilhaft mit Estradiol oder anderen Abkömmlingen des Östrogens kombiniert werden.

Schließlich konnte gezeigt werden, daß Calcitriol die Synthese eines Wuchsstoffes für Nervenzellen (nerve growth factor) steigert [M.S. Saporito et al. *Brain Res.* **633**, 189 (1994)]. Daher eignen sich die erfindungsgemäßen Verbindungen auch zur Behandlung von degenerativen Erkrankungen des peripheren und zentralen Nervensystems, wie der Alzheimerschen Erkrankung und der amyotrophen Lateralsklerose.

Es wurde außerdem gefunden, daß bestimmte Verbindungen der allgemeinen Formel **I** in HL 60-Zellen überraschenderweise die Wirkung von Calcitriol antagonisieren.

Solche Verbindungen können bei der Therapie von Hypercalcämien eingesetzt werden, wie zum Beispiel bei Hypervitaminose D oder Intoxikation mit Calcitriol und calcitriolartig wirksamen Substanzen, oder bei erhöhter extrarenaler Calcitriolsynthese bei granulomatösen Erkrankungen (Sarkoidose, Tuberkulose). Auch paraneoplastische Hypercalcämien (zum Beispiel bei osteolytischen Metastasen und Tumoren mit erhöhter Synthese von Parathormonrelated peptide) sowie bei Hypercalcämie bei Hyperparathyreoidismus.

Weiterhin sind Calcitriolantagonisten zur Fertilitätskontrolle einzusetzen. Im Reproduktionstrakt weiblicher und männlicher Tiere wird der Vitamin D-Rezeptor exprimiert. Es ist bekannt, daß die weibliche und männliche Fertilität Vitamin D-defizienter Tiere herabgesetzt ist. Durch kurzfristige Substitution von Calcitriol kann die Reproduktionsleistung erhöht werden. Daher sind Calcitriolantagonisten in der Lage, die weibliche und männliche Fertilität zu beeinflussen.

Da Calcitriol unter bestimmten Bedingungen eine immunsuppressive Wirkung zeigt, sind Calcitriolrezeptorantagonisten auch als Immunstimulantien, z. B. bei Infektabwehrschwäche, AIDS einzusetzen.

Von Calcitriol ist bekannt, daß es das Haarwachstum modulieren kann. Calcitriolantagonisten können daher bei unerwünschtem Haarwachstum, z. B. beim Hirsutismus, therapeutische Verwendung finden.

Eine fördernde Rolle von Vitamin D auf die Bildung von arteriosklerotischen Plaques ist seit langem bekannt. In solchen Gefäßläsionen wird ein Calcitriol-reguliertes Protein, das Osteopontin, vermehrt gefunden, dem eine Rolle bei der Gefäßverkalkung zugeschrieben wird [R. Eisenstein et al. *Arch. Path*. **77**, 27 (1964), L.A. Fitzpatrick et al. *J. Clin. Invest*. **94**, 1597 (1994)]. Deshalb eignen sich Calcitriolantagonisten zur Therapie und Prophylaxe aller Erscheinungsformen der Arteriosklerose.

Schließlich eignen sich Calcitriolantagonisten infolge der Eigenschaft von Calcitriol, unspezifische Immunreaktionen von monocytären Zellen zu steigern, zur Therapie von entzündlichen Erkrankungen insbesondere chronischer Natur, wie rheumatoide Arthritis, Morbus Crohn, Colitis ulcerosa, und granulomatösen Erkrankungen wie Sarkoidose und anderen Fremdkörperreaktionen.
Für alle aufgezählten therapeutischen Anwendungen gilt, daß die erfindungsgemäßen Verbindungen in der Lage sind, in den genannten Krankheitsbildern eine therapeutische Wirkung zu erreichen, ohne die Nebenwirkungen des Calcitriols (insbesondere Hypercalcämie) hervorzurufen.

Die vorliegende Erfindung bezieht sich somit auch auf pharmazeutische Präparate, die mindestens eine Verbindung gemäß der allgemeinen Formel **I** zusammen mit einem pharmazeutisch verträglichen Trägern enthalten.

Die Verbindungen können als Lösungen in pharmazeutisch verträglichen Solventien oder als Emulsionen,

Suspensionen, oder Dispersionen in geeigneten pharmazeutischen Solventien oder Trägern oder als Pillen, Tabletten oder Kapseln, die in an sich bekannter Weise feste Trägerstoffe enthalten, formuliert werden.

Für eine topische Anwendung werden die Verbindungen vorteilhafterweise als Cremes oder Salben oder in einer ähnlichen, zur topischen Anwendung geeigneten Arzneimittelform formuliert. Jede derartige Formulierung kann auch andere pharmazeutisch verträgliche und nichttoxische Hilfsstoffe enthalten, wie z.B. Stabilisatoren, Antioxidantien, Bindemittel, Farbstoffe, Emulgatoren oder Geschmackskorrigentien.

Die Verbindungen werden vorteilhafterweise durch Injektion, intravenöse Infusion in geeigneter steriler Lösungen, als Aerosol über Bronchien und Lunge oder als orale Dosierung über den Ernährungstrakt oder topisch in der Form von Cremes, Salben, Lotions oder geeigneter transdermaler Pflaster appliziert, wie in der EP-A 0 387 077 beschrieben ist.

| | |
|---|---|
| Die tägliche Dosis liegt bei | 0,1 µg/Patient/Tag - 1000 µg/Patient/Tag, |
| vorzugsweise bei | 1,0 µg/Patient/Tag - 500 µg/Patient/Tag. |

**Verfahren zur Herstellung der erfindungsgemäßen Verbindungen**

Die Herstellung der Vitamin D-Derivate der allgemeinen Formel **I** erfolgt erfindungsgemäß aus einer Verbindung der allgemeinen Formel **II**,

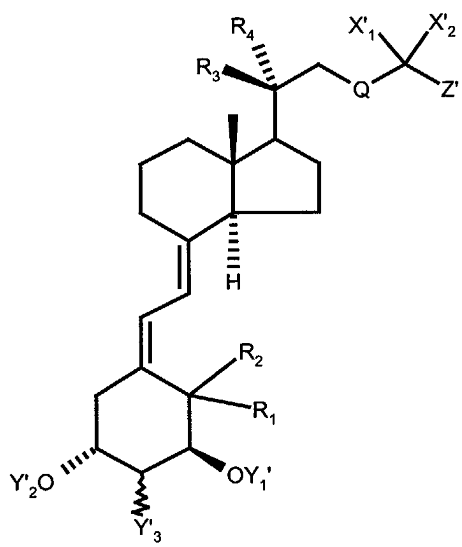

**II**

worin $Y'_1$ und $Y'_2$ Hydroxyschutzgruppen bedeuten und $Y'_3$ ein Wasserstoffatom, ein Halogenatom oder eine geschützte Hydroxygruppe ist.

$X'_1$, $X'_2$ und Z' unterscheiden sich von $X_1$, $X_2$ und Z dadurch, daß eventuell vorliegende Hydroxy- oder Ketogruppen in geschützter Form vorliegen können.

Bei den Schutzgruppen handelt es sich vorzugsweise um alkyl-, aryl- oder gemischt alkylarylsubstituierte Silylgruppen, z.B. die Trimethylsilyl- (TMS), Triethylsilyl- (TES), tert.-Butyldimethylsilyl- (TBDMS), tert.-Butyldiphenylsilyl- (TBDPS) oder Triisopropylsilylgruppen (TIPS) oder eine andere gängige Hydroxyschutzgruppe (Trimethylsilylethoxymethyl-, Methoxymethyl-, Methoxyethoxymethyl-, Ethoxyethyl-, Tetrahydrofuranyl- Tetrahydropyranyl-Gruppen) sowie Acetyl-, Propionyl- oder Pivaloylgruppen, für die Ketogruppen handelt es sich vorzugsweise um Ketale (1,3-Dioxolane, 1,3-Dioxane, Dialkoxyketale), (siehe T.W. Greene, P.G.M. Wuts "Protective Groups in Organic Synthesis", 2nd Edition, John Wiley & Sons, 1991).

Durch gleichzeitige oder sukzessive Abspaltung der Hydroxy- sowie Ketoschutzgruppen und gegebenenfalls durch partielle, sukzessive oder vollständige Veresterung der freien Hydroxylgruppen wird die Verbindung der allgemeinen Formel **II** in eine Verbindung der allgemeinen Formel **I** überführt.

Im Falle der Silylschutzgruppen oder der Trimethylsilylethoxymethylgruppe werden zu deren Abspaltung Tetrabutylammoniumfluorid, Fluorwasserstoffsäure oder Fluorwasserstoffsäure/Pyridin oder saure Ionentauscher verwendet. Im Falle von Ethergruppen (Methoxymethyl-, Methoxyethoxymethyl-, Ethoxyethyl-, Tetrahydropyranylether) und Ketalen werden diese unter katalytischer Einwirkung von Säure, beispielsweise p-Toluolsulfonsäure, Pyridinium-p-toluolsulfonat, Essigsäure, Salzsäure, Phosphorsäure oder einem sauren Ionenaustauscher abgespalten. Estergruppen werden hingegen im basischen Milieu (Natriumcarbonat, Kaliumcarbonat, Natriumhydroxid, Kaliumhydroxid in Wasser, Ethanol, Methanol oder Gemischen dieser Lösungsmittel) hydrolysiert.

Die Veresterung der freien Hydroxygruppen kann falls gewünscht nach gängigen Verfahren mit den entsprechenden Carbonsäurechloriden, -bromiden oder -anhydriden erfolgen.

Die Herstellung der Ausgangsverbindungen für die allgemeine Formel **II**, in denen Q mindestens eine Ethylengruppe bedeutet, geht je nach letztendlich gewünschtem Substitutionsmuster in der 20-Position von verschiedenen Startverbindungen aus. Für die Verbindungen mit natürlicher Konfiguration an C-20 verwendet man das bekannte CD-Fragment **III** als Ausgangsmaterial [H.H. Inhoffen, G. Quinkert, S. Schütz, G, Friedrich, E. Tober *Chem. Ber.* **91,** 781-791 (1958)].

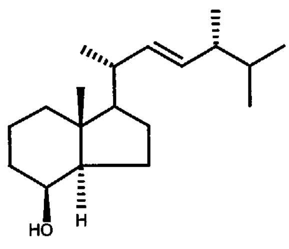

**III**

Durch Einführung einer Schutzgruppe gelangt man zur Verbindung der allgemeinen Formel **IV**,

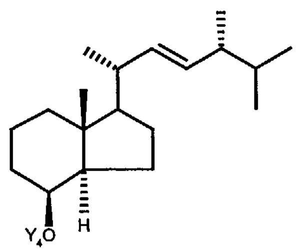

**IV**

wobei $Y_4$ unter anderem eine trialkylsubstituierte oder eine gemischt arylalkylsubstituierte Silylgruppe oder eine Tetrapyranyl- oder Tetrafuranylgruppe bedeuten kann. Ozonolytische Spaltung der Seitenkettendoppelbindung gefolgt von reduktiver Aufarbeitung (z.B. Natriumborhydrid) ergibt die Verbindung der allgemeinen Formel **V**.

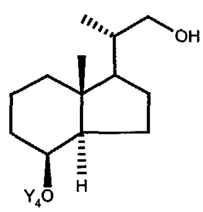

**V**

Die freie Hydroxygruppe kann nun in eine Fluchtgruppe überführt werden, wobei die Verbindung der allgemeinen Formel **VI** entsteht,

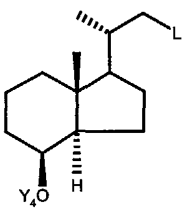

**VI**

für die gilt, daß L für eine beliebige Fluchtgruppe insbesondere für ein Halogenatom (Fluor, Chlor, Brom, Iod) oder ein Mesylat, Tosylat, Triflat oder Nonaflat steht.

Die Verbindung der Formel VI eröffnet über den unten beschriebenen Propargylalkohol VII und die Überführung in den Alkohol VIII und Oxidation zum Aldehyd IX einen neuen bisher nicht bekannten Zugang zum weiter unten beschriebenen Keton XIII, das ein wichtiges Ausgangsmaterial für die Synthese von Vitamin D-Derivaten nach De Luca darstellt [H.F. DeLuca et al. *Tetrahedron Lett.* **32,** 7663 (1991); H.F. DeLuca et al. *J. Med. Chem.* **37,** 3730 (1994)].

Somit betrifft die vorliegende Erfindung auch ein Verfahren zur Herstellung des Ketons XIII über die neuen Zwischenstufen der Formeln VII, VIII und IX. Der Syntheseweg läßt sich für eine andere Kettenlänge beliebig abwandeln, indem ein entsprechendes geschütztes Alkinol eingesetzt wird. Exemplarisch wird im folgenden der Syntheseweg für eine Verbindung, in der Q eine Ethylengruppe bedeutet, beschrieben.

Umsetzung der Verbindung der allgemeinen Formel VI mit einem geschützen Propargylalkohol, den man zuvor mit einer Base (z.B. Natriumhydrid, Kaliumhydrid, Butyllithium, Natriumamid) deprotoniert hat, liefert die Verbindung der allgemeinen Formel VII,

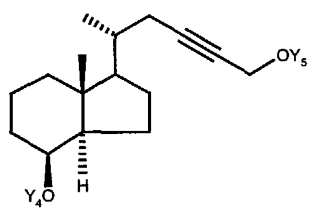

**VII**

für die gilt, daß $Y_5$ eine Tetrahydropyranyl-, eine Benzylgruppe oder eine verwandte Schutzgruppe darstellen soll. Hydrierung der Dreifachbindung und des Benzylethers oder gegebenenfalls gefolgt von Spaltung des Tetrahydropyranylethers unter Säureeinwirkung (p-Toluolsulfonsäure, Pyridinium-p-Toluolsulfonat, Essigsäure, Dimethylaluminiumchlorid, Methylaluminiumdichlorid usw.) ergibt nun die Verbindung der allgemeinen Formel VIII, deren freie Hydroxygruppe mit einem Oxidationsmittel (Pyridiniumchlorochromat, Pyridiniumdichromat, Swern-Bedingungen, Collins-Bedingungen) in den Aldehyd der allgemeinen Formel IX überführt wird.

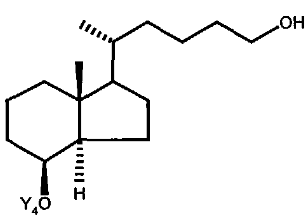

**VIII**

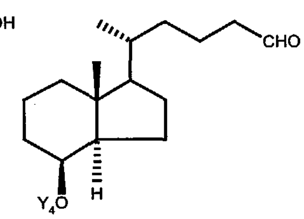

**IX**

Durch Umsetzung mit einem beliebigen Nukleophil Nu, das mit dem Aldehyd zur Reaktion gebracht werden kann, wie z.B. Anionen gegebenenfalls Sauerstoff- oder Schwefelatome enthaltender organischer Reste, gegebenenfalls substituierte, gegebenenfalls an empfindlichen funktionellen Gruppen geschützte, Grignard-Reagenzien oder Lithium-Verbindungen, die nach literaturbekannten Methoden hergestellt werden können, erhält man eine Verbindung der allgemeinen Formel Xa als Gemisch der diastereomeren Alkohole.

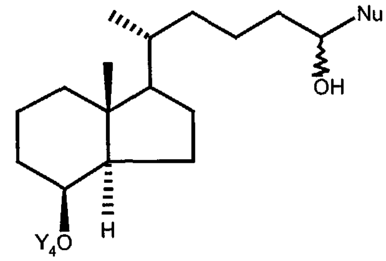

**Xa**

Für die Synthese der erfindungsgemäßen Verbindungen erhält man durch Umsetzung mit einer nukleophilen Form von Z mit der Bezeichnung Z', bevorzugt mit einer metallierten aromatischen oder heteroaromatischen Verbindung die Verbindung der allgemeinen Formel X

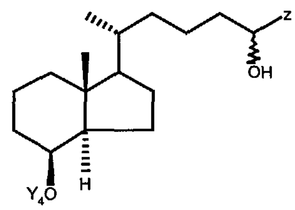

X

Z kann jeder der für Z definierten Reste sein, bevorzugt Furan, Thiophen, Oxazol, Thiazol, Imidazol, Pyrazol, Pyrrol, Benzofuran, Benzothiophen, Benzoxazol, Benzothiazol, Benzimidazol, Indol oder Phenyl bedeuten, wobei die Ringe einen oder mehrere Substituenten, an beliebigen Positionen, tragen können. Als Substituenten kommen Fluor-, Chlor-, Brom- oder Iodatome, eine oder mehrere Hydroxygruppen, eine oder mehrere $COOR_6$-Gruppen, eine oder mehrere $C_1$-$C_5$-Alkylgruppen, die ihrerseits substituiert sein können durch ein oder mehrere Fluor-, Chlor-, Brom- oder Iodatome, $C_1$-$C_6$-Alkoxygruppen und/oder $COOR_6$-Gruppen (und $R_6$ als $C_1$-$C_6$-Alkylgruppe, eine Benzylgruppe oder eine Phenylgruppe definiert ist). Die Aromaten werden durch Wasserstoff-Lithiumaustausch, Orthometallierung, Halogen-Lithiumaustausch (Verwendung von n-Butyllithium, s-Butyllithium, t-Butyllithium, Methyllithium) oder Umsetzung der Halogenverbindungen mit Magnesium oder Zink in die metallierten Derivate überführt. Die Anknüpfung an das CD-Fragment erfolgt immer an der metallierten Position mit Ausnahme der Oxazolringe, die durch Umlagerung und Recyclisierung an der 4-Position angreifen [G. Boche et al. *Chem. Ber./Receuil* **130**, 1213 (1997)].

Überführung der freien Hydroxygruppe in Acetat- (R'=Me), Propionat- (R'=Et) oder Pivaloat- (R'=*t*-Bu) Gruppierungen ergibt Verbindungen der allgemeinen Formel **XI**, die durch Spaltung der Cyclohexanol-Schutzgruppen $Y_4$ in eine Verbindung der allgemeinen Formel **XII** überführt wird. Bedeutet $Y_4$ eine Silylgruppe, so kann deren Abspaltung z.B. unter Verwendung von Tetrabutylammoniumfluorid, Fluorwasserstoff, Fluorwasserstoff/Pyridin-Komplex erfolgen, bedeutet $Y_4$ hingegen eine Tetrahydropyranyl- oder Tetrahydrofuranylgruppe, so kann deren Spaltung unter sauren Bedingungen vollzogen werden. Anschließende Oxidation der freien Hydroxygruppe mit einem Oxidationsmittel (Pyridiniumchlorochromat, Pyridiniumdichromat, Swern-Bedingungen, Collins-Bedingungen) liefert die Verbindung der allgemeinen Formel **XIII**.

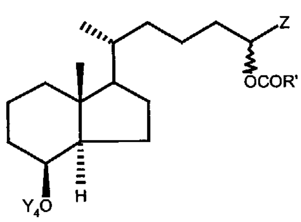

**XI**

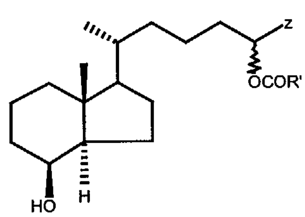

**XII**

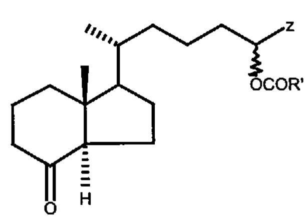

**XIII**

Umsetzung des Ketons der allgemeinen Formel **XIII** mit einem der bekannten Phosphinoxide **XIV, XV** oder **XVI** [**XIV**: M.R. Uskokovic et al. *Tetrahedron Lett.* **33**, 7701 (1992), A. Mourino et al. *Tetrahedron Lett.* **38**, 4713 (1997), **XV**: H.F. DeLuca et al. *Tetrahedron Lett.* **32**, 7663 (1991), **XVI**: H.F. DeLuca et al. *J. Med. Chem.* **37**, 3730 (1994)],

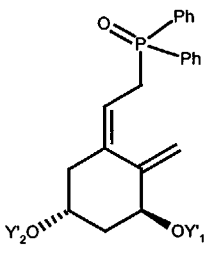

**XIV**

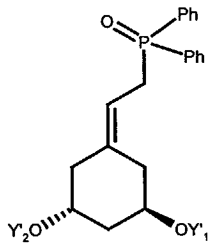

**XV**

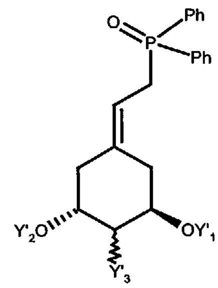

**XVI**

wobei $Y'_1$ und $Y'_2$ alkyl- oder gemischt arylalkylsubstituierte Silylgruppen drastellen (vorzugsweise *tert*.-Butylydimethylsilyl-, *tert*.-Butyldiphenylsilyl-, Trimethylsilyl-, Triethylsilyl-, Triisopropylsilylgruppen) und $Y'_3$ die entsprechende Silyloxygruppierung bedeutet, liefert die Vitamin D-Systeme der allgemeinen Formeln **XVII, XVIII** und **XIX**.

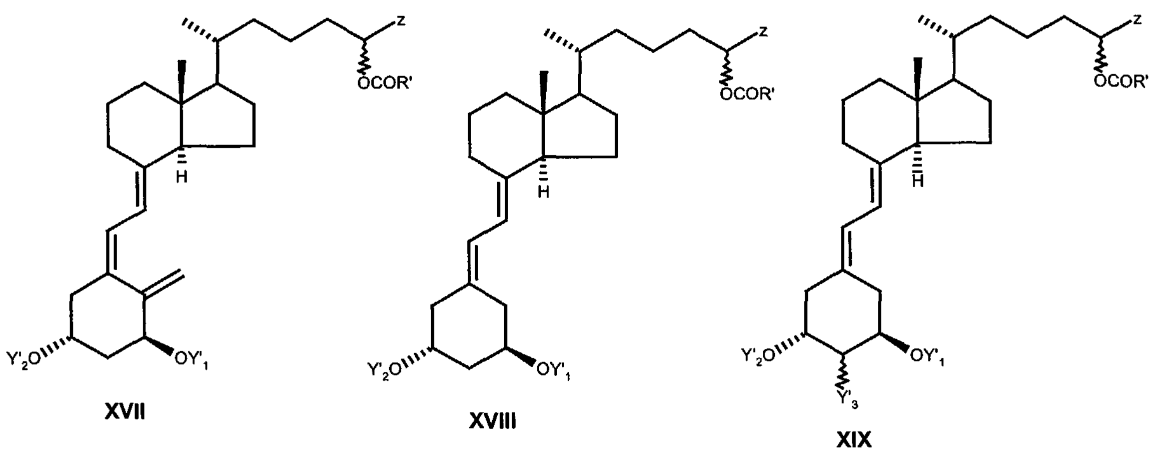

**XVII** **XVIII** **XIX**

Die Verbindungen der allgemeinen Formeln **XVII**, **XVIII** und **XIX** stellen Sonderfälle der allgemeinen Formel **II** dar und werden, wie vorstehend beschrieben, in Verbindungen der allgemeinen Formel **I** überführt.

Insbesondere kann die Hydroxygruppe in Position C-24 in Freiheit gesetzt und mit einem Oxidationsmittel (z. B. Pyridiniumchlorochromat, Pyridiniumdichromat, Collins-Reagenz, Swern-Bedingungen, Mangandioxid) in das korrespondierende Keton überführt werden, das gleichfalls einen Sonderfall der allgemeinen Formel **II** darstellt. Die C-24-Alkohole bzw. das C-24-Keton sind unter bekannten Bedingungen in Halogenide oder Dihalogenide überführbar. Ferner besteht die Möglichkeit der reduktiven Entfernung der Hydroxy-, Keto- oder Halogeneinheiten.

Jegliche Manipulationen der funktionellen Gruppen in der Seitenkette können auch auf früheren Stufen vollzogen werden.

Für die Herstellung von Verbindungen der allgemeinen Formel **II** mit verändertem Substitutionsmuster an C-20 wird der Alkohol der allgemeinen Formel **V** zum Aldehyd der allgemeinen Formel **XX** mit einem Oxidationsmittel (z. B. Pyridiniumchlorochromat, Pyidiniumdichromat, Collins-Reagenz, Swern-Bedingungen, Dess-Martin-Bedingungen) oxidiert . Dieser kann wie beschrieben [DE 42 20 757, 20-epi: M.J. Calverley *Bioorg. Med. Chem. Lett.* **3**, 1845 (1993)] in Verbindungen der allgemeinen Formel **XXI** überführt werden, in der $R_3$ und $R_4$ die schon genannten Bedeutungen haben. Anschließend erfolgt die Reduktion mit einem Reduktionsmittel (z.B. Natriumborhydrid, Lithiumaluminiumhydrid, Diisobutylaluminiumhydrid) zum Alkohol der allgemeinn Formel **XXII**, der wie vorstehend beschrieben weiter umgesetzt wird.

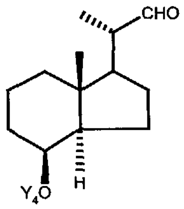

XX

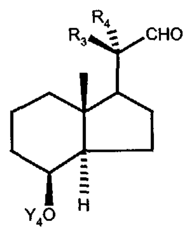

XXI

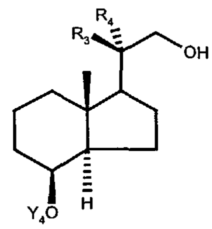

XXII

Für Verbindungen der allgemeinen Formel **II**, für die gilt, daß Q eine Methylengrupppe ist, geht man vom bekannten Vitamin D-Aldehyd **XXIII** aus, der wie zuvor beschrieben (DE 196 19 036), an Position 20 modifiziert werden kann. Exemplarisch wird die weitere Umsetzung am Aldehyd mit natürlicher Konfiguration an C-20 beschrieben.

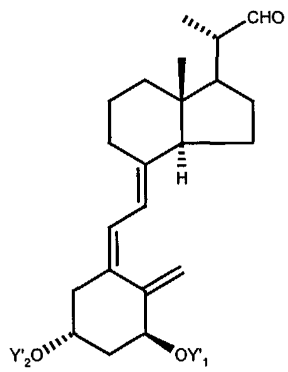

XXIII

Mit einem Reduktionsmittel (z.B. Natriumborhydrid, Lithiumaluminiumhydrid, Diisobutylaluminiumhydrid) erhält man den Alkohohol der allgemeinen Formel **XXIV**, der wie vorstehend beschrieben in eine Fluchtgruppe überführt wird, wobei die Verbindung der allgemeinen Formel **XXV** anfällt.

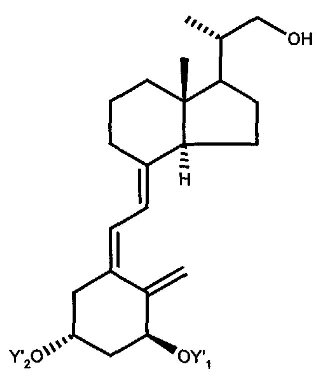

XXIV

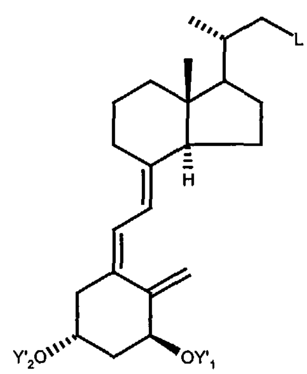

XXV

Reaktion der Verbindung der allgemeinen Formel **XXV** mit dem durch eine Base (z.B. Lithiumdiisopropylamid, Natriumhexamethyldisilazid, Lithiumhexamethyldisilazid, Kaliumhexamethyldisilazid) deprotonierten Acetonitrils ergibt eine Verbindung der allgemeinen Formel **XXVI**, die durch Reduktion mit einem Reduktionsmittel (z.B. Diisobutylaluminiumhydrid) in eine Verbindung der allgemeinen Formel **XXVII** überführt wird.

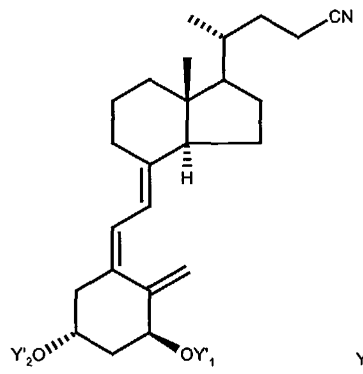

XXVI

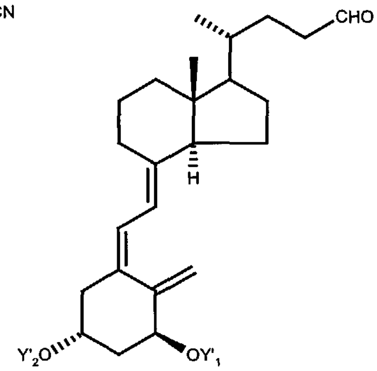

XXVII

Durch Umsetzung mit dem Nukleophil einer carbocyclischen, heterocyclischen, bevorzugt einer metallierten aromatischen oder heteroaromatischen Verbindung erhält man, wie schon vorher gezeigt, Verbindungen der allgemeinen Formel **XXVIII**,

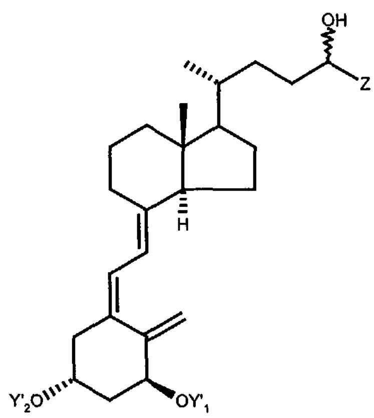

XXVIII

welche als Spezialfall der allgemeinen Formel **II** zu betrachten sind und wie beschrieben in Verbindungen der allgemeinen Formel **I** zu überführen sind. Insbesondere kann die Hydroxygruppe in Position C-24 mit einem Oxidationsmittel (z.B. Pyridiniumchlorochromat, Pyridiniumdichromat, Collins-Reagenz, Swern-Bedingungen, Mangandioxid) in das korrespondierende Keton überführt werden, das gleichfalls einen Sonderfall der allgemeinen Formel **II** darstellt. Die C-24-Alkohole bzw. das C-24-Keton sind unter bekannten Bedingungen in Halogenide oder Dihalogenide überführbar. Ferner besteht die Möglichkeit der reduktiven Entfernung der Hydroxy-, Keto- oder Halogeneinheiten.

Sollen Verbindungen der allgemeinen Formel **I** generiert werden, für die gilt $Y_3$ ist ein Halogenatom (z.B. Fluor-, Chlor- oder Bromatom), so muß ein A-Baustein der allgemeinen Formel **XXX**, wobei $Y_3$ die oben genannte Bedeutung haben soll, aus dem literaturbekannten Cyclohexan-Derivat **XXIX** [J.-L. Montchamp, J.W. Frost *J. Am. Chem. Soc.* **113**, 6296 (1991)] durch Hanessian-Reaktion synthetisiert werden.

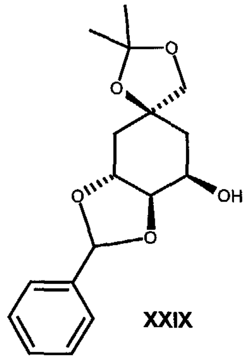

XXIX

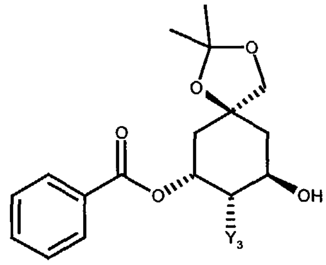

XXX

Einführung einer Schutzgruppe für die freie Hydroxygruppe ergibt die Verbindung der allgemeinen Formel **XXXI**, die im sauren Milieu wie üblich in das Diol **XXXII** und durch anschließende Diolspaltung (Natriumperiodat, Periodsäure) in das Keton **XXXIII** überführt wird, wobei die Definitionen für $Y'_1$ und $Y_3$ schon angeben wurden.

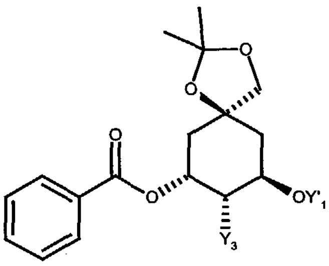

XXXI

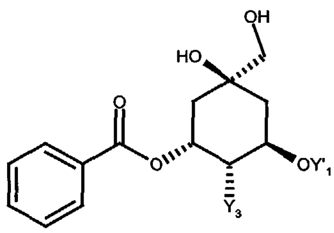

XXXII

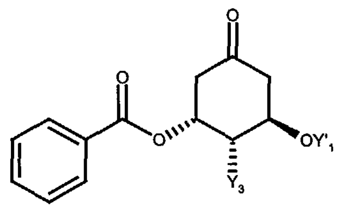

XXXIII

Zum Aufbau des Vitamin D-Systems wird für $Y_3$ = Halogen ein neuer Weg beschritten, der jedoch für alle möglichen Substituenten für $Y_3$ (auch $Y_3$ = ein Wasserstoffatom), sowie auch für andere Substitutionsmuster am A-Ring (z.B. statt $OY_1$ in der 1-Position Halogen, $(CH_2)_n$-OH, oder $(CH_2)_n$-O(CO)$R_5$ mit $R_5$ = aliphatischer $C_1$-$C_{12}$ Alkylrest, der gegebenenfalls unterbrochen ist von 1-2 Sauerstoffatomen, 1-2 Schwefelatomen und/oder 1-2 NH-Gruppen und/oder gegebenenfalls substituiert ist durch 1-2 Hydroxygruppen, 1-2 Aminogruppen, 1-2 SH-Gruppen, 1-2 COOH-Gruppen und/oder 1-2 Phenylgruppen, mit n = 0-4, oder ein aromatischer Rest mit 5 bis 12-C-Atomen), und auch beliebige Seitenketten in 17-Position mit gegebenenfalls geschützt vorliegenden Hydroxygruppen und/oder Ketogruppen ebenfalls anwendbar ist.

Die Umsetzungen werden hier exemplarisch am Keton der allgemeinen Formel **XIII** beschrieben, sind aber für andere Seitenkettenvarianten ebenfalls gültig:
Durch Peterson-Olefinierung (Umsetzung mit Trimethylsilyessigester in Gegenwart einer Base wie z.B. *n*-Butyllithium oder Lithiumdiisopropylamid) erhält man aus dem Keton **XIII** die Verbindung der allgemeinen Formel **XXXIV**, deren Estergruppe durch Reduktion mit einem Reduktionsmittel (z.B. Diisobutylaluminiumhydrid, Lithiumaluminiumhydrid) in den Alkohol der allgemeinen Formel **XXXV** überführt wird. Die Umwandlung der Hydroxygruppe in ein Diphenylphosphinoxid-Derivat der allgemeinen Formel **XXXVI** erfolgt nach Standardbedingungen über ein Allylhalogenid (Chlorid, Bromid) oder ein intermediäres Tosylat oder Mesylat.

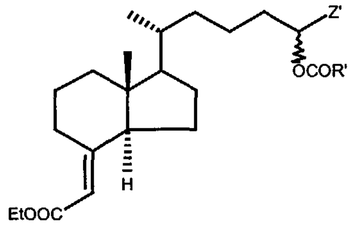

XXXIV

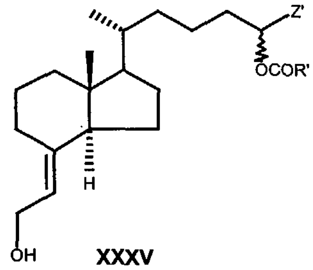

XXXV

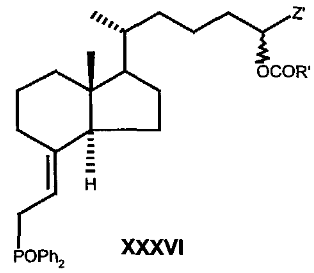

XXXVI

Die Verknüpfung mit dem Keton der allgemeinen Formel **XXXIII** ergibt schließlich das Vitamin D-System der allgemeinen Formel **II** mit den schon genannten Definitionen für $Y_3$. Die Überführung in eine Verbindung der allgemeinen Formel **I** erfolgt wie vorher beschrieben durch Abspaltung der Schutzgruppen.

Somit ist Gegenstand der vorliegenden Erfindung auch ein neues Verfahren zur Herstellung von Vitamin D-Derivaten der allgemeinen Formel IIa,

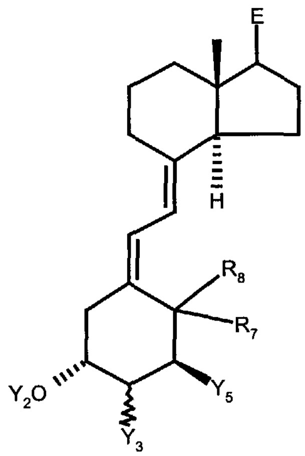

**IIa**

worin
E jede beliebige Seitenkette bedeutet,

- $R_7$, $R_8$ unabhängig voneinander ein Wasserstoffatom, eine Methylgruppe, oder gemeinsam eine exocyclische Methylengruppe oder einen Cyclopropylring bedeuten,

- $Y_2$ ein Wasserstoffatom oder eine Gruppe $-(CO)R_5$ bedeutet,

- $Y_3$ ein Wasserstoffatom, eine Hydroxygruppe, ein Halogenatom, eine Gruppe $-O(CO)R_5$ oder eine $OR_5$-Gruppe bedeutet,
  wobei

  - $R_5$ für einen aliphatischen $C_1$-$C_{12}$ Alkylrest, der gegebenenfalls unterbrochen ist von 1-2 Sauerstoffatomen, 1-2 Schwefelatomen und/oder 1-2 NH-Gruppen und/oder gegebenenfalls substituiert ist durch 1-2 Hydroxygruppen, 1-2 Aminogruppen, 1-2 SH-Gruppen, 1-2 COOH-Gruppen und/oder 1-2 Phenylgruppen, oder für einen aromatischen Rest mit 5 bis 12-C-Atomen steht,

- $Y_5$ ein Fluoratom, eine $(CH_2)_n$-OH Gruppe oder eine $(CH2)_n$-$O(CO)R_5$-Gruppe bedeutet,
  wobei n = 0 bis 4 bedeutet.

und gegebenenfalls vorhandene Hydroxygruppen gegebenenfalls geschützt vorliegen, das dadurch gekennzeichnet ist, daß ein Keton der allgemeinen Formel XIIIc

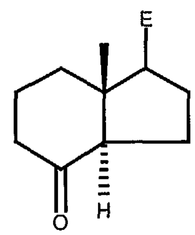

XIIIc

worin
E eine beliebige Seitenkette bedeutet,
und gegebenenfalls vorhandene Ketogruppen und/oder Hydroxygruppen geschützt vorliegen,
durch Umsetzung mit Trimethylsilylessigester in Gegenwart einer Base wie z.B. n-Butyllitium oder Lithiumaluminiumhydrid oder mit einem geeigneten Wittigreagenz in einem aprotischen Lösungsmittel wie Toluol, Tetrahydrofuran, Diethylether oder Dioxan in eine Verbindung der allgemeinen Formel XXXIVc

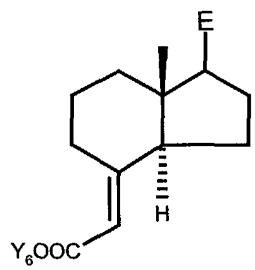

XXXIVc

worin
$Y_6$ eine $C_1$-$C_6$-Alkylgruppe, eine Benzylgruppe, oder eine Phenylgruppe bedeutet, überführt wird,
die Estergruppe durch Reaktion mit einem Reduktionsmittel wie Dibah, Lithiumaluminiumhydrid, Diboran oder RedAl in Hexan, Toluol, Tetrahydrofuran, Diethylether oder Dioxan in den Allylalkohol der allgemeinen Formel XXXVc

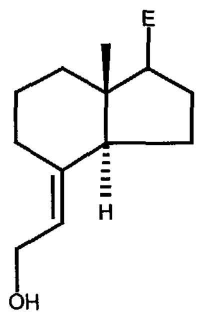

XXXVc

überführt wird,
der Allylalkohol in an sich bekannter Weise in eine Verbindung der allgemeinen Formel XXXVc

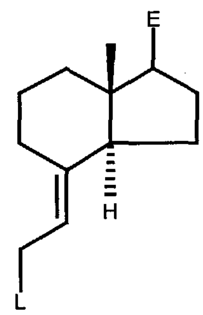

**XXXVc**

worin
L jede beliebige Abgangsgruppe bedeutet (Halogen, Mesylat, Tosylat, Triflat, Nonaflat), überführt wird, die isoliert oder gegebenenfalls in situ erzeugt und sofort weiter umgesetzt wird zu einem Wittig Reagenz der allgemeinen Formel XXXVIc,

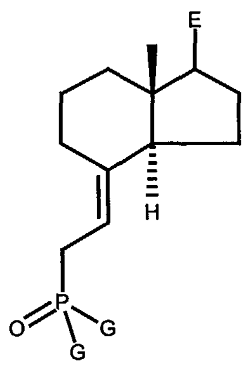

**XXXVIc**

worin
G eine $C_1$-$C_{10}$-Alkylgruppe, eine $C_1$-$C_{10}$ Alkoxygruppe, einen Phenylrest oder einen Phenoxyrest bedeutet, das dann anschließend unter bekannten Bedingungen mit einem Keton der allgemeinen Formel XXXIIIc

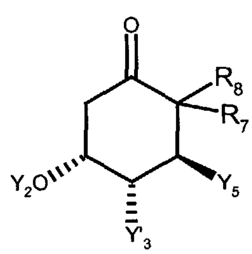

**XXXIIIc**

worin
Y'$_3$ ein Wasserstoffatom, ein Halogenatom oder eine geschützte Hydroxygruppe , eine Gruppe —O(CO)$R_5$ oder eine $OR_5$-Gruppe bedeutet, und $Y_5$, $R_7$ und $R_8$ die oben angegebene Bedeutung hat, umgesetzt wird und falls gewünscht, Schutzgruppen abgespalten werden.

Die Verbindungen der allgemeinen Formel IIa können wie oben beschrieben durch Spaltung der Schutzgruppen in die gewünschten Vitamin D-Derivate überführt werden.

Besonders geeignet ist das erfindungsgemäße Verfahren für die Herstellung von Vitamin D-Derivaten, in denen $Y_3$ ein Halogenatom bedeutet, es ist jedoch nicht darauf beschränkt.
Die Zusätze "a" zur Bezeichnung der Formeln (z.B. XXXIV**a**) soll deutlich machen, daß die Bedeutungen der Reste grundsätzlich dieselben sind wie bei der Formel ohne Zusatz "a", aber empfindliche Gruppen geschützt vorliegen.
Ein alternativer Weg zur Synthese von Verbindungen der allgemeinen Formel **II**, für die gilt, daß $X'_1$ und $X'_2$ Wasserstoffatome bedeuten und Q eine Ethylengruppe darstellt geht man vom Alkohol der allgemeinen Formel **VIII** aus, dessen Hydroxygruppe in eine Fluchtgruppe (z.B. Chlorid, Bromid, Iodid, Tosylat, Mesylat) überführt und mit metallierten aromatischen oder heteroaromatischen Verbindungen umgesetzt wird, wobei Verbindungen der allgemeinen Formel **XXXVII** anfallen,

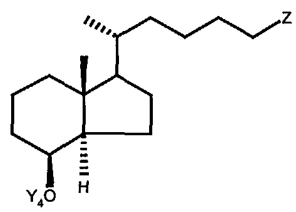

**XXXVII**

deren weitere Umsetzung analog den zuvor beschriebenen Verbindungen erfolgen kann.
Die nachstehenden Beispiele dienen der näheren Erläuterung der Erfindung.

**Beispiele:**

**Synthese der Ausgangsverbindungen (CD-Aldehyd):**

1. [1*R*-[1α[1*R**,4*S**-(*E*)],3aβ,4α,7aα]]-Octahydro-7a-methyl-4-[(triethylsilyl)oxy]-1-(1,4,5-trimethyl-2-hexenyl)-1*H*-inden **2**

Man legt 12,95 g [1*R*-[1α[1*R**,4*S**-(*E*)],3aβ,4α,7aα]]-Octahydro-7a-methyl-1-(1,4,5-trimethyl-2-hexenyl)-1*H*-inden-4-ol **1** in 220 ml Dimethylformamid vor [H.H. Inhoffen et al. *Chem. Ber.* **91,** 781 (1958)] fügt 5,33 g Imidazol und 10,78 ml Chlortriethylsilan hinzu und rührt 24 h bei Raumtemperatur. Es wird mit Natriumchlorid-Lösung gequencht, mit Essigester extrahiert, die organische Phase mit Natriumchlorid-Lösung gewaschen und über Natriumsulfat getrocknet. Nach dem Entfernen des Lösungsmittels reinigt man den Rückstand durch Chromatographie an Silicagel mit Essigester/Hexan, wobei man 14,6 g der Titelverbindung **2** als farbloses Öl erhält.
$^1$H-NMR ($CDCl_3$): δ= 0,58 ppm (q, 6H); 0,82 (d, 3H); 0,84 (d, 3H); 0,93 (t, 9H); 0,96 (d, 3H); 0,97 (s, 3H); 0,98 (d, 3H); 4,05 (m, 1H); 5,19 (m, 2H)

2. [1*R*-[1α(*S**),3aβ,4α,7aα]]-2-[Octahydro-7a-methyl-4-[(triethylsilyl)oxy]-1*H*-inden-1-yl]-1-propanol **3**

Man löst 14,6 g des Silylethers **2** in 300 ml Dichlormethan und 150 ml Methanol, fügt 14,8 ml Pyridin hinzu und kühlt auf -78°C. Bei dieser Temperatur leitet man Ozon ein, welches durch einen Ozongenerator erzeugt worden ist, bis die Lösung blau gefärbt ist. Das überschüssige Ozon wird durch Sauerstoffdurchleitung ausgetrieben und anschließend wird mit 1,61 g Natriumborhydrid versetzt. Man erwärmt auf Raumtemperatur und gießt dann das Reaktionsgemisch in Dichlormethan. Die organische Phase wird mit Natriumchlorid-Lösung gewaschen, über Natriumsulfat getrocknet und eingeengt. Der Rückstand wird durch Chromatographie an Silicagel mit Essigester/Hexan chromatographiert, wobei man 10,1 g der Titelverbindung **3** als farbloses Öl erhält.
$^1$H-NMR ($CDCl_3$): δ= 0,57 ppm (q, 6H); 0,92 (s, 3H); 0,96 (t, 9H); 1,02 (d, 3H); 3,38 (dd, 1H); 3,63 (d, 1H); 4,05 (m, 1H)

3. [1*R*-[1α(*S**),3aβ,4α,7aα]]-2-[Octahydro-7a-methyl-4-[(triethylsilyl)oxy]-1*H*-inden-1-yl]propyl-(4-methylbenzensulfonat) **4**

Man löst 9,1 g des Alkohols **3** in 135 ml Pyridin, kühlt auf 0°C und gibt dann 7,8 g p-Toluolsulfonylchlorid zu. Es wird über Nacht bei 0°C gerührt und anschließend mit Natriumhydrogencarbonat-Lösung vorsichtig gequencht. Man extrahiert mit Essigester, wäscht die organische Phase mit Natriumchlorid-Lösung, trocknet über Natriumsulfat und entfernt das Solvens im Vakuum. Der Rückstand wird an Silicagel mit Essigester/Hexan chromatographiert, wobei

man 13,3 g der Titelverbindung **4** als farbloses Öl erhält.
$^1$H-NMR ($CDCl_3$): δ= 0,56 ppm (q, 6H); 0,87 (s, 3H); 0,96 (t, 9H); 0,97 (d, 3H); 2,47 (s, 3H); 3,80 (dd, 1H); 3,97 (d, 1H); 4,01 (m, 1H); 7,35 (d, 2H); 7,80 (d, 2H)

4. [1*R*-[1α(*R**),3aβ,4α,7aα]]-Octahydro-7a-methyl-1-[5-[(tetrahydro-2*H*-pyran-2-yl)oxy]-1-methyl-3-pentinyl-4-[(triethylsilyl)oxy]-1*H*-inden **5**

Man legt 15,4 g Propargyl-THP-Ether in 350 ml Dioxan vor und tropft bei 10-15°C vorsichtig 43,8 ml *n*-Butyllithium (2,5 M in Hexan) zu. Nach einer Stunde tropft man 13,3 g des Tosylates **4** in 100 ml Dioxan hinzu und erhitzt für 48 h zum Sieden. Es wird anschließend mit Natriumchlorid-Lösung gequencht, mit Essigester extrahiert, die organische Phase mit Natriumchlorid-Lösung gewaschen und über Natriumsulfat getrocknet. Nach dem Entfernen des Lösungsmittels wird der Rückstand an Silicagel mit Essigester/Hexan chromatographiert, wobei man 12,1 g der Titelverbindung **5** als farbloses Öl erhält.
$^1$H-NMR ($CDCl_3$): δ= 0,57 ppm (q, 6H); 0,92 (s, 3H); 0,96 (t, 9H); 1,06 (d, 3H); 3,56 (m, 1H); 3,88 (m, 1H); 4,04 (m, 1H); 4,29 (d, 2H); 4,83 (m, 1H)

5. [1*R*-[1α(*R**),3aβ,4α,7aα]]-Octahydro-7a-methyl-1-[5-[(tetrahydro-2*H*-pyran-2-yl)oxy]-1-methylpentyl-4-[(triethylsilyl)oxy]-1*H*-inden **6**

Man löst 12,6 g des Alkins **5** in 250 ml Essigester, setzt 2,2 g Palladium/Kohle (10%) und 5,06 g Natriumhydrogencarbonat hinzu und hydriert unter Normaldruck in einer Hydrierapparatur. Wenn kein Wasserstoff mehr aufgenommen wird, filtriert man den Ansatz über Celite und engt ein. Der Rückstand wird an Silicagel mit Essigester/Hexan chromatographiert, wobei man 9,9 g der Titelverbindung **6** als farbloses Öl erhält.
$^1$H-NMR ($CDCl_3$): δ= 0,56 ppm (q, 6H); 0,91 (s, 3H); 0,95 (t, 9H); 0,97 (d, 3H); 3,38 (m, 1H); 3,51 (m, 1H); 3,70 (m, 1H); 3,88 (m, 1H); 4,02 (m, 1H); 4,59 (m, 1H)

6. [1*R*-[1α(*R**),3aβ,4α,7aα]]-5-[Octahydro-7a-methyl-4-[(triethylsilyl)oxy]-1*H*-inden-1-yl]-1-hexanol **7**

Man legt 11,4 g des THP-Ethers **6** in 500 ml Dichlormethan vor und tropft 50 ml Dimethylaluminiumchlorid-Lösung hinzu. Nach 2 h bei Raumtemperatur quencht man mit Isopropanol/Wasser (15:85), gibt Toluol hinzu und rührt über Nacht. Anschließend saugt man über Celite ab und engt ein. Der Rückstand wird an Silicagel mit Essigester/Hexan chromatographiert, wobei man 6,2 g der Titelverbindung **7** als farbloses Öl erhält.
$^1$H-NMR ($CDCl_3$): δ= 0,56 ppm (q, 6H); 0,90 (s, 3H); 0,95 (t, 9H); 0,96 (d, 3H); 3,64 (m, 2H); 4,02 (m, 1H)

7. [1*R*-[1α(*R**),3aβ,4α,7aα]]-5-[Octahydro-7a-methyl-4-[(triethylsilyl)oxy]-1*H*-inden-1-yl]hexanal **8**

Es werden 8,0 g des Alkohols **7** in 300 ml Dichlormethan gelöst und anschließend setzt man 6,5 g Pyridiniumchlorochromat hinzu. Es wird 2 h bei Raumtemperatur gerührt. Danach setzt man Diethylether zu, filtriert über Celite und engt ein. Der Rückstand wird an Silicagel mit Essigester/Hexan chromatographiert, wobei man 6,2 g der Titelverbindung **8** als farbloses Öl erhält.
$^1$H-NMR ($CDCl_3$): δ= 0,57 ppm (q, 6H); 0,91 (s, 3H); 0,95 (t, 9H); 0,96 (d, 3H); 4,02 (m, 1H); 9,75 (t, 1H)

**Beispiel 1**

**(7*E*)-(1*R*,3*R*,24a*R*)-24a-(Oxazol-4-yl)-24a-homo-19-nor-9,10-secochola-5,7-dien-1,3,24a-triol 16a und (7*E*)-(1*R*,3*R*,24a*S*)-24a-(Oxazol-4-yl)-24a-homo-19-nor-9,10-secochola-5,7-dien-1,3,24a-triol 16b**

8. Man legt 939 mg Oxazol in 20 ml Tetrahydrofuran vor und kühlt auf -78°C. Anschließend tropft man 5,44 ml *n*-Butyllithium (2,5 M in Hexan) hinzu, rührt 20 min nach und gibt dann 500 mg des Aldehydes **8** in 5 ml Tetrahydrofuran zu. Man rührt über Nacht, wobei sich das Gemisch auf Raumtemperatur erwärmt und quencht dann mit Natriumchlorid-Lösung. Es wird mit Essigester extrahiert, die organische Phase wird mit Natriumchlorid-Lösung gewaschen, über Natriumsulfat getrocknet und das Solvens entfernt. Der Rückstand wird an Silicagel mit Essigester/Hexan chromatographiert, wobei man 234 mg [1*R*-[1α(1*R**),3aβ,4α,7aα]]-α-[4-[Octahydro-7a-methyl-4-[(triethylsilyl)oxy]-1*H*-inden-1-yl]pentyl]oxazol-4-methanol **9** als farbloses Öl erhält.
$^1$H-NMR ($CDCl_3$): δ= 0,57 ppm (q, 6H); 0,86 (d, 3H); 0,92 (s, 3H); 0,99 (t, 9H); 4,02 (m, 1H); 4,70 (m, 1H); 7,60 (s, 1H); 7,90 (s, 1H)
9. Man legt 361 mg des Alkohols **9** in 8 ml Dichlormethan vor, gibt 0,17 ml Triethylamin, 0,12 ml Essigsäureanhydrid und eine Spatelspitze Dimethylaminopyridin hinzu und rührt über Nacht bei Raumtemperatur. Anschließend

wird Natriumchlorid-Lösung zugegeben, mit Essigester extrahiert, die organische Phase mit Natriumhydrogencarbonat-Lösung sowie Natriumchlorid-Lösung gewaschen, über Natriumsulfat getrocknet und eingeengt. Der Rückstand wird an Silicagel mit Essigester/Hexan chromatographiert, wobei man 296 mg [1*R*-[1α(1*R**),3aβ,4α,7aα]]-5-[Octahydro-7a-methyl-4-[(triethylsilyl)oxy]-1*H*-inden-1-yl]-1-(oxazol-4-yl)hexyl-acetat **10** als farbloses Öl erhält.

$^1$H-NMR ($CDCl_3$): δ= 0,57 ppm (q, 6H); 0,86/0,87 (d, 3H); 0,90 (s, 3H); 0,98 (t, 9H); 2,09 (s, 3H); 4,02 (m, 1H); 5,80 (t, 1H); 7,62 (s, 1H); 7,86 (s, 1H)

10. Man legt 281 mg des Acetates **10** in 10 ml Tetrahydrofuran vor, gibt 1 ml Fluorwasserstoff-Pyridin-Komplex zu und rührt über Nacht bei Raumtemperatur. Anschließend quencht man mit Natriumhydrogencarbonat-Lösung, extrahiert mit Essigester, wäscht die organische Phase mit Natriumchlorid-Lösung, trocknet über Natriumsulfat und entfernt das Lösungsmittel. Der Rückstand wird an Silicagel mit Essigester/Hexan chromatographiert, wobei man 193 mg [1*R*-[1α(1*R**),3aβ,4α,7aα]]-1-[5-(Acetyloxy)-1-methyl-5-(oxazol-4-yl)pentyl]octahydro-7a-methyl-1*H*-inden-4-ol **11** als farbloses Öl erhält.

$^1$H-NMR ($CDCl_3$): δ= 0,88/0,89 ppm (d, 3H); 0,91 (s, 3H); 2,09 (s, 3H); 4,07 (m, 1H); 5,80 (t, 1H); 7,61 (s, 1H); 7,85 (s, 1H)

11. Man legt 193 mg des Alkohols **11** in 8 ml Dichlormethan vor, fügt 160 mg Pyridiniumchlorochromat hinzu und rührt 1 h bei Raumtemperatur. Anschließend verdünnt man mit Diethylether, filtriert über Celite und entfernt das Solvens. Der Rückstand wird an Silicagel mit Essigester/Hexan chromatographiert, wobei man 160 mg [1*R*-[1α(1*R**), 3aβ,7aα]]-1-[5-(Acetyloxy)-1-methyl-5-(oxazol-4-yl)pentyl]octahydro-7amethyl-4*H*-inden-4-on **12** als farbloses Öl erhält.

$^1$H-NMR ($CDCl_3$): δ= 0,61 ppm (s, 3H); 0,91/0,92 (d, 3H); 2,10 (s, 3H); 4,07 (m, 1H); 5,80 (t, 1H); 7,62 (s, 1H); 7,85 (s, 1H)

12. Man legt 461 mg [2-[(3*R*-trans)-3,5-Bis[[(1,1-dimethylethyl)dimethylsilyl]oxy]cyclohexyliden]ethyl]diphenylphosphinoxid **13** [H.F. DeLuca et al. *Tetrahedron Lett.* **32**, 7663 (1991), A. Mourino et al. *Tetrahedron Lett.* **38**, 4713 (1997)] in 8 ml Tetrahydrofuran vor und kühlt auf -78°C. Bei dieser Temperatur gibt man 0,39 ml *n*-Butyllithium-Lösung (2,5 M in Hexan) zu und rührt 10 min bei -30°C. Danach tropft man 146 mg des Ketons **12** in 4 ml Tetrahydrofuran zu und rührt 1 h nach. Man quencht mit Natriumchlorid-Lösung, extrahiert mit Essigester, wäscht die organische Phase mit Natriumchlorid-Lösung, trocknet über Natriumsulfat und engt ein. Der Rückstand wird an Silicagel mit Essigester/Hexan chromatographiert, wobei man 180 mg (7*E*)-(1*R*,3*R*)-24a-(Acetyloxy)-1,3-bis[[(1-1-dimethylethyl)dimethylsilyl]oxy]-24a-(oxazol-4-yl)-24a-homo-19-nor-9,10-secochola-5,7-dien **14** als farblosen Schaum erhält.

$^1$H-NMR ($CDCl_3$): δ= 0,06 ppm (s, 12H); 0,52 (s, 3H); 0,89 (s, 18H); 0,90 (d, 3H); 2,09 (s, 3H); 4,08 (m, 2H); 5,80 (t, 1H); 5,81 (d, 1H); 6,18 (d, 1H); 7,61 (s, 1H); 7,87 (s, 1H)

13. Man legt 180 mg des Acetates **14** in 8 ml Methanol vor, gibt 140 mg Kaliumcarbonat zu und rührt 1 h bei Raumtemperatur. Anschließend versetzt man mit Natriumchlorid-Lösung, extrahiert mit Essigester, wäscht die organische Phase mit Natriumchlorid-Lösung, trocknet über Natriumsulfat und entfernt das Lösungsmittel. Der Rückstand wird an Silicagel mit Essigester/Hexan chromatographiert, wobei man 144 mg (7*E*)-(1*R*,3*R*)-1,3-Bis[[(1,1-dimethylethyl)dimethylsilyl]oxy]-24a-(oxazol-4-yl)-24a-homo-19-nor-9,10-secochola-5,7-dien-24a-ol **15** als farblosen Schaum erhält.

$^1$H-NMR ($CD_2Cl_2$): δ= 0,06 ppm (s, 12H); 0,52 (s, 3H); 0,84 (s, 18H); 0,91/0,92 (d, 3H); 4,07 (m, 2H); 4,62 (m, 1H); 5,80 (d, 1H); 6,14 (d, 1H); 7,55 (s, 1H); 7,82 (s, 1H)

14. Man legt 144 mg des Alkohols **15** in 10 ml Tetrahydrofuran vor, gibt 467 mg Tetrabutylammoniumfluorid (Hydrat) hinzu und rührt 2 d bei Raumtemperatur. Anschließend gibt man Natriumhydrogencarbonat-Lösung zu, extrahiert mit Essigester, wäscht die organische Phase mit Natriumchlorid-Lösung, trocknet über Natriumsulfat und engt ein. Der Rückstand wird an Silicagel mit Essigester/Hexan chromatographiert und anschließend werden über HPLC die Diastereomeren (bzgl. C-24a) getrennt, wobei 25 mg der Titelverbindung **16a** und 34 mg der Titelverbindung **16b** als farblose Schäume anfallen.

$^1$H-NMR ($CD_2Cl_2$): **16a:** δ= 0,52 ppm (s, 3H); 0,89 (d, 3H); 3,98 (m, 1H); 4,03 (m, 1H); 4,62 (m, 1H); 5,82 (d, 1H); 6,28 (d, 1H); 7,57 (s, 1H); 7,84 (s, 1H)

**16b**: δ= 0,52 ppm (s, 3H); 0,90 (d, 3H); 3,98 (m, 1H); 4,03 (m, 1H); 4,62 (m, 1H); 5,82 (d, 1H); 6,28 (d, 1H); 7,57 (s, 1H); 7,83 (s, 1H)

**Beispiel 2**

**(5*Z*,7*E*)-(1*S*,3*R*,24a*R*)-24a-(Oxazol-4-yl)-24a-homo-9,10-secochola-5,7,10(19)-trien-1,3,24a-triol 20a und (5*Z*,7*E*)-(1*S*,3*R*,24a*S*)-24a-(Oxazol-4-yl)-24a-homo-9,10-secochola-5,7,10(19)-trien-1,3,24a-triol 20b**

15. Man legt 257 mg [2-[[3*S*-(1Z,3α,5β)]-3,5-Bis[[(1,1-dimethylethyl)dimethylsilyl]oxy]-2-methylencyclohexyliden]ethyl]diphenylphosphinoxid **17** [M.R. Uskokovic et al. *Tetrahedron Let.* **33**, 7701 (1992)] in 6 ml Tetrahydrofuran vor und kühlt auf -78°C. Bei dieser Temperatur gibt man 0,21 ml *n*-Butyllithium-Lösung (2,5 M in Hexan) zu und rührt 10 min bei -30°C. Danach tropft man 80 mg des Ketons **12** in 4 ml Tetrahydrofuran zu und rührt 1 h nach. Man quencht mit Natriumchlorid-Lösung, extrahiert mit Essigester, wäscht die organische Phase mit Natriumchlorid-Lösung, trocknet

über Natriumsulfat und engt ein. Der Rückstand wird an Silicagel mit Essigester/Hexan chromatographiert, wobei man 140 mg (5*Z*,7*E*)-(1*S*,3*R*)-24a-(Acetyloxy)-1,3-bis[[(1,1-dimethylethyl)dimethylsilyl]oxy]-24a-(oxazol-4-yl)-24a-homo-9,10-secochola-5,7,10(19)-trien **18** als farbloser Schaum erhält.
$^1$H-NMR ($CDCl_3$): δ= 0,06 ppm (s, 12H); 0,52 (s, 3H); 0,90 (s, 18H); 0,90 (d, 3H); 2,09 (s, 3H); 4,19 (m, 1H); 4,38 (m, 1H); 4,88 (s, 1H); 5,19 (s, 1H); 5,81 (t, 1H); 6,02 (d, 1H); 6,23 (d, 1H); 7,62 (s, 1H); 7,87 (s, 1H)

16. Man behandelt 140 mg des Acetates **18** analog 13. und erhält 120 mg (5Z,7E)-(1*S*,3*R*)-1,3-Bis[[(1,1-dimethylethyl)dimethylsilyl]oxy]-24a-(oxazol-4-yl)-24a-homo-9,10-secochola-5,7,10(19)-trien-24a-ol **19** als farblosen Schaum.
$^1$H-NMR ($CDCl_3$): δ= 0,07 ppm (s, 12H); 0,52 (s, 3H); 0,89 (s, 18H); 0,90 (d, 3H); 4,19 (m, 1H); 4,38 (m, 1H); 4,87 (s, 1H); 5,20 (s, 1H); 5,81 (t, 1H); 6,02 (d, 1H); 6,25 (d, 1H); 7,60 (s, 1H); 7,88 (s, 1H)

17. Man behandelt 120 mg des Alkohols **19** analog 14. und erhält nach Trennung der Diastereomeren (bzgl. C-24a) über HPLC 14 mg der Titelverbindung **20a** und 18 mg der Titelverbindung **20b** als farblose Schäume.
$^1$H-NMR ($CD_2Cl_2$): **20a:** δ= 0,53 ppm (s, 3H); 0,91 (d, 3H); 4,15 (m, 1H); 4,36 (m, 1H); 4,63 (m, 1H); 4,94 (s, 1H); 5,26 (s, 1H); 6,01 (d, 1H); 6,35 (d, 1H); 7,56 (s, 1H); 7,84 (s, 1H)
**20b:** δ= 0,53 ppm (s, 3H); 0,90 (d, 3H); 4,15 (m, 1H); 4,36 (m, 1H); 4,63 (m, 1H); 4,93 (s, 1H); 5,26 (s, 1H); 6,01 (d, 1H); 6,35 (d, 1H); 7,56 (s, 1H); 7,85 (s, 1H)

### Beispiel 3

**(7*E*)-(1*R*,3*R*,24a*R*)-24a-(Thiazol-2-yl)-24a-homo-19-nor-9,10-secochola-5,7-dien-1,3,24a-triol 27a** und **(7*E*)-(1*R*,3*R*,24a*S*)-24a-(Thiazol-2-yl)-24a-homo-19-nor-9,10-secochola-5,7-dien-1,3,24a-triol 27b**

18. Man legt 3,1 ml 2-Bromthiazol in 40 ml Tetrahydrofuran vor und gibt bei -78°C 13,8 ml *n*-Butyllithium-Lösung (2,5 M in Hexan) zu. Nach 30 min bei dieser Temperatur tropft man 2,52 g des Aldehydes **8** in 10 ml Tetrahydrofuran zu und rührt 1 h nach. Anschließend wird mit Natriumchlorid-Lösung gequencht, mit Essigester extrahiert, die organische Phase mit Natriumchlorid-Lösung gewaschen und über Natriumsulfat getrocknet. Das Lösungsmittel wird entfernt und der Rückstand wird an Silicagel mit Essigester/Hexan chromatographiert, wobei man 2,6 g [1*R*-[1α(1*R**),3aβ,4α,7aα]]-α-[4-[Octahydro-7a-methyl-4-[(triethylsilyl)oxy]-1*H*-inden-1-yl]pentyl]thiazol-2-methanol **21** als farbloses Öl erhält.
$^1$H-NMR ($CDCl_3$): δ= 0,57 ppm (q, 6H); 0,88 (d, 3H); 0,91 (s, 3H); 0,97 (t, 9H); 4,02 (m, 1H); 5,01 (m, 1H); 7,30 (d, 1H); 7,71 (d, 1H)

19. Man behandelt 948 mg des Alkohols **21** analog 9. und erhält 824 mg [1*R*-[1α(1*R**),3aβ,4α,7aα]]-5-[Octahydro-7a-methyl-4-[(triethylsilyl)oxy]-1*H*-inden-1-yl]-1-(thiazol-2-yl)hexyl-acetat **22** als farbloses Öl.
$^1$H-NMR ($CDCl_3$): δ= 0,56 ppm (q, 6H); 0,87/0,88 (d, 3H); 0,90 (s, 3H); 0,96 (t, 9H); 2,14 (s, 3H); 4,02 (m, 1H); 6,10 (t, 1H); 7,31 (d, 1H); 7,78 (d, 1H)

20. Man behandelt 824 mg des Acetates **22** analog 10. und erhält 583 mg [1*R*-[1α(1*R**),3aβ,4α,7aα]]-1-[5-(Acetyloxy)-1-methyl-5-(thiazol-2-yl)pentyl]octahydro-7amethyl-1*H*-inden-4-ol **23** als farbloses Öl.
$^1$H-NMR ($CDCl_3$): δ= 0,88/0,89 ppm (d, 3H); 0,93 (s, 3H); 2,18 (s, 3H); 4,07 (m, 1H); 6,10 (t, 1H); 7,31 (d, 1H); 7,78 (d, 1H)

21. Man behandelt 583 mg des Alkohols **23** analog 11. und erhält 514 mg [1*R*-[1 α(1*R**),3aβ,7aα]]-1-[5-(Acetyloxy)-1-methyl-5-(thiazol-2-yl)pentyl]octahydro-7amethyl-4*H*-inden-4-on **24** als farbloses Öl.
$^1$H-NMR ($CDCl_3$): δ= 0,56 ppm (s, 3H); 0,91/0,92 (d, 3H); 2,12 (s, 3H); 6,10 (t, 1H); 7,31 (d, 1H); 7,78 (d, 1H)

22. Man setzt 1,39 g des Phosphinoxides **13**, welches mit 1,16 ml n-Butyllithium-Lösung (2,5 M in Hexan) deprotoniert wurde, mit 460 mg des Ketons **24** analog 12. um und erhält 672 mg (7*E*)-(1*R*,3*R*)-24a-(Acetyloxy)-1,3-bis[[(1,1-dimethylethyl)dimethylsilyl]oxy]-24a-(thiazol-2-yl)-24a-homo-19-nor-9,10-secochola-5,7-dien **25** als farblosen Schaum.
$^1$H-NMR ($CDCl_3$): δ= 0,07 ppm (s, 12H); 0,53 (s, 3H); 0,89 (s, 18H); 0,93 (d, 3H); 2,18 (s, 3H); 4,09 (m, 2H); 5,82 (d, 1H); 6,10 (t, 1H); 6,18 (d, 1H); 7,31 (d, 1H); 7,78 (d, 1H)

23. Man setzt 672 mg des Acetates **25** analog 13. um und erhält 526 mg (7*E*)-(1*R*,3*R*)-1,3-Bis[[(1,1-dimethylethyl)dimethylsilyl]oxy]-24a-(thiazol-2-yl)-24a-homo-19-nor-9,10-secochola-5,7-dien-24a-ol **26** als farblosen Schaum.
$^1$H-NMR ($CD_2Cl_2$): δ= 0,04 ppm (s, 12H); 0,51 (s, 3H); 0,87 (s, 18H); 0,92 (d, 3H); 4,07 (m, 2H); 4,95 (m, 1H); 5,81 (d, 1H); 6,15 (d, 1H); 7,31 (d, 1H); 7,70 (d, 1H)

24. Man behandelt 426 mg des Alkohols **26** analog 14. und erhält nach Trennung der Diastereomeren (bzgl. C-24a) über HPLC 134 mg der Titelverbindung **27a** und 149 mg der Titelverbindung **27b** als farblose Schäume.
$^1$H-NMR ($CD_2Cl_2/CD_3OD$): **27a**: δ= 0,50 ppm (s, 3H); 0,88 (d, 3H); 3,93 (m, 1H); 4,01 (m, 1H); 4,90 (t, 1H); 5,83 (d, 1H); 6,23 (d, 1H); 7,29 (d, 1H); 7,67 (d, 1H)
**27b:** δ= 0,50 ppm (s, 3H); 0,90 (d, 3H); 3,93 (m, 1H); 4,01 (m, 1H); 4,90 (t, 1H); 5,83 (d, 1H); 6,23 (d, 1H); 7,29 (d,

1H); 7,67 (d, 1H)

**Beispiel 4**

**(7*E*)-(1*R*,3*R*)-1,3-Dihydroxy-24a-(thiazol-2-yl-)-24-a-homo-19-nor-9,10-secochola-5,7-dien-24a-on 29**

25. Man legt 100 mg des Alkohols **26** in 6 ml Dichlormethan vor und setzt 378 mg Mangandioxid zu. Es wird 2 h bei Raumtemperatur gerührt, anschließend über Celite filtriert, das Lösungsmittel entfernt und der Rückstand an Silicagel chromatographiert, wobei 78 mg (7*E*)-(1*R*,3*R*)-1,3-Bis[[(1,1-dimethylethyl)dimethylsilyl]oxy]-24a-(thiazol-2-yl)-24a-homo-19-nor-9,10-secochola-5,7-dien-24a-on **28** als farbloser Schaum anfallen.
$^1$H-NMR ($CD_2Cl_2$): δ= 0,04 ppm (s, 12H); 0,51 (s, 3H); 0,84 (s, 18H); 0,93 (d, 3H); 4,05 (m, 2H); 5,80 (d, 1H); 6,15 (d, 1H); 7,66 (d, 1H); 7,97 (d, 1H)

26. Man legt 78 mg des Ketons **28** in 7 ml Methanol/Dichlormethan (9:1) vor, fügt 780 mg aktivierten sauren Dowex-Ionentauscher hinzu und rührt 1 d bei Raumtemperatur. Man filtriert über Celite, wäscht gründlich mit Dichlormethan nach, entfernt das Solvens und chromatographiert den Rückstand an Silicagel, wobei man 43 mg der Titelverbindung **29** als farblosen Schaum erhält.
$^1$H-NMR ($CD_2Cl_2$): δ= 0,51 ppm (s, 3H); 0,97 (d, 3H); 3,97 (m, 1H); 4,06 (m, 1H); 5,81 (d, 1H); 6,27 (d, 1H); 7,68 (d, 1H); 7,97 (d, 1H)

**Beispiel 5**

**(5*Z*,7*E*)-(1*S*,3*R*,24a*R*)-24a-(Thiazol-2-yl)-24a-homo-9,10-secochola-5,7,10(19)-trien-1,3,24a-triol 32a und (5*Z*,7*E*)-(1*S*,3*R*,24a*S*)-24a-(Thiazol-2-yl)-24a-homo-9,10-secochola-5,7,10(19)-trien-1,3,24a-triol 32b**

27. Man setzt 309 mg des Phosphinoxides **17**, welches mit 0,25 ml *n*-Butyllithium-Lösung (2,5 M in Hexan) deprotoniert wurde, mit 100 mg des Ketons **24** analog 15. um und erhält 170 mg (5*Z*,7*E*)-(1*S*,3*R*)-24a-(Acetyloxy)-1,3-bis[[(1,1-dimethylethyl)dimethylsilyl]oxy]-24a-(thiazol-2-yl)-24a-homo-9,10-secochola-5,7,10(19)-trien **30** als farblosen Schaum.
$^1$H-NMR ($CDCl_3$): δ= 0,07 ppm (s, 12H); 0,52 (s, 3H); 0,89 (s, 18H); 0,90 (d, 3H); 2,18 (s, 3H); 4,19 (m, 1H); 4,38 (m, 1H); 4,87 (s, 1H); 5,19 (s, 1H); 6,01 (d, 1H); 6,10 (t, 1H); 6,23 (d, 1H); 7,31 (d, 1H); 7,83 (d, 1H)

28. Man setzt 170 mg des Acetates **30** analog 13. um und erhält 149 mg (5*Z*,7*E*)-(1*S*,3*R*)-1,3-Bis[[(1,1-dimethylethyl)dimethylsilyl]oxy]-24a-(thiazol-2-yl)-24a-homo-9,10-secochola-5,7,10(19)-trien-24a-ol **31** als farblosen Schaum.
$^1$H-NMR ($CDCl_3$): δ= 0,07 ppm (s, 12H); 0,51 (s, 3H); 0,89 (s, 18H); 0,90 (d, 3H); 2,18 (s, 3H); 4,18 (m, 1H); 4,38 (m, 1H); 4,87 (s, 1H); 5,02 (m, 1H); 5,19 (s, 1H); 6,01 (d, 1H); 6,23 (d, 1H); 7,31 (d, 1H); 7,74 (d, 1H)

29. Man behandelt 101 mg des Alkohols **31** analog 14. und erhält nach Trennung der Diastereomeren (bzgl. C-24a) über HPLC 24 mg der Titelverbindung **32a** und 29 mg der Titelverbindung **32b** als farblose Schäume.
$^1$H-NMR ($CD_2Cl_2$/$CD_3OD$): **32a**: δ= 0,50 ppm (s, 3H); 0,90 (d, 3H); 4,11 (m, 1H); 4,33 (m, 1H); 4,90 (t, 1H); 4,92 (s, 1H); 5,27 (s, 1H); 6,00 (d, 1H); 6,32 (d, 1H); 7,29 (d, 1H); 7,68 (d, 1H)
**32b**: δ= 0,51 ppm (s, 3H); 0,89 (d, 3H); 4,11 (m, 1H); 4,33 (m, 1H); 4,90 (t, 1H); 4,92 (s, 1H); 5,27 (s, 1H); 6,00 (d, 1H); 6,32 (d, 1H); 7,29 (d, 1H); 7,68 (d, 1H)

**Beispiel 6**

**(5*Z*,7*E*)-(1*S*,3*R*)-1,3-Dihydroxy-24a-(thiazol-2-yl)-24a-homo-9,10-secochola-5,7,10(19)-trien-24a-on 34**

30. Man behandelt 149 mg des Alkohols **31** analog 25. und erhält 127 mg (5*Z*,7*E*)-(1*S*,3*R*)-1,3-Bis[[(1,1-dimethylethyl)dimethylsilyl]oxy]-24a-(thiazol-2-yl)-24a-homo-9,10-secochola-5,7,10(19)-trien-24a-on **33** als farblosen Schaum.
$^1$H-NMR ($CDCl_3$): δ= 0,07 ppm (s, 12H); 0,53 (s, 3H); 0,90 (s, 18H); 0,97 (d, 3H); 4,19 (m, 1H); 4,38 (m, 1H); 4,88 (s, 1H); 5,19 (s, 1H); 6,02 (d, 1H); 6,24 (d, 1H); 7,68 (d, 1H); 8,00 (d, 1H)

31. Man behandelt 21 mg des Ketons **33** analog 26. und erhält 8 mg der Titelverbindung **34** als farblosen Schaum.
$^1$H-NMR ($CD_2Cl_2$): δ= 0,51 ppm (s, 3H); 0,94 (d, 3H); 4,17 (m, 1H); 4,34 (m, 1H); 4,90 (s, 1H); 5,26 (s, 1H); 6,00 (d, 1H); 6,33 (d, 1H); 7,66 (d, 1H); 7,97 (d, 1H)

**Beispiel 7**

**(7*E*)-(1*R*,3*R*,24a*R*)-24a-(4-Methylthiazol-2-yl)-24a-homo-19-nor-9,10-secochola-5,7-dien-1,3,24a-triol 41a und (7*E*)-(1*R*,3*R*,24a*S*)-24a-(4-Methylthiazol-2-yl)-24a-homo-19-nor-9,10-secochola-5,7-dien-1,3,24a-triol 41b**

32. Man legt 1,3 ml 4-Methylthiazol in 25 ml Tetrahydrofuran vor und gibt bei -78°C 5,78 ml *n*-Butyllithium-Lösung (2,5 M in Hexan) zu. Nach 30 min bei dieser Temperatur tropft man 1,06 g des Aldehydes **8** in 5 ml Tetrahydrofuran zu und rührt 1 h nach. Anschließend wird mit Natriumchlorid-Lösung gequencht, mit Essigester extrahiert, die organische Phase mit Natriumchlorid-Lösung gewaschen und über Natriumsulfat getrocknet. Das Lösungsmittel wird entfernt und der Rückstand wird an Silicagel mit Essigester/Hexan chromatographiert, wobei man 601 mg [1*R*-[1α(1*R**),3aβ,4α,7aα]]-α-[4-[Octahydro-7a-methyl-4-[(triethylsilyl)oxy]-1*H*-inden-1-yl]pentyl]-4-methylthiazol-2-methanol **35** als farbloses Öl erhält.

$^1$H-NMR ($CDCl_3$): δ= 0,56 ppm (q, 6H); 0,88 (d, 3H); 0,90 (s, 3H); 0,97 (t, 9H); 2,41 (s, 3H); 4,02 (m, 1H); 4,94 (m, 1H); 6,82 (s, 1H)

33. Man behandelt 601 mg des Alkohols **35** analog 9. und erhält 589 mg [1*R*-[1α(1*R**),3aβ,4α,7aα]]-5-[Octahydro-7a-methyl-4-[(triethylsilyl)oxy]-1*H*-inden-1-yl]-1-(4-methylthiazol-2-yl)hexyl-acetat **36** als farbloses Öl.

$^1$H-NMR ($CDCl_3$): δ= 0,56 ppm (q, 6H); 0,85/0,86 (d, 3H); 0,91 (s, 3H); 0,96 (t, 9H); 2,14 (s, 3H); 2,45 (s, 3H); 4,02 (m, 1H); 6,02 (t, 1H); 6,81 (s, 1H)

34. Man behandelt 589 mg des Acetates **36** analog 10. und erhält 366 mg [1*R*-[1α(1*R**),3aβ,4α,7aα]]-1-[5-(Acetyloxy)- 1 -methyl-5-(4-methylthiazol-2-yl)pentyl]octahydro-7a-methyl-1*H*-inden-4-ol **37** als farbloses Öl.

$^1$H-NMR ($CDCl_3$): δ= 0,87/0,88 ppm (d, 3H); 0,91 (s, 3H); 2,17 (s, 3H); 2,45 (s, 3H); 4,07 (m, 1H); 6,03 (t, 1H); 6,81 (s, 1H)

35. Man behandelt 360 mg des Alkohols **37** analog 11. und erhält 340 mg [1*R*-[1α(1*R**),3aβ,7aα]]-1-[5-(Acetyloxy)-1-methyl-5-(2-methylthiazol-2-yl)pentyl]octahydro-7a-methyl-4*H*-inden-4-on **38** als farbloses Öl.

$^1$H-NMR ($CDCl_3$): δ= 0,61 ppm (s, 3H); 0,92/0,93 (d, 3H); 2,13 (s, 3H); 2,45 (s, 3H); 6,05 (t, 1H); 6,82 (s, 1H)

36. Man setzt 329 mg des Phosphinoxides **13**, welches mit 0,28 ml *n*-Butyllithium-Lösung (2,5 M in Hexan) deprotoniert wurde, mit 113 mg des Ketons **38** analog 12. um und erhält 98 mg (7*E*)-(1*R*,3*R*)-24a-(Acetyloxy)-1,3-bis[[(1,1-dimethylethyl)dimethylsilyl]oxy]-24a-(4-methylthiazol-2-yl)-24a-homo-19-nor-9,10-secochola-5,7-dien **39** als farblosen Schaum.

$^1$H-NMR ($CDCl_3$): δ= 0,07 ppm (s, 12H); 0,53 (s, 3H); 0,89 (s, 18H); 0,90 (d, 3H); 2,15 (s, 3H); 2,46 (s, 3H); 4,09 (m, 2H); 5,81 (d, 1H); 6,04 (t, 1H); 6,18 (d, 1H); 6,83 (s, 1H)

37. Man setzt 93 mg des Acetates **39** analog 13. um und erhält 72 mg (7*E*)-(1*R*,3*R*)-1,3-Bis[[(1,1-dimethylethyl)dimethylsilyl]oxy]-24a-(4-methylthiazol-2-yl)-24a-homo-19-nor-9,10-secochola-5,7-dien-24a-ol **40** als farblosen Schaum.

$^1$H-NMR ($CDCl_3$): δ= 0,07 ppm (s, 12H); 0,53 (s, 3H); 0,89 (s, 18H); 0,93 (d, 3H); 2,45 (s, 3H); 4,08 (m, 2H); 4,95 (m, 1H); 5,82 (d, 1H); 6,18 (d, 1H); 6,82 (s, 1H)

38. Man behandelt 72 mg des Alkohols **40** analog 14. und erhält nach Trennung der Diastereomeren (bzgl. C-24a) über HPLC 15 mg der Titelverbindung **41a** und 21 mg der Titelverbindung **41b** als farblose Schäume.

$^1$H-NMR ($CD_2Cl_2$): **41a:** δ= 0,51 ppm (s, 3H); 0,90 (d, 3H); 2,36 (s, 3H); 3,94 (m, 1H); 4,02 (m, 1H); 4,88 (m, 1H); 5,83 (d, 1H); 6,24 (d, 1H); 6,82 (s, 1H)

**41b:** δ= 0,51 ppm (s, 3H); 0,90 (d, 3H); 2,36 (s, 3H); 3,94 (m, 1H); 4,02 (m, 1H); 4,86 (m, 1H); 5,83 (d, 1H); 6,24 (d, 1H); 6,83 (s, 1H)

**Beispiel 8**

**(7*E*)-(1*R*,3*R*)-1,3-Dihydroxy-24a-(4-methylthiazol-2-yl)-24a-homo-19-nor-9,10-secochola-5,7-dien-24a-on 43**

39. Man behandelt 53 mg des Alkohols **40** analog 25., wobei 42 mg (7*E*)-(1*R*,3*R*)-1,3-Bis[[(1,1-dimethylethyl)dimethylsilyl]oxy]-24a-(4-methylthiazol-2-yl)-24a-homo-19-nor-9,10-secochola-5,7-dien-24a-on **42** als farbloser Schaum anfallen.

$^1$H-NMR ($CDCl_3$): δ= 0,07 ppm (s, 12H); 0,53 (s, 3H); 0,89 (s, 18H); 0,97 (d, 3H); 2,54 (s, 3H); 4,09 (m, 2H); 5,81 (d, 1H); 6,18 (d, 1H); 7,23 (s, 1H)

40. Man behandelt 42 mg des Ketons **42** analog 26., wobei 13 mg der Titelverbindung **43** als farbloser Schaum anfallen.

$^1$H-NMR ($CD_2Cl_2/CD_3OD$): δ= 0,52 ppm (s, 3H); 0,93 (d, 3H); 2,48 (s, 3H); 3,94 (m, 1H); 4,03 (m, 1H); 5,82 (d, 1H); 6,23 (d, 1H); 7,23 (s, 1H)

**Beispiel 9**

**(5*Z*,7*E*)-(1*S*,3*R*,24a*R*)-24a-(4-Methylthiazol-2-yl)-24a-homo-9,10-secochola-5,7,10(19)-trien-1,3,24a-triol 46a und (5*Z*,7*E*)-(1*S*,3*R*,24a*S*)-24a-(4-Methylthiazol-2-yl)-24a-homo-9,10-secochola-5,7,10(19)-trien-1,3,24a-triol 46b**

41. Man setzt 337 mg des Phosphinoxides **17**, welches mit 0,28 ml *n*-Butyllithium-Lösung (2.5 M in Hexan) deprotoniert wurde, mit 113 mg des Ketons **38** analog 15. um und erhält 170 mg (5*Z*,7*E*)-(1*S*,3*R*)-24a-(Acetyloxy)-1,3-bis[[(1,1-dimethylethyl)dimethylsilyl]oxy]-24a-(4-methylthiazol-2-yl)-24a-homo-9,10-secochola-5,7,10(19)-trien **44** als farblosen Schaum.

$^1$H-NMR ($CDCl_3$): δ= 0,07 ppm (s, 12H); 0,52 (s, 3H); 0,89 (s, 18H); 0,90 (d, 3H); 2,14 (s, 3H); 2,45 (s, 3H); 4,19 (m, 1H); 4,38 (m, 1H); 4,88 (s, 1H); 5,19 (s, 1H); 6,02 (d, 1H); 6,03 (m, 1H); 6,23 (d, 1H); 6,83 (s, 1H)

42. Man setzt 170 mg des Acetates **44** analog 13. um und erhält 109 mg (5*Z*,7*E*)-(1*S*,3*R*)-1,3-Bis[[(1,1-dimethylethyl)dimethylsilyl] oxy]-24a-(4-methylthiazol-2-yl)-24ahomo-9,10-secochola-5,7,10(19)-trien-24a-ol **45** als farblosen Schaum.

$^1$H-NMR ($CDCl_3$): δ= 0,07 ppm (s, 12H); 0,52 (s, 3H); 0,90 (s, 18H); 0,93 (d, 3H); 2,45 (s, 3H); 4,19 (m, 1H); 4,38 (m, 1H); 4,88 (s, 1H); 4,95 (m, 1H); 5,19 (s, 1H); 6,02 (d, 1H); 6,23 (d, 1H); 6,83 (s, 1H)

43. Man behandelt 105 mg des Alkohols **45** analog 14. und erhält nach Trennung der Diastereomeren (bzgl. C-24a) über HPLC 30 mg der Titelverbindung **46a** und 29 mg der Titelverbindung **46b** als farblose Schäume.

$^1$H-NMR ($CD_2Cl_2$): **46a:** δ= 0,50 ppm (s, 3H); 0,90 (d, 3H); 2,38 (s, 3H); 4,17 (m, 1H); 4,36 (m, 1H); 4,88 (dd, 1H); 4,94 (s, 1H); 5,27 (s, 1H); 6,00 (d, 1H); 6,36 (d, 1H); 6,81 (s, 1H)

**46b:** δ= 0,50 ppm (s, 3H); 0,89 (d, 3H); 2,38 (s, 3H); 4,17 (m, 1H); 4,36 (m, 1H); 4,84 (dd, 1H); 4,94 (s, 1H); 5,27 (s, 1H); 6,00 (d, 1H); 6,35 (d, 1H); 6,81 (s, 1H)

**Beispiel 10**

**(5*Z*,7*E*)-(1*S*,3*R*)-1,3-Dihydroxy-24a-(4-methylthiazol-2-yl)-24a-homo-9,10-secochola-5,7,10(19)-trien-24a-on 48**

44. Man behandelt 48 mg des Alkohols **45** analog 25. und erhält 40 mg (5*Z*,7*E*)-(1*S*,3*R*)-1,3-Bis[[(1,1-dimethylethyl)dimethylsilyl]oxy]-24a-(4-methylthiazol-2-yl)-24a-homo-9,10-secochola-5,7,10(19)-trien-24a-on **47** als farblosen Schaum.

$^1$H-NMR ($CDCl_3$): δ= 0,07 ppm (s, 12H); 0,52 (s, 3H); 0,88 (s, 18H); 0,95 (d, 3H); 2,52 (s, 3H); 4,19 (m, 1H); 4,37 (m, 1H); 4,84 (s, 1H); 5,18 (s, 1H); 6,00 (d, 1H); 6,23 (d, 1H); 7,21 (s, 1H)

45. Man behandelt 39 mg des Ketons **47** analog 26. und erhält 11 mg der Titelverbindung **48** als farblosen Schaum.

$^1$H-NMR ($CD_2Cl_2$): δ= 0,53 ppm (s, 3H); 0,95 (d, 3H); 2,45 (s, 3H); 4,15 (m, 1H); 4,36 (m, 1H); 4,95 (s, 1H); 5,27 (s, 1H); 6,00 (d, 1H); 6,35 (d, 1H); 7,24 (s, 1H)

**Beispiel 11**

**(7*E*)-(1*R*,3*R*,24a*R*)-24a-(Thien-2-yl)-24a-homo-19-nor-9,10-secochola-5,7-dien-1,3,24a-triol 55a und (7*E*)-(1*R*,3*R*,24a*S*)-24a-(Thien-2-yl)-24a-homo-19-nor-9,10-secochola-5,7-dien-1,3,24a-triol 55b**

46. Man legt 0,65 ml Thiophen in 20 ml Tetrahydrofuran vor und gibt bei -78°C 3,27 ml *n*-Butyllithium-Lösung (2,5 M in Hexan) zu. Nach 30 min bei dieser Temperatur tropft man 600 mg des Aldehydes **8** in 3 ml Tetrahydrofuran zu und rührt 1 h nach. Anschließend wird mit Natriumchlorid-Lösung gequencht, mit Essigester extrahiert, die organische Phase mit Natriumchlorid-Lösung gewaschen und über Natriumsulfat getrocknet. Das Lösungsmittel wird entfernt und der Rückstand wird an Silicagel mit Essigester/Hexan chromatographiert, wobei man 673 mg [1*R*-[1α(1*R**),3aβ,4α,7aα]]-α-[4-[Octahydro-7a-methyl-4-[(triethylsilyl)oxy]-1*H*-inden-1-yl]pentyl]thiophen-2-methanol **49** als farbloses Öl erhält.

$^1$H-NMR ($CDCl_3$): δ= 0,56 ppm (q, 6H); 0,89 (d, 3H); 0,91 (s, 3H); 0,97 (t, 9H); 4,03 (m, 1H); 4,93 (m, 1H); 6,98 (m, 2H); 7,27 (m, 1H)

47. Man behandelt 673 mg des Alkohols **49** analog 9. und erhält 712 mg [1*R*-[1α(1*R**),3aβ,4α,7aα]]-5-[Octahydro-7a-methyl-4-[(triethylsilyl)oxy]-1*H*-inden-1-yl]-1-(thien-2-yl)hexyl-acetat **50** als farbloses Öl.

$^1$H-NMR ($CDCl_3$): δ= 0,56 ppm (q, 6H); 0,87/0,88 (d, 3H); 0,91 (s, 3H); 0,97 (t, 9H); 2,08 (s, 3H); 4,02 (m, 1H); 6,05 (t, 1H); 6,98 (m, 1H); 7,05 (m, 1H); 7,27 (m, 1H)

48. Man behandelt 704 mg des Acetates **50** analog 10. und erhält 412 mg [1*R*-[1α(1*R**),3aβ,4α,7aα]]-1-[5-(Acetyloxy)-1-methyl-5-(thien-2-yl)pentyl]octahydro-7a methyl-1*H*-inden-4-ol **51** als farbloses Öl.

$^1$H-NMR ($CDCl_3$): δ= 0,87/0,88 ppm (d, 3H); 0,92 (s, 3H); 2,08 (s, 3H); 4,07 (m, 1H); 6,03 (t, 1H); 6,97 (m, 1H); 7,07 (m, 1H); 7,28 (m, 1H)

49. Man behandelt 407 mg des Alkohols **51** analog 11. und erhält 373 mg [1*R*-[1α(1*R**),3aβ,7aα]]-1-[5-(Acetyloxy)-1-methyl-5-(thien-2-yl)pentyl]octahydro-7a-methyl-4*H*-inden-4-on **52** als farbloses Öl.

$^1$H-NMR ($CDCl_3$): δ= 0,62 ppm (s, 3H); 0,92/0,93 (d, 3H); 2,08 (s, 3H); 6,04 (t, 1H); 6,98 (m, 1H); 7,05 (m, 1H); 7,28 (m, 1H)

50. Man setzt 329 mg des Phosphinoxides **13,** welches mit 0,28 ml *n*-Butyllithium-Lösung (2,5 M in Hexan) deprotoniert wurde, mit 109 mg des Ketons **52** analog 12. um und erhält 183 mg (7*E*)-(1*R*,3*R*)-24a-(Acetyloxy)-1,3-bis[[(1,1-dimethylethyl)dimethylsilyl]oxy]-24a-(thien-2-yl)-24a-homo-19-nor-9,10-secochola-5,7-dien **53** als farblosen Schaum.

$^1$H-NMR ($CDCl_3$): δ= 0,07 ppm (s, 12H); 0,53 (s, 3H); 0,90 (s, 18H); 0,90 (d, 3H); 2,07 (s, 3H); 4,08 (m, 2H); 5,81 (d, 1H); 6,04 (t, 1H); 6,18 (d, 1H); 6,98 (m, 1H); 7,05 (m, 1H); 7,29 (m, 1H)

51. Man setzt 178 mg des Acetates **53** analog 13. um und erhält 138 mg (7*E*)-(1*R*,3*R*)-1,3-Bis[[(1,1-dimethylethyl)dimethylsilyl]oxy]-24a-(thien-2-yl)-24a-homo-19-nor-9,10-secochola-5,7-dien-24a-ol **54** als farblosen Schaum.

$^1$H-NMR ($CDCl_3$): δ= 0,07 ppm (s, 12H); 0,53 (s, 3H); 0,89 (s, 18H); 0,91 (d, 3H); 4,09 (m, 2H); 4,94 (m, 1H); 5,82 (d, 1H); 6,18 (d, 1H); 6,98 (m, 2H); 7,28 (m, 1H)

52. Man behandelt 97 mg des Alkohols **54** analog 14. und erhält nach Trennung der Diastereomeren (bzgl. C-24a) über HPLC 19 mg der Titelverbindung **55a** und 24 mg der Titelverbindung **55b** als farblose Schäume.

$^1$H-NMR ($CD_2Cl_2$): **55a**: δ= 0,52 ppm (s, 3H); 0,90 (d, 3H); 3,95 (m, 1H); 4,02 (m, 1H); 4,87 (dd, 1H); 5,84 (d, 1H); 6,25 (d, 1H); 6,98 (m, 2H); 7,23 (m, 1H)

**41b**: δ= 0,52 ppm (s, 3H); 0,89 (d, 3H); 3,95 (m, 1H); 4,02 (m, 1H); 4,86 (t, 1H); 5,84 (d, 1H); 6,25 (d, 1H); 6,97 (m, 2H); 7,22 (m, 1H)

**Beispiel 12**

**(7*E*)-(1*R*,3*R*)-1,3-Dihydroxy-24a-(thien-2-yl)-24a-homo-19-nor-9,10-secochola-5,7-dien-24a-on 57**

53. Man behandelt 40 mg des Alkohols **54** analog 25., wobei 28 mg (7*E*)-(1*R*,3*R*)-1,3-Bis[[(1,1-dimethylethyl)dimethylsilyl]oxy]-24a-(thien-2-yl)-24a-homo-19-nor-9,10-secochola-5,7-dien-24a-on **56** als farbloser Schaum anfallen.

$^1$H-NMR ($CDCl_3$): δ= 0,07 ppm (s, 12H); 0,53 (s, 3H); 0,88 (s, 18H); 0,98 (d, 3H); 4,09 (m, 2H); 5,81 (d, 1H); 6,18 (d, 1H); 7,14 (dd, 1H); 7,62 (d, 1H); 7,72 (d, 1H)

54. Man behandelt 27 mg des Ketons **42** analog 26., wobei 13 mg der Titelverbindung **57** als farbloser Schaum anfallen.

$^1$H-NMR ($CD_2Cl_2$): δ= 0,52 ppm (s, 3H); 0,92 (d, 3H); 3,93 (m, 1H); 4,03 (m, 1H); 5,82 (d, 1H); 6,23 (d, 1H); 7,12 (dd, 1H); 7,60 (d, 1H); 7,67 (d, 1H)

**Beispiel 13**

(**5*Z*,7*E*)-(1*S*,3*R*,24a*R*)-24a-(Thien-2-yl)-24a-homo-9,10-secochola-5,7,10(19)-trien-1,3,24a-triol 60a** und **(5*Z*,7*E*)-(1*S*,3*R*,24a*S*)-24a-(Thien-2-yl)-24a-homo-9,10-secochola-5,7,10(19)-trien-1,3,24a-triol 60b**

55. Man setzt 350 mg des Phosphinoxides **17**, welches mit 0,29 ml *n*-Butyllithium-Lösung (2,5 M in Hexan) deprotoniert wurde, mit 113 mg des Ketons **52** analog 15. um und erhält 93 mg (5*Z*,7*E*)-(1*S*,3*R*)-24a-(Acetyloxy)-1,3-bis[[(1,1-dimethylethyl)dimethylsilyl]oxy]-24a-(thien-2-yl)-24a-homo-9,10-secochola-5,7,10(19)-trien **58** als farblosen Schaum.

$^1$H-NMR ($CDCl_3$): δ= 0,07 ppm (s, 12H); 0,53 (s, 3H); 0,90 (s, 18H); 0,90 (d, 3H); 2,07 (s, 3H); 4,19 (m, 1H); 4,38 (m, 1H); 4,88 (s, 1H); 5,20 (s, 1H); 6,01 (d, 1H); 6,02 (t, 1H); 6,24 (d, 1H); 6,98 (m, 1H); 7,06 (m, 1H); 7,28 (m, 1H)

56. Man setzt 92 mg des Acetates **58** analog 13. um und erhält 79 mg (5*Z*,7*E*)-(1*S*,3*R*)-1,3-Bis[[dimethyl(1,1-dimethylethyl)silyl]oxy]-24a-(thien-2-yl)-24a-homo-9,10-secochola-5,7,10(19)-trien-24a-ol **59** als farblosen Schaum.

$^1$H-NMR ($CDCl_3$): δ= 0,07 ppm (s, 12H); 0,52 (s, 3H); 0,88 (s, 18H); 0,90 (d, 3H); 4,18 (m, 1H); 4,38 (m, 1H); 4,88 (s, 1H); 4,93 (m, 1H); 5,18 (s, 1H); 6,00 (d, 1H); 6,22 (d, 1H); 6,98 (m, 2H); 7,28 (m, 1H)

57. Man behandelt 78 mg des Alkohols **59** analog 14. und erhält nach Trennung der Diastereomeren (bzgl. C-24a) über HPLC 21 mg der Titelverbindung **60a** und 24 mg der Titelverbindung **60b** als farblose Schäume.

$^1$H-NMR ($CD_2Cl_2/CD_3OD$): **60a:** δ= 0,50 ppm (s, 3H); 0,88 (d, 3H); 4,10 (m, 1H); 4,32 (m, 1H); 4,86 (dd, 1H); 4,94 (s, 1H); 5,26 (s, 1H); 6,00 (d, 1H); 6,27 (d, 1H); 6,92 (m, 2H); 7,20 (m, 1H)

**60b**: δ= 0,50 ppm (s, 3H); 0,86 (d, 3H); 4,10 (m, 1H); 4,31 (m, 1H); 4,83 (t, 1H); 4,94 (s, 1H); 5,26 (s, 1H); 6,00 (d, 1H);

6,27 (d, 1H); 6,92 (m, 2H); 7,20 (m, 1H)

**Beispiel 14**

**(7*E*)-(1*R*,2*S*,3*R*,24a*R*)-24a-Thien-2-yl-24a-homo-19-nor-9,10-secochola-5,7-dien-1,2,3,24-tetrol 64a** und **(7*E*)-(1*R*,2*S*,3*R*,24a*S*)-24a-Thien-2-yl-24a-homo-19-nor-9,10-secochola-5,7-dien-1,2,3,24-tetrol 64b**

58. Man legt 405 mg [2-[[3*R*-(3*R*,4*S*,5*R*)]-3,4,5-Tris[[(1,1-dimethylethyl)dimethylsilyl]oxy]cyclo-hexyliden] ethyl]diphenylphosphinoxid **61a** [H.F. DeLuca et al. *J. Med. Chem.* **37**, 3730 (1994)] in 6 ml Tetrahydrofuran vor und tropft bei -78°C 0,28 ml *n*-Butyllithium-Lösung (2,5 M in Hexan) zu. Bei -30°C gibt man nach 10 min 109 mg des Ketons **52** zu und rührt 1 h bei dieser Temperatur. Anschließend wird mit Natriumchlorid-Lösung gequencht, mit Essigester extrahiert, die organische Phase mit Natriumchlorid-Lösung gewaschen, über Natriumsulfat getrocknet und das Lösungsmittel entfernt. Den Rückstand chromatographiert man an Silicagel mit Essigester/Hexan, wobei man 206 mg (7*E*)-(1*R*,2*S*,3*R*)-24a-(Acetyloxy)-24a-(thien-2-yl)-1,2,3-tris[[(1,1-dimethylethyl)dimethylsilyl]-oxy]-24a-homo-19-nor-9,10-secochola-5,7-dien **62** als farblosen Schaum erhält.
$^1$H-NMR ($CDCl_3$): δ= 0,02-0,07 ppm (4 x s, 18H); 0,53 (s, 3H); 0,83 (s, 9H); 0,89 (s, 18H); 0,90 (d, 3H); 2,07 (s, 3H); 3,64 (m, 1H); 3,80 (m, 1H); 3,85 (m, 1H); 5,84 (d, 1H); 6,03 (t, 1H); 6,04 (d, 1H); 6,97 (m, 2H); 7,05 (m, 1H)
59. Man setzt 205 mg des Acetates **62** analog 13. um und erhält 180 mg (7*E*)-(1*R*,2*S*,3*R*)-24a-(Thien-2-yl)-1,2,3-tris[[(1,1-dimethylethyl)dimethylsilyl]oxy]-24ahomo-19-nor-9,10-secochola-5,7-dien-24a-ol **63** als farblosen Schaum.
$^1$H-NMR ($CDCl_3$): δ= 0,02-0,07 ppm (4 x s, 18H); 0,53 (s, 3H); 0,83 (s, 9H); 0,89 (s, 18H); 0,90 (d, 3H); 3,63 (m, 1H); 3,79 (m, 1H); 3,83 (m, 1H); 4,93 (m, 1H); 5,83 (d, 1H); 6,04 (d, 1H); 6,99 (m, 2H); 7,28 (m, 1H)
60. Man behandelt 134 mg des Alkohols **63** analog 14. und erhält nach Trennung der Diastereomeren (bzgl. C-24a) über HPLC 18 mg der Titelverbindung **64a** und 25 mg der Titelverbindung **64b** als farblose Schäume.
$^1$H-NMR ($CD_2Cl_2$): **64a**: δ= 0,52 ppm (s, 3H); 0,89 (d, 3H); 3,44 (dd, 1H); 3,72 (m, 1H); 4,00 (m, 1H); 4,87 (dd, 1H); 5,76 (d, 1H); 6,28 (d, 1H); 6,93 (m, 2H); 7,22 (m, 1H)
**64b**: δ= 0,53 ppm (s, 3H); 0,90 (d, 3H); 3,46 (dd, 1H); 3,70 (m, 1H); 4,00 (m, 1H); 4,87 (t, 1H); 5,77 (d, 1H); 6,28 (d, 1H); 6,93 (m, 2H); 7,21 (m, 1H)

**Beispiel 15**

**(7*E*)-(1*R*,2*S*,3*R*)-24a-Thien-2-yl-1,2,3-trihydroxy-24a-homo-9,10-secochola-5,7-dien-24a-on 66**

61. Man behandelt 45 mg des Alkohols **63** analog 25., wobei 38 mg (7*E*)-(1*R*,2*S*,3*R*)-24a-(Thien-2-yl)-1,2,3-tris[[(1,1-dimethylethyl)dimethylsilyl]oxy]-24a-homo-19-nor-9,10-secochola-5,7-dien-24a-on **65** als farbloser Schaum anfallen.
$^1$H-NMR ($CDCl_3$): δ= 0,02-0,07 ppm (4 x s, 18H); 0,53 (s, 3H); 0,83 (s, 9H); 0,90(s, 18H); 0,97 (d, 3H); 3,62 (m, 1H); 3,80 (m, 1H); 3,85 (m, 1H); 5,85 (d, 1H); 6,06 (d, 1H); 7,12 (dd, 1H); 7,61 (d, 1H); 7,70 (d, 1H)
62. Man behandelt 38 mg des Ketons **65** analog 26., wobei 13 mg der Titelverbindung **66** als farbloser Schaum anfallen.
$^1$H-NMR ($CD_2Cl_2$): δ= 0,53 ppm (s, 3H); 0,95 (d, 3H); 3,48 (m, 1H); 3,74 (m, 1H); 4,03 (m, 1H); 5,85 (d, 1H); 6,30 (d, 1H); 7,13 (dd, 1H); 7,61 (d, 1H); 7,69 (d, 1H)

**Beispiel 16**

**(7*E*)-(1*R*,3*R*,24a*R*)-24a-(4-Methylthien-2-yl)-24a-homo-19-nor-9,10-secochola-5,7-dien-1,3,24a-triol 73a** und **(7*E*)-(1*R*,3*R*,24a*S*)-24a-(4-Methylthien-2-yl)-24a-homo-19-nor-9,10-secochola-5,7-dien-1,3,24a-triol 73b**

63. Man legt 0,79 ml 3-Methylthiophen in 20 ml Tetrahydrofuran vor und gibt bei -78°C 3,27 ml *n*-Butyllithium-Lösung (2,5 M in Hexan) zu. Nach 30 min bei dieser Temperatur tropft man 600 mg des Aldehydes **8** in 3 ml Tetrahydrofuran zu und rührt 1 h nach. Anschließend wird mit Natriumchlorid-Lösung gequencht, mit Essigester extrahiert, die organische Phase mit Natriumchlorid-Lösung gewaschen und über Natriumsulfat getrocknet. Das Lösungsmittel wird entfernt und der Rückstand wird an Silicagel mit Essigester/Hexan chromatographiert, wobei man 681 mg [1*R*-[1α(1*R**),3aβ,4α,7aα]]-α-[4-[Octahydro-7a-methyl-4-[(triethylsilyl)oxy]-1*H*-inden-1-yl]pentyl]-4-methylthiophen-2-methanol **67** als farbloses Öl erhält.
$^1$H-NMR ($CDCl_3$): δ= 0,55 ppm (q, 6H); 0,88 (d, 3H); 0,90 (s, 3H); 0,96 (t, 9H); 2,23 (s, 3H); 4,03 (m, 1H); 4,83 (m, 1H); 6,80 (s, 1H); 6,82 (s, 1H)
64. Man behandelt 671 mg des Alkohols **67** analog 9. und erhält 692 mg [1*R*-[1α(1*R**),3aβ,4α,7aα]]-5-[Octahydro-7a-methyl-4-[(triethylsiyl)oxy]-1*H*-inden-1-yl]-1-(4-methylthien-2-yl)hexyl-acetat **68** als farbloses Öl.
$^1$H-NMR ($CDCl_3$): δ= 0,56 ppm (q, 6H); 0,87/0,88 (d, 3H); 0,90 (s, 3H); 0,96 (t, 9H); 2,08 (s, 3H); 2,25 (s, 3H); 4,03 (m, 1H); 5,98 (t, 1H); 6,83 (s, 1H); 6,85 (s, 1H)

65. Man behandelt 682 mg des Acetates **68** analog 10. und erhält 425 mg [1*R*-[1α(1*R**),3aβ,4α,7aα]]-1[-5-(Acetyloxy)-1-methyl-5-(4-methylthien-2-yl)pentyl]octahydro-7a-methyl-1*H*-inden-4-ol **69** als farbloses Öl.
$^1$H-NMR ($CDCl_3$): δ= 0,87/0,88 ppm (d, 3H); 0,92 (s, 3H); 2,08 (s, 3H); 2,23 (s, 3H); 4,07 (m, 1H); 5,98 (t, 1H); 6,82 (s, 1H); 6,85 (s, 1H)

66. Man behandelt 415 mg des Alkohols **69** analog 11. und erhält 352 mg [1*R*-[1α(1*R**),3aβ,7aα]]-1-[5-(Acetyloxy)-1-methyl-5-(4-methylthien-2-yl)pentyl]octahydro-7a-methyl-4H-inden-4-on **70** als farbloses Öl.
$^1$H-NMR ($CDCl_3$): δ= 0,62 ppm (s, 3H); 0,92/0,93 (d, 3H); 2,08 (s, 3H); 2,25 (s, 3H); 5,97 (t, 1H); 6,83 (s, 1H); 6,84 (s, 1H)

67. Man setzt 306 mg des Phosphinoxides **13**, welches mit 0,26 ml *n*-Butyllithium-Lösung deprotoniert wurde, mit 105 mg des Ketons (7*E*)-(1*R*,3*R*)-24a-(Acetyloxy)-1,3-bis[[(1,1-dimethylethyl)dimethylsilyl]oxy]-24a-(4-methylthien-2-yl)-24a-homo-19-nor-9,10-secochola-5,7-dien **70** analog 12. um und erhält 166 mg **71** als farblosen Schaum.
$^1$H-NMR ($CDCl_3$): δ= 0,07 ppm (s, 12H); 0,53 (s, 3H); 0,90 (s, 18H); 0,91 (d, 3H); 2,07 (s, 3H); 2,23 (s, 3H); 4,09 (m, 2H); 5,81 (d, 1H); 5,97 (t, 1H); 6,18 (d, 1H); 6,82 (s, 1H); 6,84 (s, 1H)

68. Man setzt 165 mg des Acetates **71** analog 13. um und erhält 128 mg (7*E*)-(1*R*,3*R*)-1,3-Bis[[dimethyl (1,1-dimethylethyl)silyl]oxy]-24a-(4-methylthien-2-yl)-24a-homo-19-nor-9,10-secochola-5,7-dien-24a-ol **72** als farblosen Schaum.
$^1$H-NMR ($CDCl_3$): δ= 0,07 ppm (s, 12H); 0,54 (s, 3H); 0,89 (s, 18H); 0,91 (d, 3H); 2,25 (s, 3H); 4,09 (m, 2H); 4,85 (m, 1H); 5,82 (d, 1H); 6,18 (d, 1H); 6,80 (s, 1H); 6,82 (s, 1H)

69. Man behandelt 91 mg des Alkohols **72** analog 14. und erhält nach Trennung der Diastereomeren (bzgl. C-24a) über HPLC 16 mg der Titelverbindung **73a** und 19 mg der Titelverbindung **73b** als farblose Schäume.
$^1$H-NMR ($CD_2Cl_2/CD_3OD$): **73a:** δ= 0,51 ppm (s, 3H); 0,91 (d, 3H); 2,18 (s, 3H); 3,94 (m, 1H); 4,02 (m, 1H); 4,76 (t, 1H); 5,84 (d, 1H); 6,25 (d, 1H); 6,75 (s, 1H); 6,77 (s, 1H) **73b**: δ= 0,51 ppm (s, 3H); 0,90 (d, 3H); 2,18 (s, 3H); 3,94 (m, 1H); 4,02 (m, 1H); 4,78 (t, 1H); 5,84 (d, 1H); 6,25 (d, 1H); 6,75 (s, 1H); 6,77 (s, 1H)

**Beispiel 17**

**(7*E*)-(1*R*,3*R*)-1,3-Dihydroxy-24a-(4-methylthien-2-yl)-24a-homo-19-nor-9,10-secochola-5,7-dien-24a-on 75**

70. Man behandelt 36 mg des Alkohols **72** analog 25., wobei 30 mg (7*E*)-(1*R*,3*R*)-1,3-Bis[[dimethyl(1,1-dimethylethyl)silyl]oxy]-24a-(4-methylthien-2-yl)-24a-homo-19-nor-9,10-secochola-5,7-dien-24a-on **74** als farbloser Schaum anfallen.
$^1$H-NMR ($CDCl_3$): δ= 0,07 ppm (s, 12H); 0,54 (s, 3H); 0,88 (s, 18H); 0,98 (d, 3H); 2,30 (s, 3H); 4,09 (m, 2H); 5,82 (d, 1H); 6,18 (d, 1H); 7,22 (s, 1H); 7,52 (s, 1H)

71. Man behandelt 29 mg des Ketons **74** analog 26., wobei 13 mg **75** der Titelverbindung als farbloser Schaum anfallen.
$^1$H-NMR ($CD_2Cl_2$): δ= 0,52 ppm (s, 3H); 0,94 (d, 3H); 2,27 (s, 3H); 3,95 (m, 1H); 4,04 (m, 1H); 5,82 (d, 1H); 6,24 (d, 1H); 7,19 (s, 1H); 7,48 (s, 1H)

**Beispiel 18**

**(5*Z*,7*E*)-(1*S*,3*R*,24a*R*)-24a-(4-Methylthien-2-yl)-24a-homo-9,10-secochola-5,7,10(19)-trien-1,3,24a-triol 78a** und **(5*Z*,7*E*)-(1*S*,3*R*,24a*S*)-24a-(4-Methylthien-2-yl)-24a-homo-9,10-secochola-5,7,10(19)-trien-1,3,24a-triol 78b**

72. Man setzt 316 mg des Phosphinoxides **17**, welches mit 0,26 ml *n*-Butyllithium-Lösung (2,5 M in Hexan) deprotoniert wurde, mit 106 mg des Ketons **70** analog 15. um und erhält 177 mg (5*Z*,7*E*)-(1*S*,3*R*)-24a-(Acetyloxy)-1,3-bis[[(1,1-dimethylethyl)dimethylsilyl]oxy]-24a-(4-methylthien-2-yl)-24a-homo-9,10-secochola-5,7,10(19)-trien **76** als farblosen Schaum.
$^1$H-NMR ($CDCl_3$): δ= 0,07 ppm (s, 12H); 0,53 (s, 3H); 0,89 (s, 18H); 0,90 (d, 3H); 2,08 (s, 3H); 2,23 (s, 3H); 4,19 (m, 1H); 4,38 (m, 1H); 4,88 (s, 1H); 5,20 (s, 1H); 5,98 (t, 1H); 6,01 (d, 1H); 6,23 (d, 1H); 6,83 (s, 1H); 6,85 (s, 1H)

73. Man setzt 176 mg des Acetates **76** analog 13. um und erhält 149 mg (5*Z*,7*E*)-(1*S*,3*R*)-1,3-Bis[[(1,1-dimethylethyl)dimethylsilyl]oxy]-24a-(4-methylthien-2-yl)-24ahomo-9,10-secochola-5,7,10(19)-trien-24a-ol **77** als farblosen Schaum.
$^1$H-NMR ($CDCl_3$): δ= 0,07 ppm (s, 12H); 0,53 (s, 3H); 0,89 (s, 18H); 0,90 (d, 3H); 2,23 (s, 3H); 4,19 (m, 1H); 4,38 (m, 1H); 4,88 (s, 1H); 4,95 (m, 1H); 5,19 (s, 1H); 6,02 (d, 1H); 6,25 (d, 1H); 6,80 (s, 1H); 6,82 (s, 1H)

74. Man behandelt 103 mg des Alkohols **77** analog 14. und erhält nach Trennung der Diastereomeren (bzgl. C-24a) über HPLC 26 mg der Titelverbindung **78a** und 27 mg der Titelverbindung **78b** als farblose Schäume.

$^1$H-NMR ($CD_2Cl_2/CD_3OD$): **78a**: δ= 0,50 ppm (s, 3H); 0,88 (d, 3H); 2,17 (s, 3H); 4,09 (m, 1H); 4,30 (m, 1H); 4,76 (t, 1H); 4,92 (s, 1H); 5,27 (s, 1H); 6,00 (d, 1H); 6,28 (d, 1H); 6,82 (s, 1H); 6,84 (s, 1H)
78b: δ= 0,50 ppm (s, 3H); 0,89 (d, 3H); 2,17 (s, 3H); 4,09 (m, 1H); 4,30 (m, 1H); 4,78 (t, 1H); 4,92 (s, 1H); 5,27 (s, 1H); 6,00 (d, 1H); 6,28 (d, 1H); 6,82 (s, 1H); 6,84 (s, 1H)

**Beispiel 19**

**(5*Z*,7*E*)-(1*S*,3*R*)-1,3-Dihydroxy-24a-(4-methylthien-2-yl)-24a-homo-9,10-secochola-5,7,10(19)-trien-24a-on 80**

75. Man behandelt 45 mg des Alkohols **77** analog 25., wobei 20 mg (5*Z*,7*E*)-(1*S*,3*R*)-1,3-Bis[[(1,1-dimethylethyl)dimethylsilyl]oxy]-24a-(4-methylthien-2-yl)-24a-homo-9,10-secochola-5,7,10(19)-trien-24a-on **79** als farbloser Schaum anfallen.
$^1$H-NMR ($CDCl_3$): δ= 0,06 ppm (s, 12H); 0,53 (s, 3H); 0,88 (s, 18H); 0,97 (d, 3H); 2,29 (s, 3H); 4,18 (m, 1H); 4,38 (m, 1H); 4,96 (s, 1H); 5,18 (s, 1H); 6,00 (d, 1H); 6,23 (d, 1H); 7,22 (s, 1H); 7,52 (s, 1H)
76. Man behandelt 19 mg des Ketons **79** analog 26., wobei 9 mg der Titelverbindung **80** als farbloser Schaum anfallen.
$^1$H-NMR ($CD_2Cl_2$): δ= 0,52 ppm (s, 3H); 0,94 (d, 3H); 2,27 (s, 3H); 4,16 (m, 1H); 4,36 (m, 1H); 4,94 (s, 1H); 5,28 (s, 1H); 5,98 (d, 1H); 6,36 (d, 1H); 7,20 (s, 1H); 7,48 (s, 1H)

**Beispiel 20**

**(7*E*)-(1*R*,2*S*,3*R*,24a*R*)-24a-(4-Methylthien-2-yl)-24a-homo-19-nor-9,10-secochola-5,7-dien-1,2,3,24-tetrol 83a** und **(7*E*)-(1*R*,2*S*,3*R*,24a*S*)-24a-(4-Methylthien-2-yl)-24ahomo-19-nor-9,10-secochola-5,7-dien-1,2,3,24-tetrol 83b**

77. Man legt 377 mg des Phosphinoxides **61a** in 6 ml Tetrahydrofuran vor und tropft bei - 78°C 0,26 ml *n*-Butyllithium-Lösung (2,5 M in Hexan) zu. Bei -30°C gibt man nach 10 min 105 mg des Ketons **70** zu und rührt 1 h bei dieser Temperatur. Anschließend wird mit Natriumchlorid-Lösung gequencht, mit Essigester extrahiert, die organische Phase mit Natriumchlorid-Lösung gewaschen, über Natriumsulfat getrocknet und das Lösungsmittel entfernt. Den Rückstand chromatographiert man an Silicagel mit Essigester/Hexan, wobei man 174 mg (7*E*)-(1*R*,2*S*,3*R*)-24a-(Acetyloxy)-24a-(4-methylthien-2-yl)-1,2,3-tris[[(1,1-dimethylethyl)dimethylsilyl]oxy]-24a-homo-19-nor-9,10-secochola-5,7-dien **81** als farblosen Schaum erhält.
$^1$H-NMR ($CDCl_3$): δ= 0,02-0,07 ppm (4 x s, 18H); 0,53 (s, 3H); 0,83 (s, 9H); 0,89 (s, 18H); 0,90 (d, 3H); 2,06 (s, 3H); 2,22 (s, 3H); 3,63 (m, 1H); 3,80 (m, 1H); 3,85 (m, 1H); 5,85 (d, 1H); 5,97 (t, 1H); 6,05 (d, 1H); 6,82 (s, 2H); 6,84 (s, 1H)
78. Man setzt 173 mg des Acetates **81** analog 13. um und erhält 146 mg (7*E*)-(1*R*,2*S*,3*R*)-24a-(4-Methylthien-2-yl)-1,2,3-tris[[(1,1-dimethylethyl)dimethylsilyl]oxy]-24a-homo-19-nor-9,10-secochola-5,7-dien-24a-ol **82** als farblosen Schaum.
$^1$H-NMR ($CDCl_3$): δ= 0,02-0,07 ppm (4 x s, 18H); 0,53 (s, 3H); 0,83 (s, 9H); 0,89 (s, 18H); 0,90 (d, 3H); 2,22 (s, 3H); 3,63 (m, 1H); 3,79 (m, 1H); 3,84 (m, 1H); 4,93 (m, 1H); 5,83 (d, 1H); 6,04 (d, 1H); 6,78 (s, 1H); 6,80 (s, 1H)
79. Man behandelt 99 mg des Alkohols **82** analog 14. und erhält nach Trennung der Diastereomeren (bzgl. C-24a) über HPLC 19 mg der Titelverbindung **83a** und 23 mg der Titelverbindung **83b** als farblose Schäume.
$^1$H-NMR ($CD_2Cl_2$): **83a:** δ= 0,52 ppm (s, 3H); 0,88 (d, 3H); 2,18 (s, 3H); 3,45 (m, 1H); 3,70 (m, 1H); 4,00 (m, 1H); 4,78 (t, 1H); 5,77 (d, 1H); 6,28 (d, 1H); 6,86 (s, 1H); 6,88 (s, 1H)
**83b:** δ= 0,52 ppm (s, 3H); 0,89 (d, 3H); 2,18 (s, 3H); 3,45 (m, 1H); 3,70 (m, 1H); 4,00 (m, 1H); 4,80 (t, 1H); 5,77 (d, 1H); 6,28 (d, 1H); 6,86 (s, 1H); 6,89 (s, 1H)

**Beispiel 21**

**(7*E*-(1*R*,2*S*,3*R*)-24a-(4-Methylthien-2-yl)-1,2,3-trihydroxy-24a-homo-19-nor-9,10-secochola-5,7-dien-24a-on 85**

80. Man behandelt 46 mg des Alkohols **82** analog 25., wobei 41 mg (7*E*)-(1*R*,2*S*,3*R*)-24a-(4-Methylthien-2-yl)-1,2,3-tris[[(1,1-dimethylethyl)dimethylsilyl]oxy]-24a-homo-19-nor-9,10-secochola-5,7-dien-24a-on **84** als farbloser Schaum anfallen.
$^1$H-NMR ($CDCl_3$): δ= 0,02-0,07 ppm (4 x s, 18H); 0,53 (s, 3H); 0,83 (s, 9H); 0,89 (s, 18H); 0,97 (d, 3H); 2,28 (s, 3H); 3,63 (m, 1H); 3,79 (m, 1H); 3,84 (m, 1H); 5,83 (d, 1H); 6,05 (d, 1H); 7,21 (s, 1H); 7,51 (s, 1H)
81. Man behandelt 40 mg des Ketons **84** analog 26., wobei 18 mg der Titelverbindung **85** als farbloser Schaum anfallen.
$^1$H-NMR ($CD_2Cl_2$): δ= 0,53 ppm (s, 3H); 0,95 (d, 3H); 2,26 (s, 3H); 3,47 (m, 1H); 3,70 (m, 1H); 4,01 (m, 1H); 5,77 (d,

1H); 6,30 (d, 1H); 7,20 (s, 1H); 7,50 (s, 1H)

**Beispiel 22**

**(7*E*)-(1*R*,3*R*,24a*R*)-24a-(5-Ethylthien-2-yl)-24a-homo-19-nor-9,10-secochola-5,7-dien-1,3,24a-triol 92a und (7*E*) -(1*R*,3*R*,24a*S*)-24a-(5-Ethylthien-2-yl)-24a-homo-19- nor-9,10-secochola-5,7-dien-1,3,24a-triol 92b**

82. Man legt 1,19 ml 2-Ethylthiophen in 20 ml Tetrahydrofuran vor und gibt bei -78°C 4,2 ml *n*-Butyllithium-Lösung (2,5 M in Hexan) zu. Nach 30 min bei dieser Temperatur tropft man 770 mg des Aldehydes **8** in 5 ml Tetrahydrofuran zu und rührt 1 h nach. Anschließend wird mit Natriumchlorid-Lösung gequencht, mit Essigester extrahiert, die organische Phase mit Natriumchlorid-Lösung gewaschen und über Natriumsulfat getrocknet. Das Lösungsmittel wird entfernt und der Rückstand wird an Silicagel mit Essigester/Hexan chromatographiert, wobei man 773 mg [1*R*-[1α (1*R**),3aβ,4α,7aα]]-5-Ethyl-α-[4-[octahydro-7a-methyl-4-[(triethylsilyl)oxy]-1*H*-inden-1-yl]pentyl]thiophen-2-methanol **86** als farbloses Öl erhält.
$^1$H-NMR ($CDCl_3$): δ= 0,56 ppm (q, 6H); 0,89 (d, 3H); 0,90 (s, 3H); 0,97 (t, 9H); 1,31 (t, 3H); 2,82 (q, 2H); 4,03 (m, 1H); 4,81 (m, 1H); 6,62 (d, 1H); 6,80 (d, 1H)

83. Man behandelt 763 mg des Alkohols **86** analog 9. und erhält 745 mg [1*R*-[1α(1*R**),3aβ,4α,7aα]]-1-(5-Ethylthien-2-yl)-5-[octahydro-7a-methyl-4-[(triethylsilyl)oxy]-1*H*-inden-1-yl]hexyl-acetat **87** als farbloses Öl.
$^1$H-NMR ($CDCl_3$): δ= 0,55 ppm (q, 6H); 0,87/0,88 (d, 3H); 0,90 (s, 3H); 0,95 (t, 9H); 1,30 (t, 3H); 2,07 (s, 3H); 2,82 (q, 2H); 4,03 (m, 1H); 5,97 (t, 1H); 6,62 (d, 1H); 6,87 (d, 1H)

84. Man behandelt 682 mg des Acetates **87** analog 10. und erhält 287 mg [1*R*-[1α(1*R**),3aβ,4α,7aα]]-1-[5-(Acetyloxy)-5-(5-ethylthien-2-yl)-1-methylpentyl]octahydro-7*a*-methyl-1*H*-inden-4-ol **88** als farbloses Öl.
$^1$H-NMR ($CDCl_3$): δ= 0,87/0,88 ppm (d, 3H); 0,92 (s, 3H); 1,31 (t, 3H); 2,08 (s, 3H); 2,82 (q, 2H); 4,07 (m, 1H); 5,97 (t, 1H); 6,63 (d, 1H); 6,85 (d, 1H)

85. Man behandelt 142 mg des Alkohols **88** analog 11. und erhält 123 mg [1*R*-[1α(1*R**),3aβ,7aα]]-1-[5-(Acetyloxy)-5-(5-ethylthien-2-yl)-1-methylpentyl]octahydro-7*a*-methyl-4*H*-inden-4-on **89** als farbloses Öl.
$^1$H-NMR ($CDCl_3$): δ= 0,62 ppm (s, 3H); 0,92/0,93 (d, 3H); 1,30 (t, 3H); 2,07 (s, 3H); 2,82 (q, 2H); 5,97 (t, 1H); 6,62 (d, 1H); 6,86 (d, 1H)

86. Man setzt 347 mg des Phosphinoxides **13**, welches mit 0,3 ml *n*-Butyllithium-Lösung (2,5 M in Hexan) deprotoniert wurde, mit 123 mg des Ketons **89** analog 12. um und erhält 129 mg (7*E*)-(1*R*,3*R*)-24a-(Acetyloxy)-1,3-bis[[(1,1-dimethylethyl)dimethylsilyl]oxy]-24a-(5-ethylthien-2-yl)-24a-homo- 19-nor-9,10-secochola-5,7-dien **90** als farblosen Schaum.
$^1$H-NMR ($CDCl_3$): δ= 0,07 ppm (s, 12H); 0,53 (s, 3H); 0,90 (s, 18H); 0,91 (d, 3H); 1,30 (t, 3H); 2,07 (s, 3H); 2,82 (q, 2H); 4,09 (m, 2H); 5,82 (d, 1H); 5,96 (t, 1H); 6,18 (d, 1H); 6,62 (d, 1H); 6,86 (d, 1H)

87. Man setzt 129 mg des Acetates **90** analog 13. um und erhält 98 mg (7*E*)-(1*R*,3*R*)-1,3-Bis[[(1,1-dimethylethyl)dimethylsilyl]oxy]-24a-(5-ethylthien-2-yl)-24a-homo-19-nor-9,10-secochola-5,7-dien-24a-ol **91** als farblosen Schaum.
$^1$H-NMR ($CDCl_3$): δ= 0,07 ppm (s, 12H); 0,54 (s, 3H); 0,89 (s, 18H); 0,91 (d, 3H); 1,30 (t, 3H); 2,82 (q, 2H); 4,10 (m, 2H); 4,84 (m, 1H); 5,82 (d, 1H); 6,18 (d, 1H); 6,63 (d, 1H); 6,80 (d, 1H)

88. Man behandelt 98 mg des Alkohols **91** analog 14. und erhält nach Trennung der Diastereomeren (bzgl. C-24a) über HPLC 18 mg der Titelverbindung **92a** und 19 mg der Titelverbindung **92b** als farblose Schäume.
$^1$H-NMR ($CD_2Cl_2/CD_3OD$): **92a**: δ= 0,51 ppm (s, 3H); 0,89 (d, 3H); 1,27 (t, 3H); 2,78 (q, 2H); 3,94 (m, 1H); 4,02 (m, 1H); 4,75 (t, 1H); 5,84 (d, 1H); 6,25 (d, 1H); 6,59 (d, 1H); 6,71 (d, 1H)
**92b**: δ= 0,51 ppm (s, 3H); 0,90 (d, 3H); 1,27 (t, 3H); 2,78 (q, 2H); 3,94 (m, 1H); 4,02 (m, 1H); 4,76 (t, 1H); 5,84 (d, 1H); 6,25 (d, 1H); 6,59 (d, 1H); 6,71 (d, 1H)

**Beispiel 23**

**(7*E*)-(1*R*,3*R*,24a*R*)-24a-[5-(2-Hydroxyethyl)-4-methylthiazol-2-yl]-24a-homo-19-nor-9,10-secochola-5,7-dien-1,3,24a-triol 102a und (7*E*)-(1*R*,3*R*,24a*S*)-24a-[5-(2-Hydroxyethyl)-4-methylthiazol-2-yl]-24a-homo-19-nor-9,10-secochola-S,7-dien- 1,3,24a-triol 102b**

89. Man legt 2,09 ml 5-(2-Hydroxyethyl)-4-methylthiazol **93** in 150 ml Dichlormethan vor, gibt 7,8 ml 2,3-Dihydropyran und 400 mg Pyridinium-p-Toluolsulfonat hinzu und rührt 5 d bei Raumtemperatur. Anschließend setzt man Natriumchlorid-Lösung zu, extrahiert mit Essigester, wäscht die organische Phase mit Natriumhydrogencarbonat-Lösung und Natriumchlorid-Lösung, trocknet über Natriumsulfat und engt ein. Der Rückstand wird an Silicagel mit Essigester/Hexan chromatographiert, wobei man 3,8 g 5-[2-[(Tetrahydro-2*H*-pyran-2-yl)oxy]ethyl]-4-methylthiazol **94** als farbloses Öl erhält.

$^1$H-NMR ($CDCl_3$): δ= 1,48-1,90 ppm (m, 6H); 2,41 (s, 3H); 3,05 (t, 2H); 3,48 (m, 1H); 3,56 (dt, 1H); 3,75 (m, 1H); 3,93 (dt, 1H); 4,61 (m, 1H), 8,54 (s, 1H)

90. Man legt 2,78 g des Thiazols **94** in 25 ml Tetrahydrofuran vor und gibt bei -78°C 4,9 ml *n*-Butyllithium-Lösung (2,5 M in Hexan) zu. Nach 30 min bei dieser Temperatur tropft man 900 mg des Aldehydes **8** in 5 ml Tetrahydrofuran zu und rührt 1 h nach. Anschließend wird mit Natriumchlorid-Lösung gequencht, mit Essigester extrahiert, die organische Phase mit Natriumchlorid-Lösung gewaschen und über Natriumsulfat getrocknet. Das Lösungsmittel wird entfernt und der Rückstand wird an Silicagel mit Essigester/Hexan chromatographiert, wobei man 1,12 g [1*R*-[1α(1*R**),3aβ,4α,7aα]]-α-[4-[Octahydro-7a-methyl-4-[(triethylsilyl)oxy]-1*H*-inden-1-yl]pentyl]-5-[2-[(tetrahydro-2*H*-pyran-2-yl)oxy]ethyl]-4-methylthiazol-2-methanol **95** als farbloses Öl erhält.

$^1$H-NMR ($CDCl_3$): δ= 0,56 ppm (q, 6H); 0,89 (d, 3H); 0,90 (s, 3H); 0,96 (t, 9H); 2,32 (s, 3H); 3,00 (t, 2H); 3,48 (m, 1H); 3,58 (dt, 1H); 3,75 (m, 1H); 3,90 (dt, 1H); 4,03 (m, 1H); 4,61 (m, 1H); 4,87 (m, 1H); 6,62 (d, 1H); 6,80 (d, 1H)

91. Man behandelt 1,12 g des Alkohols **95** analog 9. und erhält 104 mg [1*R*-[1α(1*R**),3aβ,4α,7aα]]-5-[Octahydro-7a-methyl-4-[(triethylsilyl)oxy]-1*H*-inden-1-yl]-1-[5-[2-[(tetrahydro-2*H*-pyran-2-yl)oxy]ethyl]-4-methylthiazol-2-yl]hexyl-acetat **96** als farbloses Öl.

$^1$H-NMR ($CDCl_3$): δ= 0,55 ppm (q, 6H); 0,88/0,89 (d, 3H); 0,90 (s, 3H); 0,96 (t, 9H); 2,11 (s, 3H); 2,35 (s, 3H); 3,00 (t, 2H); 3,50 (m, 1H); 3,53 (dt, 1H); 3,74 (m, 1H); 3,90 (dt, 1H); 4,02 (m, 1H); 4,61 (m, 1H); 5,97 (t, 1H)

92. Man behandelt 1,04 g des Acetates **96** analog 10. und erhält 540 mg [1*R*-[1α(1*R**),3aβ,4α,7aα]]-1-[5-(Acetyloxy)-5-[5-[2-[(tetrahydro-2*H*-pyran-2-yl)oxy]ethyl]-4-methylthiazol-2-yl]-1-methylpentyl]octahydro-7a-methyl-1*H*-inden-4-ol **97** als farbloses Öl.

$^1$H-NMR ($CDCl_3$): δ= 0,88/0,89 ppm (d, 3H); 0,91 (s, 3H); 2,11 (s, 3H); 2,35 (s, 3H); 3,00 (t, 2H); 3,50 (m, 1H); 3,55 (dt, 1H); 3,74 (m, 1H); 3,90 (dt, 1H); 4,07 (m, 1H); 4,61 (m, 1H); 5,97 (t, 1H)

93. Man behandelt 540 mg des Alkohols **97** analog 11. und erhält 498 mg [1*R*-[1α(1*R**),3aβ,7aα]]-1-[5-(Acetyloxy)-5-[5-[2-[(tetrahydro-2*H*-pyran-2-yl)oxy]ethyl]-4-methylthiazol-2-yl]-1-methylpentyl]octahydro-7a-methyl-4*H*-inden-4-on **98** als farbloses Öl.

$^1$H-NMR ($CDCl_3$): δ= 0,62 ppm (s, 3H); 0,92/0,93 (d, 3H); 2,13 (s, 3H); 2,37 (s, 3H); 3,00 (t, 2H); 3,49 (m 1H); 3,54 (dt, 1H); 3,73 (m, 1H); 3,90 (dt, 1H); 4,61 (m, 1H); 5,97 (t, 1H)

94. Man setzt 656 mg des Phosphinoxides **13**, welches mit 0,55 ml *n*-Butyllithium-Lösung (2,5 M in Hexan) deprotoniert wurde, mit 300 mg des Ketons **98** analog 12. um und erhält 310 mg (7*E*)-(1*R*,3*R*)-1,3-Bis[[(1,1-dimethylethyl)dimethylsilyl]oxy]-24a-[5-[2-[(tetrahydro-2*H*-pyran-2-yl)oxy]ethyl]-4-methylthiazol-2-yl]-24a-homo-19-nor-9,10-secochola-5,7-dien-24a-yl-acetat **99** als farblosen Schaum.

$^1$H-NMR ($CDCl_3$): δ= 0,07 ppm (s, 12H); 0,53 (s, 3H); 0,90 (s, 18H); 0,91 (d, 3H); 2,12 (s, 3H); 2,38 (s, 3H); 3,01 (t, 2H); 3,50 (m, 1H); 3,56 (dt, 1H); 3,75 (m, 1H); 3,90 (dt, 1H); 4,09 (m, 2H); 4,61 (m, 1H); 5,82 (d, 1H); 5,97 (t, 1H); 6,18 (d, 1H)

95. Man setzt 151 mg des Acetates **99** analog 13. um und erhält 104 mg (7*E*)-(1*R*,3*R*)-1,3-Bis[[(1,1-dimethylethyl)dimethylsilyl]oxy]-24a-[5-[2-[(tetrahydro-2*H*-pyran-2-yl)oxy]ethyl]-4-methylthiazol-2-yl]-24a-homo-19-nor-9,10-secochola-5,7-dien-24a-ol **100** als farblosen Schaum.

$^1$H-NMR ($CD_2Cl_2$): δ= 0,04 ppm (s, 12H); 0,51 (s, 3H); 0,85 (s, 18H); 0,90 (d, 3H); 2,28 (s, 3H); 2,95 (t, 2H); 3,40 (m, 1H); 3,50 (dt, 1H); 3,70 (m, 1H); 3,82 (dt, 1H); 4,07 (m, 2H); 4,80 (m, 1H); 5,80 (d, 1H); 6,17 (d, 1H)

96. Man legt 170 mg des THP-Ethers **100** in 20 ml Dichlormethan vor, gibt bei -25°C 0,41 ml Dimethylaluminiumchlorid-Lösung zu und rührt 1 h nach, wobei sich das Gemisch auf 0°C erwärmt. Anschließend quencht man mit Natriumhydrogencarbonat-Lösung, extrahiert mit Essigester, wäscht die organische Phase mit Natriumchlorid-Lösung, trocknet über Natriumsulfat und entfernt das Solvens im Vakuum. Der Rückstand wird an Silicagel mit Essigester/Hexan chromatographiert, wobei man 153 mg (7*E*)-(1*R*,3*R*)-1,3-Bis[[(1,1-dimethylethyl)dimethylsilyl]oxy]-24a-[5-(2-hydroxyethyl)-4-methylthiazol-2-yl]-24a-homo-19-nor-9,10-secochola-5,7-dien-24a-ol **101** als farblosen Schaum erhält.

$^1$H-NMR ($CD_2Cl_2$): δ= 0,04 ppm (s, 12H); 0,51 (s, 3H); 0,85 (s, 18H); 0,90 (d, 3H); 2,28 (s, 3H); 2,92 (t, 2H); 3,26 (t, 2H); 4,08 (m, 2H); 4,80 (m, 1H); 5,80 (d, 1H); 6,17 (d, 1H)

97. Man behandelt 113 mg des Alkohols **101** analog 14. und erhält nach Trennung der Diastereomeren (bzgl. C-24a) über HPLC 21 mg der Titelverbindung **102a** und 24 mg der Titelverbindung **102b** als farblose Schäume.

$^1$H-NMR ($CD_2Cl_2/CD_3OD$): **102a:** δ= 0,50 ppm (s, 3H); 0,88 (d, 3H); 2,27 (s, 3H); 2,88 (t, 2H); 3,68 (d, 2H); 3,92 (m 1H); 4,00 (m, 1H); 4,74 (m, 1H); 5,82 (d, 1H); 6,24 (d, 1H)

**102b**: δ= 0,50 ppm (s, 3H); 0,89 (d, 3H); 2,28 (s, 3H); 2,88 (t, 2H); 3,68 (d, 2H); 3,92 (m 1H); 4,00 (m, 1H); 4,75 (dd, 1H); 5,82 (d, 1H); 6,24 (d, 1H)

**Beispiel 24**

**(7*E*)-(1*R*,3*R*)-1,3-Dihydroxy-24a-[5-(2-hydroxyethyl)-4-methylthiazol-2-yl]-24a homo-19-nor-9,10-secochola-5,7-dien-24a-on 104**

98. Man behandelt 70 mg des Alkohols **100** analog 25., wobei 40 mg (7*E*)-(1*R*,3*R*)-1,3-Bis[[(1,1-dimethylethyl) dimethylsilyl]oxy]-24a-[5-(2-hydroxyethyl)-4-methylthiazol-2-yl]-24a-homo-19-nor-9,10-secochola-5,7-dien-24a-on **103** als farbloser Schaum anfallen.
$^1$H-NMR ($CD_2Cl_2$): δ= 0,04 ppm (s, 12H); 0,51 (s, 3H); 0,84 (s, 18H); 0,93 (d, 3H); 2,37 (s, 3H); 3,01 (t, 2H); 3,40 (m, 1H); 3,53 (dt, 1H); 3,68 (m, 1H); 3,89 (dt, 1H); 4,08 (m, 2H); 4,54 (m,1H); 5,80 (d, 1H); 6,15 (d, 1H)

99. Man behandelt 40 mg des Ketons **103** analog 26., wobei 12 mg der Titelverbindung **104** als farbloser Schaum anfallen.
$^1$H-NMR ($CD_2Cl_2$): δ= 0,52 ppm (s, 3H); 0,95 (d, 3H); 2,41 (s, 3H); 3,01 (t, 2H); 3,80 (t, 2H); 3,95 (m, 1H); 4,05 (m, 1H); 5,82 (d, 1H); 6,25 (d, 1H)

**Beispiel 25**

**(7*E*)-(1*R*,3*R*,24a*R*)-24a-(Benzothiazol-2-yl)-24a-homo-19-nor-9,10-secochola-5,7-dien-1,3,24a-triol 111a** und **(7*E*)-(1*R*,3*R*,24a*S*)-24a-(Benzothiazol-2-yl)-24a-homo-19-nor-9,10-secochola-5,7-dien-1,3,24a-triol 111b**

100. Man legt 0,85 ml Benzothiazol in 20 ml Tetrahydrofuran vor und gibt bei -78°C 3,1 ml *n*-Butyllithium-Lösung (2,5 M in Hexan) zu. Nach 30 min bei dieser Temperatur tropft man 574 mg des Aldehydes **8** in 3 ml Tetrahydrofuran zu und rührt 1 h nach. Anschließend wird mit Natriumchlorid-Lösung gequencht, mit Essigester extrahiert, die organische Phase mit Natriumchlorid-Lösung gewaschen und über Natriumsulfat getrocknet. Das Lösungsmittel wird entfernt und der Rückstand wird an Silicagel mit Essigester/Hexan chromatographiert, wobei man 751 mg [1*R*-[1α(1*R**),3aβ,4α,7aα]]-α-[4-[Octahydro-7a-methyl-4-[(triethylsilyl)oxy]-1*H*-inden-1-yl]pentyl]benzothiazol-2-methanol **105** als farbloses Öl erhält.
$^1$H-NMR ($CDCl_3$): δ= 0,55 ppm (q, 6H); 0,89 (d, 3H); 0,91 (s, 3H); 0,95 (t, 9H); 4,02 (m, 1H); 5,10 (m, 1H); 7,40 (t, 1H); 7,48 (t, 1H); 7,90 (d, 1H); 8,00 (d, 1H)

101. Man behandelt 751 mg des Alkohols **105** analog 9. und erhält 764 mg [1*R*-[1α(1*R**),3aβ,4α,7aα]]-1-(Benzothiazol-2-yl)-5-[octahydro-7a-methyl-4-[(triethylsilyl)oxy]-1*H*-inden-1-yl)hexyl-acetat **106** als farbloses Öl.
$^1$H-NMR ($CDCl_3$: δ= 0,55 ppm (q, 6H); 0,88/0,89 (d, 3H); 0,90 (s, 3H); 0,96 (t, 9H); 2,20 (s, 3H); 4,02 (m, 1H); 6,15 (t, 1H); 7,40 (t, 1H); 7,49 (t, 1H); 7,90 (d, 1H); 8,03 (d, 1H)

102. Man behandelt 764 mg des Acetates **106** analog 10. und erhält 310 mg [1*R*-[1α(1*R**),3aβ,4α,7aα]]-1-[5-(Acetyloxy)-5-(benzothiazol-2-yl)-1-methylpentyl]octahydro-7a-methyl-1*H*-inden-4-ol **107** als farbloses Öl.
$^1$H-NMR ($CDCl_3$): δ= 0,88/0,89 ppm (d, 3H); 0,91 (s, 3H); 2,19 (s, 3H); 4,07 (m, 1H); 6,17 (t, 1H); 7,40 (t, 1H); 7,49 (t, 1H); 7,90 (d, 1H); 8,03 (d, 1H)

103. Man behandelt 310 mg des Alkohols **107** analog 11. und erhält 277 mg [1*R*-[1α(1*R**),3aβ,7aα]]-1[5-(Acetyloxy)-5-(benzothiazol-2-yl)-1-methylpentyl]octahydro-7a-methyl-4*H*-inden-4-on **108** als farbloses Öl.
$^1$H-NMR ($CDCl_3$): δ= 0,62 ppm (s, 3H); 0,92/0,93 (d, 3H); 2,19 (s, 3H); 6,17 (t, 1H); 7,40 (t, 1H); 7,49 (t, 1H); 7,89 (d, 1H); 8,02 (d, 1H)

104. Man setzt 736 mg des Phosphinoxides **13**, welches mit 0,62 ml *n*-Butyllithium-Lösung (2,5 M inHexan) deprotoniert wurde, mit 277 mg des Ketons **108** analog 12. um und erhält 193 mg (7*E*)-(1*R*,3*R*)-24a-(Acetyloxy)-24a-(2-benzothiazol-2-yl)-1,3-bis[[(1,1-dimethylethyl)dimethylsilyl]oxy]-24a-homo-19-nor-9,10-secochola-5,7-dien **109** als farblosen Schaum.
$^1$H-NMR ($CDCl_3$): δ= 0,07 ppm (s, 12H); 0,53 (s, 3H); 0,90 (s, 18H); 0,91 (d, 3H); 2,19 (s, 3H); 4,08 (m, 2H); 5,82 (d, 1H); 6,18 (d, 1H); 6,18 (m, 1H); 7,38 (t, 1H); 7,49 (t, 1H); 7,88 (d, 1H); 8,02 (d, 1H)

105. Man setzt 193 mg des Acetates **109** analog 13. um und erhält 177 mg (7*E*)-(1*R*,3*R*)-24a-(Benzothiazol-2-yl)-1,3-bis[[(1,1-dimethylethyl)dimethylsilyl]oxy]-24a-homo-19-nor-9,10-secochola-5,7-dien-24a-ol **110** als farblosen Schaum.
$^1$H-NMR ($CD_2Cl_2$): δ= 0,04 ppm (s, 12H); 0,51 (s, 3H); 0,86 (s, 18H); 0,92 (d, 3H); 4,06 (m, 2H); 5,04 (m, 1H); 5,80 (d, 1H); 6,16 (d, 1H); 7,33 (t, 1H); 7,43 (t, 1H); 7,87 (d, 1H); 7,92 (d, 1H)

106. Man behandelt 124 mg des Alkohols **110** analog 14. und erhält nach Trennung der Diastereomeren (bzgl. C-24a) über HPLC 23 mg der Titelverbindung **111a** und 22 mg der Titelverbindung **111b** als farblose Schäume.
$^1$H-NMR ($CD_2Cl_2$): **111a**: δ= 0,50 ppm (s, 3H); 0,90 (d, 3H); 3,95 (m 1H); 4,02 (m, 1H); 5,07 (m, 1H); 5,82 (d, 1H); 6,24 (d, 1H); 7,36 (t, 1H); 7,46 (t, 1H); 7,90 (d, 1H); 7,93 (d, 1H)
**111b**: δ= 0,51 ppm (s, 3H); 0,91 (d, 3H); 3,95 (m 1H); 4,02 (m, 1H); 5,06 (m, 1H); 5,82 (d, 1H); 6,24 (d, 1H); 7,36 (t, 1H); 7,46 (t, 1H); 7,90 (d, 1H); 7,93 (d, 1H)

**Beispiel 26**

**(7*E*)-(1*R*,3*R*)-24a-(Benzothiazol-2-yl)-1,3-dihydroxy-24a-homo-19-nor-9,10-secochola-5,7-dien-24a-on 113**

107. Man behandelt 53 mg des Alkohols **110** analog 25., wobei 39 mg (7*E*)-(1*R*,3*R*)-24a-(Benzothiazol-2-yl)-1,3-bis[[(1,1-dimethylethyl)dimethylsilyl]oxy]-24a-homo-19-nor-9,10-secochola-5,7-dien-24a-on **112** als farbloser Schaum anfallen.
$^1$H-NMR ($CD_2Cl_2$): δ= 0,04 ppm (s, 12H); 0,52 (s, 3H); 0,85 (s, 18H); 0,96 (d, 3H); 4,06 (m, 2H); 4,54 (m,1H); 5,80 (d, 1H); 6,15 (d, 1H); 7,52 (t, 1H); 7,59 (t, 1H); 8,01 (d, 1H); 8,15 (d, 1H)

108. Man behandelt 39 mg des Ketons **112** analog 26., wobei 20 mg der Titelverbindung **113** als farbloser Schaum anfallen.
$^1$H-NMR ($CD_2Cl_2$): δ= 0,53 ppm (s, 3H); 0,95 (d, 3H); 3,94 (m, 1H); 4,02 (m, 1H); 5,82 (d, 1H); 6,25 (d, 1H); 7,53 (t, 1H); 7,56 (t, 1H); 7,98 (d, 1H); 8,13 (d, 1H)

**Beispiel 27**

**(7*E*)-(1*R*,3*R*,24a*R*)-24a-(Benzofuran-2-yl)-24a-homo-19-nor-9,10-secochola-5,7-dien-1,3,24a-triol 120a und (7*E*)-(1*R*,3*R*,24a*S*)-24a-(Benzofuran-2-yl)-24a-homo-19-nor-9,10-secochola-5,7-dien-1,3,24a-triol 120b**

109. Man legt 1,44 ml 2,3-Benzofuran in 25 ml Tetrahydrofuran vor und gibt bei -78°C 5,2 ml *n*-Butyllithium-Lösung (2,5 M in Hexan) zu. Nach 30 min bei dieser Temperatur tropft man 960 mg des Aldehydes **8** in 5 ml Tetrahydrofuran zu und rührt 1 h nach. Anschließend wird mit Natriumchlorid-Lösung gequencht, mit Essigester extrahiert, die organische Phase mit Natriumchlorid-Lösung gewaschen und über Natriumsulfat getrocknet. Das Lösungsmittel wird entfernt und der Rückstand wird an Silicagel mit Essigester/Hexan chromatographiert, wobei man 1,06 g [1*R*-[1α(1*R**),3aβ,4α,7aα]]-α-[4-[Octahydro-7a-methyl-4-[(triethylsilyl)oxy]-1*H*-inden-1-yl]pentyl]benzofuran-2-methanol **114** als farbloses Öl erhält.
$^1$H-NMR ($CDCl_3$): δ= 0,56 ppm (q, 6H); 0,89 (d, 3H); 0,91 (s, 3H); 0,95 (t, 9H); 4,02 (m, 1H); 4,83 (m, 1H); 7,22 (t, 1H); 7,29 (t, 1H); 7,48 (d, 1H); 7,57 (d, 1H)

110. Man legt 1,06 g des Alkohols **114** in 18 ml Dichlormethan vor und setzt 0,83 ml 2,3-Dihydropyran sowie 48 mg Pyridinium-p-Toluolsulfonat zu. Man rührt 1 d bei Raumtemperatur und gibt dann Natriumchlorid-Lösung zu. Es wird mit Essigester extrahiert, die organische Phase mit Natriumchlorid-Lösung gewaschen, über Natriumsulfat getrocknet und eingeengt. Der Rückstand wird an Silicagel mit Essigester/Hexan chromatographiert, wobei man 1,11 g [1*R*-[1α(1*R**),3aβ,4α,7aα]]1-[5-(Benzofuran-2-yl)-1-methyl-5-[(tetrahydro-2*H*-pyran-2-yl)oxy]pentyl]octahydro-7a-methyl-4-[(triethylsilyl)oxy]-1*H*-inden **115** als farbloses Öl erhält.
$^1$H-NMR ($CDCl_3$): δ= 0,55 ppm (q, 6H); 0,88/0,89 (d, 3H); 0,90 (s, 3H); 0,96 (t, 9H); 3,40 (m, 1H); 3,55 (m, 1H); 4,02 (m, 1H); 4,77 (t, 1H); 6,65 (m, 1H); 7,23 (m, 2H); 7,49 (d, 1H); 7,54 (d, 1H)

111. Man legt 1,11 g des Silylethers **115** in 50 ml Tetrahydrofuran vor, gibt 1,81 g Tetrabutylammoniumfluorid zu und rührt 1 d bei Raumtemperatur. Anschließend gibt man Natriumchlorid-Lösung zu, extrahiert mit Essigester, wäscht mit Natriumchlorid-Lösung, trocknet über Natriumsulfat und engt ein. Der Rückstand wird an Silicagel mit Essigester/Hexan chromatographiert, wobei man 760 mg [1*R*-[1α(1*R**),3aβ,4α,7aα]]-1-[5-(Benzofuran-2-yl)-1-methyl-5-[(tetrahydro-2H-pyran-2-yl)oxy]pentyl]octahydro-7amethyl-1*H*-inden-4-ol 116 als farbloses Öl erhält.
$^1$H-NMR ($CDCl_3$): δ= 0,88/0,89 ppm (d, 3H); 0,91 (s, 3H); 3,40 (m, 1H); 3,55 (m, 1H); 4,04 (m, 1H); 4,60 (m, 1H); 4,78 (t, 1H); 6,65 (m, 1H); 7,25 (m, 2H); 7,48 (d, 1H); 7,55 (d, 1H)

112. Man behandelt 750 mg des Alkohols **116** analog 11. und erhält 669 mg [1*R*-[1α(1*R**),3aβ,7aα]]-1-[5-(Benzofuran-2-yl)-1-methyl-5-[(tetrahydro-2*H*-pyran-2-yl)oxy]pentyl]octahydro-7a-methyl-4*H*-inden-4-on 117 als farbloses Öl.
$^1$H-NMR ($CDCl_3$): δ= 0,62 ppm (s, 3H); 0,92/0,93 (d, 3H); 3,40 (m, 1H); 3,54 (m, 1H); 4,60 (m, 1H); 4,80 (t, 1H); 6,65 (m, 1H); 7,25 (m, 2H); 7,49 (d, 1H); 7,55 (d, 1H)

113. Man legt 314 mg des THP-Ethers **117** in 10 ml Methanol vor und fügt 244 mg Pyridinium-p-Toluolsulfonat zu. Es wird 1 h bei Raumtemperatur gerührt und anschließend mit Natriumchlorid-Lösung behandelt. Man extrahiert mit Essigester, wäscht die organische Phase mit Natriumhydrogencarbonat-Lösung und Natriumchlorid-Lösung, trocknet über Natriumsulfat und engt ein. Der Rückstand wird an Silicagel mit Essigester/Hexan chromatographiert, wobei man 760 mg [1*R*-[1α(1*R**),3aβ,7aα]]-1-[5-(Benzofuran-2-yl)-5-hydroxy-1-methylpentyl]octahydro-7a-methyl-4*H*-inden-4-on **118** als farbloses Öl erhält.
$^1$H-NMR ($CDCl_3$): δ= 0,62 ppm (s, 3H); 0,92/0,93 (d, 3H); 4,81 (m, 1H); 6,61 (s, 1H); 7,22 (t, 1H); 7,28 (t, 1H); 7,47 (d, 1H); 7,54 (d, 1H)

114. Man behandelt 180 mg des Alkohols **118** analog 9. und erhält 169 mg [1*R*-[1α(1*R**),3aβ,7aα]]-1-[5-(Acetyloxy)-5-(benzofuran-2-yl)-1-methylpentyl]octahydro-7a-methyl-4*H*-inden-4-on **119** als farbloses Öl.

$^1$H-NMR ($CDCl_3$): δ= 0,62 ppm (s, 3H); 0,92/0,93 (d, 3H); 2,10 (s, 3H); 5,97 (m, 1H); 6,69 (s, 1H); 7,23 (t, 1H); 7,30 (t, 1H); 7,50 (d, 1H); 7,55 (d, 1H)

115. Man setzt 444 mg des Phosphinoxides **13**, welches mit 0,37 ml *n*-Butyllithium-Lösung (2,5 M in Hexan) deprotoniert wurde, mit 160 mg des Ketons **119** analog 12. um und erhält 189 mg (7*E*)-(1*R*,3*R*)-24a-(Acetyloxy)-24a-(2-benzofuran-2-yl)-1,3-bis[[(1,1-dimethylethyl)dimethylsilyl]oxy]-24a-homo-19-nor-9,10-secochola-5,7-dien **120** als farblosen Schaum.

$^1$H-NMR ($CDCl_3$): δ= 0,07 ppm (s, 12H); 0,53 (s, 3H); 0,90 (s, 18H); 0,91 (d, 3H); 2,10 (s, 3H); 4,08 (m, 2H); 5,81 (d, 1H); 5,97 (t, 1H); 6,18 (m, 1H); 6,70 (s, 1H); 7,22 (t, 1H); 7,30 (t, 1H); 7,49 (d, 1H); 7,55 (d, 1H)

116. Man setzt 142 mg des Acetates **120** analog 13. um und erhält 134 mg (7*E*)-(1*R*,3*R*)-24a-(Benzothiazol-2-yl)-1,3-bis[[(1,1-dimethylethyl)dimethylsilyl]oxy]-24a-homo-19-nor-9,10-secochola-5,7-dien-24a-ol **121** als farblosen Schaum.

$^1$H-NMR ($CDCl_3$): δ= 0,07 ppm (s, 12H); 0,53 (s, 3H); 0,89 (s, 18H); 0,94 (d, 3H); 4,08 (m, 2H); 4,83 (m, 1H); 5,82 (d, 1H); 6,18 (d, 1H); 6,62 (s, 1H); 7,22 (t, 1H); 7,28 (t, 1H); 7,47 (d, 1H); 7,55 (d, 1H)

117. Man behandelt 134 mg des Alkohols **121** analog 14. und erhält nach Trennung der Diastereomeren (bzgl. C-24a) über HPLC 29 mg der Titelverbindung **122a** und 26 mg der Titelverbindung **122b** als farblose Schäume.

$^1$H-NMR ($CD_2Cl_2$/$CD_3OD$): **122a:** δ= 0,50 ppm (s, 3H); 0,88 (d, 3H); 3,92 (m, 1H); 4,00 (m, 1H); 4,71 (t, 1H); 5,81 (d, 1H); 6,22 (d, 1H); 6,58 (s, 1H); 7,18 (t, 1H); 7,21 (t, 1H); 7,41 (d, 1H); 7,52 (d, 1H)

**122b:** δ= 0,50 ppm (s, 3H); 0,89 (d, 3H); 3,92 (m 1H); 4,00 (m, 1H); 4,72 (t, 1H); 5,81 (d, 1H); 6,22 (d, 1H); 6,58 (s, 1H); 7,18 (t, 1H); 7,21 (t, 1H); 7,41 (d, 1H); 7,53 (d, 1H)

**Beispiel 28**

**(7*E*)-(1*R*,3*R*)-24a-(Benzofuran-2-yl)-1,3-dihydroxy-24a-homo-19-nor-9,10- secochola-5,7-dien-24a-on 124**

118. Man behandelt 49 mg des Alkohols **121** analog 25., wobei 39 mg (7*E*)-(1*R*,3*R*)-24a-(Benzofuran-2-yl)-1,3-bis[[(1,1-dimethylethyl)dimethylsilyl]oxy]-24a-homo-19-nor-9,10-secochola-5,7-dien-24a-on **123** als farbloser Schaum anfallen.

$^1$H-NMR ($CD_2Cl_2$): δ= 0,04 ppm (s, 12H); 0,52 (s, 3H); 0,85 (s, 18H); 0,97 (d, 3H); 4,06 (m, 2H); 4,54 (m,1H); 5,81 (d, 1H); 6,16 (d, 1H); 7,29 (t, 1H); 7,46 (t, 1H); 7,47 (s, 1H); 7,59 (d, 1H); 7,72 (d, 1H)

119. Man behandelt 39 mg des Ketons **123** analog 26., wobei 12 mg der Titelverbindung **124** als farbloser Schaum anfallen.

$^1$H-NMR ($CD_2Cl_2$): δ= 0,53 ppm (s, 3H); 0,95 (d, 3H); 3,95 (m, 1H); 4,03 (m, 1H); 5,84 (d, 1H); 6,25 (d, 1H); 7,30 (t, 1H); 7,46 (t, 1H); 7,49(s, 1H); 7,56 (d, 1H); 7,72 (d, 1H)

**Beispiel 29**

**(7*E*)-(1*R*,3*R*,24a*R*)-24a-(Benzothiophen-2-yl)-24a-homo-19-nor-9,10-secochola-5,7-dien-1,3,24a-triol 131a** und **(7*E*)-(1*R*,3*R*,24a*S*)-24a-(Benzothiophen-2-yl)-24a-homo-19-nor-9,10-secochola-5,7-dien-1,3,24a-triol 131b**

120. Man legt 0,79 ml 1-Benzothiophen in 12 ml Tetrahydrofuran vor und gibt bei -78°C 2,72 ml *n*-Butyllithium-Lösung (2,5 M in Hexan) zu. Nach 30 min bei dieser Temperatur tropft man 500 mg des Aldehydes **8** in 3 ml Tetrahydrofuran zu und rührt 1 h nach. Anschließend wird mit Natriumchlorid-Lösung gequencht, mit Essigester extrahiert, die organische Phase mit Natriumchlorid-Lösung gewaschen und über Natriumsulfat getrocknet. Das Lösungsmittel wird entfernt und der Rückstand wird an Silicagel mit Essigester/Hexan chromatographiert, wobei man 679 mg [1*R*-[1α(1*R**),3aβ,4aα,7aα]]-α-[4-[Octahydro-7a-methyl-4-[(triethylsilyl)oxy]-1*H*-inden-1-yl]pentyl]benzothiophen-2-methanol **125** als farbloses Öl erhält.

$^1$H-NMR ($CDCl_3$): δ= 0,56 ppm (q, 6H); 0,89 (d, 3H); 0,91 (s, 3H); 0,96 (t, 9H); 4,03 (m, 1H); 5,02 (m, 1H); 7,36 (m, 3H); 7,45 (d, 1H); 7,88 (d, 2H)

121. Man behandelt 670 mg des Alkohols **125** analog 9. und erhält 699 mg [1*R*-[1α(1*R**),3aβ,4α,7aα]]-1-(Benzothiophen-2-yl)-5-[octahydro-7a-methyl-4-[(triethylsilyl)oxy]-1*H*-inden-1-yl)hexyl-acetat **126** als farbloses Öl.

$^1$H-NMR ($CDCl_3$): δ= 0,55 ppm (q, 6H); 0,88/0,89 (d, 3H); 0,90 (s, 3H); 0,95 (t, 9H); 2,10 (s, 3H); 4,02 (m, 1H); 6,10 (t, 1H); 7,33 (m, 3H); 7,75 (d, 1H); 7,81 (d, 1H)

122. Man behandelt 336 mg des Acetates **126** analog 10. und erhält 221 mg [1*R*-[1α(1*R**),3aβ,4α,7aα]]-1-[5-(Acetyloxy)-5-(benzothiophen-2-yl)-1-methylpentyl]octahydro-7a-methyl-1*H*-inden-4-ol **127** als farbloses Öl.

$^1$H-NMR ($CDCl_3$): δ= 0,88/0,89 ppm (d, 3H); 0,91/0,92 (s, 3H); 2,10 (s, 3H); 4,07 (m, 1H); 6,10 (t, 1H); 7,33 (m, 3H); 7,74 (d, 1H); 7,81 (d, 1H)

123. Man behandelt 215 mg des Alkohols **127** analog 11. und erhält 195 mg [1*R*-[1 α(1*R**),3aβ,7aα]]-1-[5-(Acetyloxy)-5-(benzothiophen-2-yl)-1-methylpentyl]octahydro-7a-methyl-4*H*-inden-4-on **128** als farbloses Öl.

$^1$H-NMR ($CDCl_3$): δ= 0,62 ppm (s, 3H); 0,92/0,93 (d, 3H); 2,10 (s, 3H); 6,10 (t, 1H); 7,35 (m, 3H); 7,73 (d, 1H); 7,82 (d, 1H)

124. Man setzt 480 mg des Phosphinoxides **13**, welches mit 0,4 ml *n*-Butyllithium-Lösung (2,5 M in Hexan) deprotoniert wurde, mit 180 mg des Ketons **128** analog 12. um und erhält 266 mg (7*E*)-(1*R*,3*R*)-24a-(Acetyloxy)-24a-(2-benzothiophen-2-yl)-1,3-bis[[(1,1-dimethylethyl)dimethyl-silyl]oxy]- 24a-homo-19-nor-9,10-secochola-5,7-dien **129** als farblosen Schaum.

$^1$H-NMR ($CDCl_3$): δ= 0,07 ppm (s, 12H); 0,53 (s, 3H); 0,90 (s, 18H); 0,91 (d, 3H); 2,10 (s, 3H); 4,08 (m, 2H); 5,80 (d, 1H); 6,10 (t, 1H); 6,18 (m, 1H); 7,33 (m, 2H); 7,72 (d, 1H); 7,82 (d, 1H)

125. Man setzt 260 mg des Acetates **129** analog 13. um und erhält 228 mg (7*E*)-(1*R*,3*R*)-24a-(Benzothiophen-2-yl)-1,3-bis[[(1,1-dimethylethyl)dimethylsilyl]oxy]-24a-homo-19-nor-9,10-secochola-5,7-dien-24a-ol **130** als farblosen Schaum.

$^1$H-NMR ($CDCl_3$): δ= 0,07 ppm (s, 12H); 0,53 (s, 3H); 0,89 (s, 18H); 0,93 (d, 3H); 4,08 (m, 2H); 5,00 (m, 1H); 5,82 (d, 1H); 6,19 (d, 1H); 7,21 (s, 1H); 7,33 (m, 2H); 7,73 (d, 1H); 7,83 (d, 1H)

126. Man behandelt 172 mg des Alkohols **130** analog 14. und erhält nach Trennung der Diastereomeren (bzgl. C-24a) über HPLC 22 mg der Titelverbindung **131a** und 24 mg der Titelverbindung **131b** als farblose Schäume.

$^1$H-NMR ($CD_2Cl_2/CD_3OD$): **131a**: δ= 0,50 ppm (s, 3H); 0,88 (d, 3H); 3,92 (m 1H); 4,00 (m, 1H); 4,90 (t, 1H); 5,81 (d, 1H); 6,22 (d, 1H); 7,16 (s, 1H); 7,26 (t, 1H); 7,28 (t, 1H); 7,68 (d, 1H); 7,77 (d, 1H)

**131b:** δ= 0,50 ppm (s, 3H); 0,89 (d, 3H); 3,92 (m 1H); 4,00 (m, 1H); 4,91 (t, 1H); 5,81 (d, 1H); 6,22 (d, 1H); 7,16 (s, 1H); 7,26 (t, 1H); 7,27 (t, 1H); 7,68 (d, 1H); 7,77 (d, 1H)

**Beispiel 30**

**(7*E*)-(1*R*,3*R*)-24a-(Benzothiophen-2-yl)-1,3-dihydroxy-24a-homo-19-nor-9,10-secochola-5,7-dien-24a-on 133**

127. Man behandelt 50 mg des Alkohols **130** analog 25., wobei 38 mg (7*E*)-(1*R*,3*R*)-24a-(Benzothiophen-2-yl)-1,3-bis[[(1,1-dimethylethyl)dimethylsilyl]oxy]-24a-homo-19-nor-9,10-secochola-5,7-dien-24a-on **132** als farbloser Schaum anfallen.

$^1$H-NMR ($CD_2Cl_2$): δ= 0,04 ppm (s, 12H); 0,52 (s, 3H); 0,85 (s, 18H); 0,96 (d, 3H); 4,05 (m, 2H); 5,82 (d, 1H); 6,16 (d, 1H); 7,41 (t, 1H); 7,46 (t, 1H); 7,88 (d, 1H); 7,91 (d, 1H); 7,95 (s, 1H)

128. Man behandelt 38 mg des Ketons **132** analog 26., wobei 21 mg der Titelverbindung **133** als farbloser Schaum anfallen.

$^1$H-NMR ($CD_2Cl_2$): δ= 0,53 ppm (s, 3H); 0,97 (d, 3H); 3,96 (m, 1H); 4,03 (m, 1H); 5,84 (d, 1H); 6,26 (d, 1H); 7,39 (t, 1H); 7,46 (t, 1H); 7,88 (d, 1H); 7,90 (d, 1H); 7,97 (s, 1H)

**Beispiel 31**

**(7*E*)-(1*R*,3*R*,24a*R*)-24a-(1-Methylbenzimidazol-2-yl)-19-nor-9,10-secochola-5,7-dien-1,3,24a-triol 140a** und **(7*E*) -(1*R*,3*R*,24a*S*)-24a-(1-Methylbenzimidazol-2-yl)-19-nor-9,10-secochola-5,7-dien-1,3,24a-triol 140b**

129. Man legt 899 mg 1-Methylbenzimidazol in 12 ml Tetrahydrofuran vor und gibt bei -78°C 2,72 ml *n*-Butyllithium-Lösung (2,5 M in Hexan) zu. Nach 30 min bei dieser Temperatur tropft man 500 mg des Aldehydes **8** in 3 ml Tetrahydrofuran zu und rührt 1 h nach. Anschließend wird mit Natriumchlorid-Lösung gequencht, mit Essigester extrahiert, die organische Phase mit Natriumchlorid-Lösung gewaschen und über Natriumsulfat getrocknet. Das Lösungsmittel wird entfernt und der Rückstand wird an Silicagel mit Essigester/Hexan chromatographiert, wobei man 487 mg [1*R*-[1α(1*R**),3aβ,4α,7aα]]-α-[4-[Octahydro-7a-methyl-4-[(triethylsilyl)oxy]-1*H*-inden-1-yl]pentyl]-1-methylbenzimidazol-2-methanol **134** als farbloses Öl erhält.

$^1$H-NMR ($CDCl_3$): δ= 0,56 ppm (q, 6H); 0,89 (d, 3H); 0,91 (s, 3H); 0,96 (t, 9H); 3,78 (s, 3H); 4,02 (m, 1H); 4,93 (m, 1H); 7,25 (m, 3H); 7,71 (d, 1H)

130. Man behandelt 480 mg des Alkohols **134** analog 9. und erhält 382 mg [1*R*-[1α(1*R**),3aβ,4α,7aα]]-5-[Octahydro-7a-methyl-4-[(triethylsilyl)oxy]-1*H*-inden-1-yl]-1-(1-methylbenzimidazol-2-yl)hexyl-acetat **135** als farbloses Öl.

$^1$H-NMR ($CDCl_3$): δ= 0,56 ppm (q, 6H); 0,88/0,89 (d, 3H); 0,90 (s, 3H); 0,95 (t, 9H); 2,11 (s, 3H); 3,87 (s, 3H); 4,02 (m, 1H); 6,02 (t, 1H); 7,32 (m, 3H); 7,80 (d, 1H)

131. Man behandelt 375 mg des Acetates **135** analog 10. und erhält 259 mg [1*R*-[1α(1*R**),3aβ,4α,7aα]]-1-[5-(Acetyloxy)-1-methyl-5-(1-methylbenzimidazol-2-yl)pentyl]octahydro-7a-methyl-1*H*-inden-4-ol **136** als farbloses Öl.

$^1$H-NMR ($CDCl_3$): δ= 0,88/0,89 ppm (d, 3H); 0,91/0,92 (s, 3H); 2,11 (s, 3H); 3,88 (s, 3H); 4,07 (m, 1H); 6,03 (t, 1H); 7,32 (m, 3H); 7,79 (d, 1H)

132. Man behandelt 250 mg des Alkohols **136** analog 11. und erhält 118 mg [1*R*-[1α(1*R**),3aβ,7aα]]-1-[5-(Acetyloxy)-1-methyl-5-(1-methylbenzimidazol-2-yl)pentyl]octahydro-7a-methyl-4*H*-inden-4-on **137** als farbloses Öl.
$^1$H-NMR ($CDCl_3$): δ= 0,62/0,63 ppm (s, 3H); 0,92/0,93 (d, 3H); 2,14 (s, 3H); 3,88 (s, 3H); 6,03 (t, 1H); 7,32 (m, 3H); 7,80 (d, 1H)

133. Man setzt 317 mg des Phosphinoxides **13**, welches mit 0,27 ml n-Butyllithium-Lösung (2,5 M in Hexan) deprotoniert wurde, mit 118 mg des Ketons **137** analog 12. um und erhält 208 mg (7*E*)-(1*R*,3*R*)-24a-(Acetyloxy)-1,3-bis[[(1,1-dimethylethyl)dimethylsilyl]oxy]-24a-(1-methylbenz-imidazol-2-yl)-24a-homo-19-nor-9,10-secochola-5,7-dien **138** als farblosen Schaum.
$^1$H-NMR ($CDCl_3$): δ= 0,07 ppm (s, 12H); 0,52 (s, 3H); 0,90 (s, 18H); 0,91 (d, 3H); 2,10 (s, 3H); 3,85 (s, 3H); 4,08 (m, 2H); 5,80 (d, 1H); 6,02 (t, 1H); 6,16 (d, 1H); 7,33 (m, 3H); 7,78 (d, 1H)

134. Man setzt 218 mg des Acetates **138** analog 13. um und erhält 167 mg (7*E*)-(1*R*,3*R*)-1 ,3-Bis[[dimethyl (1,1-dimethylethyl)silyl]oxy]-24a-(1 -methylbenzimidazol-2-yl)-24ahomo-19-nor-9,10-secochola-5,7-dien-24a-ol **139** als farblosen Schaum.
$^1$H-NMR ($CD_2Cl_2$): δ= 0,04 ppm (s, 12H); 0,50 (s, 3H); 0,85 (s, 18H); 0,90 (d, 3H); 3,73 (s, 3H); 4,06 (m, 2H); 4,89 (m, 1H); 5,80 (d, 1H); 6,15 (d, 1H); 7,22 (m, 3H); 7,60 (m, 1H)

135. Man behandelt 115 mg des Alkohols **139** analog 14. und erhält nach Trennung der Diastereomeren (bzgl. C-24a) über HPLC 23 mg der Titelverbindung **140a** und 22 mg der Titelverbindung **140b** als farblose Schäume.
$^1$H-NMR ($CD_2Cl_2$): **140a:** δ= 0,50 ppm (s, 3H); 0,88 (d, 3H); 3,72 (s, 3H); 3,96 (m, 1H); 4,02 (m, 1H); 4,88 (t, 1H); 5,82 (d, 1H); 6,23 (d, 1H); 7,23 (m, 3H); 7,62 (d, 1H)
**140b:** δ= 0,50 ppm (s, 3H); 0,89 (d, 3H); 3,72 (s, 3H); 3,96 (m, 1H); 4,02 (m, 1H); 4,89 (t, 1H); 5,82 (d, 1H); 6,23 (d, 1H); 7,23 (m, 3H); 7,62 (d, 1H)

**Beispiel 32**

**(7*E*)-(1*R*,3*R*)-1,3-Dihydroxy-24a-(1-methylbenzimidazol-2-yl)-24a-homo-19-nor-9,10-secochola-5,7-dien-24a-on 142**

136. Man behandelt 45 mg des Alkohols **139** analog 25., wobei 26 mg (7*E*)-(1*R*,3*R*)-1,3-Bis[[( 1,1 dimethylethyl)dimethylsilyl]oxy]-24a-(1-methylbenzimidazol-2-yl)-24ahomo-19-nor-9,10-secochola-5,7-dien-24a-on **141** als farbloser Schaum anfallen.
$^1$H-NMR ($CD_2Cl_2$): δ= 0,04 ppm (s, 12H); 0,52 (s, 3H); 0,86 (s, 18H); 0,97 (d, 3H); 3,81 (s, 3H); 4,07 (m, 2H); 5,82 (d, 1H); 6,16 (d, 1H); 7,32 (t, 1H); 7,43 (m, 2H); 7,80 (d, 1H)

137. Man behandelt 26 mg des Ketons **141** analog 26., wobei 8 mg der Titelverbindung **142** als farbloser Schaum anfallen.
$^1$H-NMR ($CD_2Cl_2$): δ= 0,53 ppm (s, 3H); 0,97 (d, 3H); 3,82 (s, 3H); 3,96 (m, 1H); 4,03 (m, 1H); 4,05 (s, 3H); 5,84 (d, 1H); 6,26 (d, 1H); 7,31 (t, 1H); 7,43 (m, 2H); 7,80 (d, 1H)

**Beispiel 33**

**(7*E*)-(1*R*,3*R*)-1-(1,3-Dihydroxy-24a-homo-19-nor-9,10-secochola-5,7-dien-24a-yl)-3-[(4-methoxyphenyl)methoxy]-1*H* pyrazol-4-carbonsäureethylester 153**

138. Man legt 10,1 ml Ethoxymethylenmalonsäurediethylester **143** und 3,3 ml Hydrazin in 100 ml Ethanol vor und erhitzt für 1 h zum Sieden. Nach dem Abkühlen wird Natriumchlorid-Lösung zugegeben, mit Essigester extrahiert, die organische Phase mit Natriumchlorid-Lösung gewaschen, über Natriumsulfat getrocknet und das Lösungsmittel entfernt. Der Rückstand wird an Silicagel mit Essigester/Hexan chromatographiert, wobei man 11,2 g Ethoxymethylenmalonsäure-ethylesterhydrazid **144** als farbloses Öl erhält.
$^1$H-NMR ($CDCl_3$/$CD_3OD$): δ= 1,25 ppm (t, 3H); 1,29 (t, 3H); 3,78 (sbr, 1H); 4,12 (q, 2H); 4,23 (q, 2H); 8,18 (s, 1H)

139. Man behandelt 11,2 g des Hydrazides **144** mit 30 ml wässriger NatriumhydroxidLösung (25%) und rührt 1 h bei Raumtemperatur. Anschließend neutralisiert man mit verdünnter Salzsäure, extrahiert mit Essigester, wäscht die organische Phase mit Natriumchlorid-Lösung, trocknet über Natriumsulfat und engt ein. Der Rückstand wird an Silicagel mit Essigester/Hexan chromatographiert, wobei man 2,6 g 3-Hydroxypyrazol-4-carbonsäureethylester **145** als farbloses Öl erhält.
$^1$H-NMR ($CDCl_3$/$CD_3OD$): δ= 1,25 ppm (t, 3H); 4,20 (q, 2H); 4,25 (sbr, 1H); 7,63 (s, 1H)

140. Man legt 516 mg Natriumhydrid (80% Suspension) in 10 ml Tetrahydrofuran/Dimethylformamid (1:1) vor und tropft das Pyrazol **145** in 1 ml Tetrahydrofuran/Dimethylformamid (1:1) zu. Man rührt 10 min nach und gibt dann 2,3 ml p-Methoxybenzylbromid zu. Es wird 4 h zum Sieden erhitzt und anschließend nach dem Abkühlen Natriumchlorid-Lösung zugesetzt, mit Essigester extrahiert, die organische Phase mit Natriumchlorid-Lösung gewaschen, über Natriumsulfat getrocknet und das Solvens entfernt. Der Rückstand wird an Silicagel mit Essigester/Hexan chromatographiert, wobei man nacheinander 1,6 g 3-Hydroxy-1-[(4-methylphenyl)methyl]pyrazol-4-carbonsäureester **146** sowie 600 mg 3-[[(4-Methoxyphenyl)methyl]oxy]pyrazol-4-carbonsäureethylester **147** als farblose Öle erhält.

$^1$H-NMR ($CDCl_3$): **146**: δ= 1,27 ppm (t, 3H); 3,80 (s, 3H); 4,22 (q, 2H); 5,04 (s, 2H); 7,18 (d, 2H); 7,41 (d, 2H); 7,60 (s, 1H) **147**: δ= 1,30 ppm (t, 3H); 3,80 (s, 3H); 4,28 (q, 2H); 5,30 (s, 2H); 6,89 (d, 2H); 7,42 (d, 2H); 7,88 (s, 1H)

141. Man legt 540 mg des Alkohols **7** in 30 ml Dichlormethan vor und gibt bei 0°C 0,4 ml Triethylamin und 0,24 ml Methansulfonylchlorid hinzu. Man rührt 1 h bei 0°C, gibt dann Natriumchlorid-Lösung hinzu, extrahiert mit Dichlormethan, wäscht die organische Phase mit Natriumchlorid-Lösung, trocknet über Natriumsulfat und entfernt das Lösungsmittel, wobei 510 mg [1*R*-[1α(1*R**),3aβ,4α,7aα]]-5-[Octahydro-7a-methyl-4-[(triethylsilyl)oxy]-1*H*-inden-1-yl] hexyl-(methylsulfonat) **148** als gelbliches Öl anfallen, welchs roh weiter umgesetzt wird.

142. Man legt 36 mg Natriumhydrid (80% Suspension) in 10 ml Dimethylformamid vor und gibt bei 0°C 240 mg des Mesylates **148** und 88 mg Natriumiodid zu. Nach 30 min fügt man den Benzylether **147** in 2 ml Dimethylformamid zu und rührt über Nacht bei Raumtemperatur nach. Anschließend wird mit Natriumchlorid-Lösung gequencht, mit Essigester extrahiert, mit Natriumchlorid-Lösung gewaschen, über Natriumsulfat getrocknet und das Solvens entfernt. Der Rückstand wird an Silicagel mit Essigester/Hexan chromatographiert, wobei man 580 mg [1*R*-[1α(1*R**),3aβ,4α,7aα]]-1-[5-[Octahydro-7amethyl-4-[(triethylsilyl)oxy]-1*H*-inden-1-yl]hexyl]-3-[(4-methoxyphenyl)methoxy]-1*H*pyrazol-4-carbonsäureethylester **149** als farbloses Öl erhält.

$^1$H-NMR ($CDCl_3$: δ= 0,55 ppm (q, 6H); 0,88/0,89 (d, 3H); 0,90 (s, 3H); 0,96 (t, 9H); 1,33 (t, 3H); 3,80 (s, 3H); 3,92 (t, 2H); 4,03 (m, 1H); 4,24 (q, 2H); 5,22 (s, 2H); 6,90 (d, 2H); 7,42 (d, 2H); 7,69 (s, 1H)

143. Man legt 500 mg des Silylethers **149** in 50 ml Tetrahydrofuran vor und gibt 262 mg Tetrabutylammoniumfluorid (Hydrat) dazu. Es wird 24 h bei Raumtemperatur gerührt und anschließend mit Natriumchlorid-Lösung verdünnt. Man extrahiert mit Essigester, wäscht die organische Phase mit Natriumchlorid-Lösung, trocknet über Natriumsulfat und entfernt das Lösungsmittel. Der Rückstand wird an Silicagel mit Essigester/Hexan chromatographiert, wobei man 183 mg [1*R*-[1α(1*R**),3aβ,4α,7aα]]-1-[5-(Octahydro-4-hydroxy-7a-methyl-1*H*-inden-1-yl]hexyl]-3-[(4-methoxyphenyl)methoxy]-1*H*-pyrazol-4-carbonsäureethylester **150** als farbloses Öl erhält.

$^1$H-NMR ($CD_2Cl_2$): δ= 0,88 ppm (d, 3H); 0,91 (s, 3H); 1,28 (t, 3H); 3,78 (s, 3H); 3,90 (t, 2H); 4,03 (m, 1H); 4,22 (q, 2H); 5,18 (s, 2H); 6,88 (d, 2H); 7,38 (d, 2H); 7,67 (s, 1H)

144. Man behandelt 180 mg des Alkohols **150** analog 11. und erhält 110 mg [1*R*-[1α(1*R**),3aβ,7aα]]-1-[5-(Octahydro-7a-methyl-4-oxo-1*H*-inden-1-yl]hexyl]-3-[(4-methoxyphenyl)-methoxy]-1*H*-pyrazol-4-carbonsäureethylester **151** als farbloses Öl.

$^1$H-NMR ($CD_2Cl_2$): δ= 0,62 ppm (s, 3H); 0,97 (d, 3H); 1,28 (t, 3H); 3,80 (s, 3H); 3,91 (t, 2H); 4,18 (q, 2H); 5,20 (s, 2H); 6,88 (d, 2H); 7,39 (d, 2H); 7,69 (s, 1H)

145. Man setzt 137 mg des Phosphinoxides **13**, welches mit 0,12 ml *n*-Butyllithium-Lösung (2,5 M in Hexan) deprotoniert wurde, mit 60 mg des Ketons **151** analog 12. um und erhält 76 mg (7*E*)-(1*R*,3*R*)-1-[1,3-Bis[[(1,1-dimethylethyl)dimethylsilyl]oxy]-24ahomo-19-nor-9,10-secochola-5,7-dien-24a-yl]-3-[(4-methoxyphenyl)methoxy]-1*H*-pyrazol-4-carbonsäureester **152** als farblosen Schaum.

$^1$H-NMR ($CD_2Cl_2$): δ= 0,04 ppm (s, 12H); 0,50 (s, 3H); 0,85 (s, 18H); 0,91 (d, 3H); 1,28 (t, 3H); 3,77 (s, 3H); 3,88 (t, 2H); 4,04 (m, 2H); 4,18 (q, 2H); 5,17 (s, 2H); 5,79 (d, 1H); 6,14 (d, 1H); 6,88 (d, 2H); 7,38 (d, 2H); 7,65 (s, 1H)

146. Man setzt 20 mg des Silylethers 152 analog 14. um und erhält nach chromatographischer Reinigung mit Essigester/Hexan an Silicagel 8 mg der Titelverbindung **153** als farblosen Schaum.

$^1$H-NMR ($CD_2Cl_2$): δ= 0,50 ppm (s, 3H); 0,88 (d, 3H); 1,24 (t, 3H); 3,73 (s, 3H); 3,88 (t, 2H); 3,98 (m, 1H); 4,02 (m, 1H); 4,18 (q, 2H); 5,15 (s, 2H); 5,80 (d, 1H); 6,23 (d, 1H); 6,86 (d, 2H); 7,358 (d, 2H); 7,64 (s, 1H)

**Beispiel 34**

**(7*E*)-(1*R*,3*R*)-1-(1,3-Dihydroxy-24a-homo-19-nor-9,10-secochola-5,7-dien-24a-yl)-3-hydroxy-1*H*-pyrazol-4-carbonsäureethylester 157**

147. Man legt 110 mg des Ketons **151** in 5 ml Ethanol vor, setzt 40 mg Palladium/Kohle zu und hydriert bis kein Wasserstoff mehr aufgenommen wird. Der Katalysator wird abfiltriert, anschließend entfernt man die organische Phase und chromatographiert den Rückstand mit Essigester/Hexan an Silicagel, wobei 90 mg [1*R*-[1α(1*R**),3aβ,7aα]]-1-[5-(Octahydro-7a-methyl-4-oxo-1*H*-inden-1-yl]hexyl]-3-hydroxy-1*H*-pyrazol-4-carbonsäureethylester **154** als farbloses Öl anfallen.

$^1$H-NMR ($CD_2Cl_2$): δ= 0,58 ppm (s, 3H); 0,90 (d, 3H); 1,30 (t, 3H); 3,90 (t, 2H); 4,28 (q, 2H); 7,57 (s, 1H)

148. Man legt 90 mg des Alkohols **154** in 3 ml Dimethylformamid vor, gibt 0,06 ml Triethylamin und 0,42 ml *t*-Butyldimethylsilylchlorid (1 M in Hexan) zu und rührt 2 h bei Raumtemperatur. Anschließend wird mit Natriumchlorid-Lösung verdünnt, mit Essigester extrahiert, die organische Phase mit Natriumchlorid-Lösung gewaschen und über Natriumsulfat getrocknet. Das Lösungsmittel wird entfernt und der Rückstand an Silicagel mit Essigester/Hexan chromatographiert, wobei man 111 mg des [1*R*-[1α(*R**),3aβ,7aα]]-1 -[5-(Octahydro-7a-methyl-4-oxo-1*H*-inden- 1 -yl]hexyl]-3-[[(1,1-dimethylethyl)dimethylsilyl]oxy]-1*H*-pyrazol-4-carbonsäure-ethylester **155** als farbloses Öl erhält.
$^1$H-NMR ($CD_2Cl_2$): δ= 0,23 ppm (s, 6H); 0,56 (s, 3H); 0,90 (d, 3H); 0,99 (s, 9H); 1,26 (t, 3H); 3,84 (t, 2H); 4,20 (q, 2H); 7,64 (s, 1H)

149. Man setzt 171 mg des Phosphinoxides **13**, welches mit 0,15 ml n-Butyllithium-Lösung (2,5 M in Hexan) deprotoniert wurde, mit 77 mg des Ketons **155** analog 12. um und erhält 41 mg (7*E*)-(1*R*,3*R*)-1-[1,3-Bis-[[(1,1-dimethylethyl)dimethylsilyl]oxy]-24a-homo-19-nor-9,10-secochola-5,7-dien-24a-yl)-3-[[(1,1-dimethylethyl)dimethylsilyl]oxy]-1*H*-pyrazol-4-carbonsäureethylester **156** als farblosen Schaum.
$^1$H-NMR ($CDCl_3$): δ= 0,07 ppm (s, 12H); 0,29 (s, 6H); 0,53 (s, 3H); 0,91 (s, 18H); 0,92 (d, 3H); 1,02 (s, 9H); 1,32 (t, 3H); 3,90 (t, 2H); 4,09 (m, 2H); 4,27 (q, 2H); 5,82 (d, 1H); 6,18 (d, 1H); 7,65 (s, 1H)

150. Man setzt 41 mg des Silylethers **156** analog 14. um und erhält nach chromatographischer Reinigung mit Essigester/Hexan an Silicagel 19 mg der Titelverbindung **157** als farblosen Schaum.
$^1$H-NMR ($CD_2Cl_2$/$CD_3OD$): δ= 0,50 ppm (s, 3H); 0,83 (d, 3H); 1,27 (t, 3H); 3,83 (t, 2H); 3,92 (m, 1H); 3,99 (m, 1H); 4,23 (q, 2H); 5,82 (d, 1H); 6,21 (d, 1H); 7,57 (s, 1H)

**Beispiel 35**

**(7*E*)-(1*R*,3*R*,24a*R*)-24a-(4-Methylphenyl)-24a-homo-19-nor-9,10-secochola-5,7-dien-1,3,24a-triol 164a** und **(7*E*)-(1*R*,3*R*,24a*S*)-24a-(4-Methylphenyl)-24a-homo-19-nor-9,10-secochola-5,7-dien-1,3,24a-triol 164b**

151. Man legt 0,84 ml 4-Bromtoluol in 18 ml Tetrahydrofuran vor und gibt bei -78°C 2,72 ml *n*-Butyllithium-Lösung (2,5 M in Hexan) zu. Nach 30 min bei dieser Temperatur tropft man 500 mg des Aldehydes **8** in 3 ml Tetrahydrofuran zu und rührt 1 h nach. Anschließend wird mit Natriumchlorid-Lösung gequencht, mit Essigester extrahiert, die organische Phase mit Natriumchlorid-Lösung gewaschen und über Natriumsulfat getrocknet. Das Lösungsmittel wird entfernt und der Rückstand wird an Silicagel mit Essigester/Hexan chromatographiert, wobei man 381 mg [1*R*-[1α(1*R**),3aβ,4α,7aα]]-α-[4-[Octahydro-7a-methyl-4-[(triethylsilyl)oxy]-1*H*-inden-1-yl]pentyl]-4-methylphenylmethanol **158** als farbloses Öl erhält.
$^1$H-NMR ($CDCl_3$): δ= 0,56 ppm (q, 6H); 0,89 (d, 3H); 0,91 (s, 3H); 0,95 (t, 9H); 2,33 (s, 3H); 4,02 (m, 1H); 4,62 (m, 1H); 7,13 (d, 2H); 7,23 (d, 2H)

152. Man behandelt 315 mg des Alkohols **158** analog 9. und erhält 302 mg [1*R*-[1α(1*R**),3aβ,4α,7aα]]-5-[Octahydro-7a-methyl-4-[(triethylsilyl)oxy]-1*H*-inden-1-yl]-1-(4-methylphenyl)hexyl-acetat **159** als farbloses Öl.
$^1$H-NMR ($CDCl_3$): δ= 0,56 ppm (q, 6H); 0,88/0,89 (d, 3H); 0,90 (s, 3H); 0,95 (t, 9H); 2,05 (s, 3H); 2,35 (s, 3H); 4,02 (m, 1H); 5,70 (m, 1H); 7,14 (d, 2H); 7,22 (d, 2H)

153. Man behandelt 300 mg des Acetates **159** analog 10. und erhält 228 mg [1*R*-[1α(1*R**),3aβ,4α,7aα]]-1-[5-(Acetyloxy)-1-methyl-5-(4-methylphenyl)pentyl]octahydro-7a-methyl-1*H*-inden-4-ol **160** als farbloses Öl.
$^1$H-NMR ($CDCl_3$): δ= 0,87/0,88 ppm (d, 3H); 0,90/0,91 (s, 3H); 2,07 (s, 3H); 2,35 (s, 3H); 4,08 (m, 1H); 5,70 (m, 1H); 7,15 (d, 2H); 7,22 (d, 2H)

154. Man behandelt 227 mg des Alkohols **160** analog 11. und erhält 185 mg [1*R*-[1α(1*R**),3aβ,7aα]]-1-[5-(Acetyloxy)-1-methyl-5-(4-methylphenyl)pentyl]octahydro-7a-methyl-4*H*-inden-4-on **161** als farbloses Öl.
$^1$H-NMR ($CDCl_3$): δ=0,62/0,63 ppm (s, 3H); 0,92/0,93 (d, 3H); 2,05 (s, 3H); 2,35 (s, 3H); 5,69 (m, 1H); 7,12 (d, 2H); 7,21 (d, 2H)

155. Man setzt 267 mg des Phosphinoxides **13**, welches mit 0,23 ml *n*-Butyllithium-Lösung (2,5 M in Hexan) deprotoniert wurde, mit 90 mg des Ketons **161** analog 12. um und erhält 163 mg (7*E*)-(1*R*,3*R*)-24a-(Acetyloxy)-1,3-bis[[(1,1-dimethylethyl)dimethylsilyl]oxy]-24a-(4-methylphenyl)-24a-homo-19-nor-9,10-secochola-5,7-dien **162** als farblosen Schaum.
$^1$H-NMR ($CDCl_3$): δ= 0,07 ppm (s, 12H); 0,52/0,53 (s, 3H); 0,90 (s, 18H); 0,91 (d, 3H); 2,07 (s, 3H); 2,35 (s, 3H); 4,08 (m, 2H); 5,70 (m, 1H); 5,80 (d, 1H); 6,18 (d, 1H); 7,15 (d, 2H); 7,22 (d, 2H)

156. Man setzt 162 mg des Acetates **162** analog 13. um und erhält 147 mg (7*E*)-(1*R*,3*R*)-1,3-Bis[[(1,1-dimethylethyl)dimethylsilyl]oxy]-24a-(4-methylphenyl)-24a-homo-19-nor-9,10-secochola-5,7-dien-24a-ol **163** als farblosen Schaum.
$^1$H-NMR ($CDCl_3$): δ= 0,04 ppm (s, 12H); 0,52/0,53 (s, 3H); 0,88 (s, 18H); 0,89 (d, 3H); 2,25 (s, 3H); 4,08 (m, 2H); 4,63 (m, 1H); 5,82 (d, 1H); 6,18 (d, 1H); 7,17 (d, 2H); 7,25 (d, 2H)

157. Man behandelt 115 mg des Alkohols **163** analog 14. und erhält nach Trennung der Diastereomeren (bzgl. C-24a) über HPLC 27 mg der Titelverbindung **164a** und 30 mg der Titelverbindung 164b als farblose Schäume.
$^1$H-NMR ($CD_2Cl_2$/$CD_3OD$): **164a**: δ= 0,48 ppm (s, 3H); 0,86 (d, 3H); 2,28 (s, 3H); 3,91 (m, 1H); 3,98 (m, 1H); 4,51 (t, 1H); 5,80 (d, 1H); 6,21 (d, 1H); 7,10 (d, 2H); 7,17 (d, 2H)
**164b:** δ= 0,49 ppm (s, 3H); 0,86 (d, 3H); 2,28 (s, 3H); 3,91 (m, 1H); 3,98 (m, 1H); 4,52 (t, 1H); 5,80 (d, 1H); 6,22 (d, 1H); 7,10 (d, 2H); 7,17 (d, 2H)

**Beispiel 36**

**(7*E*)-(1*R*,3*R*)-1,3-Dihydroxy-24a-(4-methylphenyl)-24a-homo-19-nor-9,10-secochola-5,7-dien-24a-on 166**

158. Man behandelt 31 mg des Alkohols **163** analog 25., wobei 19 mg (7*E*)-(1*R*,3*R*)-1,3-Bis[[(1,1-dimethylethyl)dimethylsilyl]oxy]-24a-(4-methylphenyl)-24a-homo-19-nor-9,10-secochola-5,7-dien-24a-on **165** als farbloser Schaum anfallen.
$^1$H-NMR ($CD_2Cl_2$): δ= 0,04 ppm (s, 12H); 0,52 (s, 3H); 0,86 (s, 18H); 0,93 (d, 3H); 2,38 (s, 3H); 4,06 (m, 2H); 5,80 (d, 1H); 6,15 (d, 1H); 7,22 (d, 2H); 7,80 (d, 2H)
159. Man behandelt 18 mg des Ketons **165** analog 26., wobei 9 mg der Titelverbindung **166** als farbloser Schaum anfallen.
$^1$H-NMR ($CD_2Cl_2$): δ= 0,53 ppm (s, 3H); 0,95 (d, 3H); 2,38 (s, 3H); 3,96 (m, 1H); 4,03 (m, 1H); 5,84 (d, 1H); 6,26 (d, 1H); 7,26 (d, 2H); 7,84 (d, 2H)

**Beispiel 37**

**(7*E*)-(1*R*,2*R*,3*R*,24a*R*)-24a-(4-Methylphenyl)-24a-homo-19-nor-9,10-secochola-5,7-dien-1,2,3,24a-tetrol 169a** und **(7*E*)-(1*R*,2*S*,3*R*,24a*S*)-24a-(4-Methylphenyl)-24ahomo-19-nor-9,10-secochola-5,7-dien-1,2,3,24a-tetrol 169b**

160. Man setzt 327 mg [2-[[3*S*-(3*R*,4*S*,5*R*)]-3,4,5-Tris[[(1,1-dimethylethyl)dimethylsilyl]oxy]cyclo-hexyliden]ethyl]diphenylphosphinoxid **61b** [H.F. DeLuca et al. *J. Med. Chem.* **37**, 3730 (1994)], welches mit 0,22 ml *n*-Butyllithium-Lösung deprotoniert wurde, mit 90 mg des Ketons **161** analog 58. um und erhält 118 mg (7*E*)-(1*R*,2*R*,3*R*)-24a-(Acetyloxy)-1,2,3-tris[[(1,1-dimethylethyl)dimethylsilyl]oxy]-24a-(4-methylphenyl)-24a-homo-19-nor-9,10-secochola-5,7-dien **167** als farblosen Schaum.
$^1$H-NMR ($CDCl_3$): δ= 0,03-0,08 ppm (4 x s, 18H); 0,52 (s, 3H); 0,86 (s, 9H); 0,90 (s, 18H); 0,91 (d, 3H); 2,07 (s, 3H); 2,35 (s, 3H); 3,65 (m, 1H); 3,85 (m, 1H); 3,96 (m, 1H); 5,69 (m, 1H); 5,80 (d, 1H); 6,18 (d, 1H); 7,15 (d, 2H); 7,22 (d, 2H)
161. Man setzt 117 mg (7*E*)-(1*R*,2*R*,3*R*)-1,2,3-Tris[[(1,1-dimethylethyl)dimethylsilyl]oxy]-24a-(4-methylphenyl)-24a-homo-19-nor-9,10-secochola-5,7-dien-24a-ol **167** analog 13. um und erhält 109 mg **168** als farblosen Schaum.
$^1$H-NMR ($CDCl_3$): δ= 0,03-0,08 ppm (4 x s, 18H); 0,52 (s, 3H); 0,85 (s, 9H); 0,90 (s, 18H); 0,91 (d, 3H); 2,35 (s, 3H); 3,65 (m, 1H); 3,84 (m, 1H); 3,95 (m, 1H); 4,63 (m, 1H); 5,80 (d, 1H); 6,17 (d, 1H); 7,18 (d, 2H); 7,24 (d, 2H)
162. Man behandelt 108 mg des Alkohols **168** analog 14. und erhält nach Trennung der Diastereomeren (bzgl. C-24a) über HPLC 12 mg der Titelverbindung **169a** und 16 mg der Titelverbindung **169b** als farblose Schäume.
$^1$H-NMR ($CD_2Cl_2$): **169a:** δ= 0,51 ppm (s, 3H); 0,89 (d, 3H); 2,29 (s, 3H); 3,45 (m, 1H); 3,60 (m, 1H); 4,00 (m, 1H); 4,59 (m, 1H); 5,81 (d, 1H); 6,22 (d, 1H); 7,11 (d, 2H); 7,20 (d, 2H)
**169b:** δ= 0,50 ppm (s, 3H); 0,88 (d, 3H); 2,29 (s, 3H); 3,45 (m, 1H); 3,60 (m, 1H); 4,00 (m, 1H); 4,60 (t, 1H); 5,81 (d, 1H); 6,22 (d, 1H); 7,11 (d, 2H); 7,20 (d, 2H)

**Beispiel 38**

**(7*E*)-(1*R*,3*R*,24a*R*)-24a-(4-Trifluormethylphenyl)-19-nor-9,10-secochola-5,7-dien-1,3,24a-triol 176a** und **(7*E*)-(1*R*,3*R*,24a*S*)-24a-(4-Trifluormethylphenyl)-19-nor-9,10-secochola-5,7-dien-1,3,24a-triol 176b**

163. Man legt 1,39 ml 4-Trifluormethylbrombenzol in 20 ml Tetrahydrofuran vor und gibt bei -78°C 3,96 ml *n*-Butyllithium-Lösung (2,5 M in Hexan) zu. Nach 30 min bei dieser Temperatur tropft man 762 mg des Aldehydes **8** in 5 ml Tetrahydrofuran zu und rührt 1 h nach. Anschließend wird mit Natriumchlorid-Lösung gequencht, mit Essigester extrahiert, die organische Phase mit Natriumchlorid-Lösung gewaschen und über Natriumsulfat getrocknet. Das Lösungsmittel wird entfernt und der Rückstand wird an Silicagel mit Essigester/Hexan chromatographiert, wobei man 587 mg [1*R*-[1α(1*R**),3aβ,4α,7aα]]-α-[4-[Octahydro-7a-methyl-4-[(triethylsilyl)oxy]-1*H*-inden-1-yl]pentyl]-4-(trifluormethylphenyl)methanol **170** als farbloses Öl erhält.
$^1$H-NMR ($CDCl_3$): δ= 0,56 ppm (q, 6H); 0,89 (d, 3H); 0,91 (s, 3H); 0,95 (t, 9H); 2,33 (s, 3H); 4,02 (m, 1H); 4,75 (m, 1H); 7,48 (d, 2H); 7,61 (d, 2H)
164. Man behandelt 580 mg des Alkohols **170** analog 9. und erhält 566 mg [1*R*-[1α(1*R**),3aβ,4α,7aα]]-5-[Octahydro-7a-methyl-4-[(triethylsilyl)oxy]-1*H*-inden-1-yl]-1-(4-trifluormethylphenyl)hexyl-acetat **171** als farbloses Öl.
$^1$H-NMR ($CDCl_3$): δ= 0,56 ppm (q, 6H); 0,88/0,89 (d, 3H); 0,90/0,91 (s, 3H); 0,95 (t, 9H); 2,10 (s, 3H); 4,02 (m, 1H); 5,74 (m, 1H); 7,42 (d, 2H); 7,61 (d, 2H)
165. Man behandelt 556 mg des Acetates **171** analog 10. und erhält 208 mg [1*R*-[1α(*R**),3aβ,4α,7aα]]-1-[5-(Acetyloxy)-1-methyl-5-(4-trifluormethylphenyl)pentyl]octahydro-7a-methyl-1*H*-inden-4-ol **172** als farbloses Öl.

$^1$H-NMR ($CDCl_3$): δ= 0,88/0,89 ppm (d, 3H); 0,91/0,92 (s, 3H); 2,11 (s, 3H); 4,08 (m, 1H); 5,75 (m, 1H); 7,43 (d, 2H); 7,61 (d, 2H)

166. Man behandelt 160 mg des Alkohols **172** analog 11. und erhält 138 mg [1*R*-[1α(1*R**),3aβ,7aα]]-1-[5-(Acetyloxy)-1-methyl-5-(4-trifluormethylphenyl)pentyl]octahydro-7a-methyl-4*H*-inden-4-on **173** als farbloses Öl.
$^1$H-NMR ($CDCl_3$): δ= 0,61/0,62 ppm (s, 3H); 0,92/0,93 (d, 3H); 2,09 (s, 3H); 5,75 (m, 1H); 7,45 (d, 2H); 7,61 (d, 2H)

167. Man setzt 348 mg des Phosphinoxides **13**, welches mit 0,29 ml *n*-Butyllithium-Lösung (2,5 M in Hexan) deprotoniert wurde, mit 134 mg des Ketons 173 analog 12. um und erhält 173 mg (7*E*)-(1*R*,3*R*)-24a-(Acetyloxy)-1,3-bis[[(1,1-dimethylethyl)dimethylsilyl]oxy]-24a-(4-trifluormethyl-phenyl)-24a-homo-19-nor-9,10-secochola-5,7-dien **174** als farblosen Schaum.
$^1$H-NMR ($CDCl_3$): δ= 0,07 ppm (s, 12H); 0,52/0,53 (s, 3H); 0,90 (s, 18H); 0,91 (d, 3H); 2,10 (s, 3H); 4,08 (m, 2H); 5,73 (m, 1H); 5,80 (d, 1H); 6,18 (d, 1H); 7,44 (d, 2H); 7,61 (d, 2H)

168. Man setzt 173 mg des Acetates **174** analog 13. um und erhält 147 mg (7*E*)-(1*R*,3*R*)-1,3-Bis[[(1,1 -dimethylethyl)dimethylsilyl]oxy]-24a-(4-trifluormethylphenyl)-24a-homo-19-nor-9,10-secochola-5,7-dien-24a-ol **175** als farblosen Schaum.
$^1$H-NMR ($CDCl_3$): δ= 0,05 ppm (s, 12H); 0,52/0,53 (s, 3H); 0,89 (s, 18H); 0,90 (d, 3H); 4,09 (m, 2H); 4,76 (m, 1H); 5,81 (d, 1H); 6,18 (d, 1H); 7,48 (d, 2H); 7,61 (d, 2H)

169. Man behandelt 113 mg des Alkohols **175** analog 14. und erhält nach Trennung der Diastereomeren (bzgl. C-24a) über HPLC 21 mg der Titelverbindung **176a** und 19 mg der Titelverbindung **176b** als farblose Schäume.
$^1$H-NMR ($CD_2Cl_2$): **176a:** δ= 0,51 ppm (s, 3H); 0,86 (d, 3H); 3,95 (m, 1H); 4,02 (m, 1H); 4,73 (t, 1H); 5,82 (d, 1H); 6,25 (d, 1H); 7,45 (d, 2H); 7,60 (d, 2H)
**176b:** δ= 0,51 ppm (s, 3H); 0,86 (d, 3H); 3,95 (m, 1H); 4,02 (m, 1H); 4,74 (t, 1H); 5,82 (d, 1H); 6,25 (d, 1H); 7,45 (d, 2H); 7,60 (d, 2H)

**Beispiel 39**

**(7*E*)-(1*R*,3*R*)-1,3-Dihydroxy-24a-(4-trifluormethylphenyl)-24a-homo-19-nor-9,10-secochola-5,7-dien-24a-on 178**

170. Man behandelt 33 mg des Alkohols **175** analog 25., wobei 18 mg (7*E*)-(1*R*,3*R*)-1,3-Bis[[(1,1-dimethylethyl)dimethylsilyl]oxy]-24a-(4-trifluormethylphenyl)-24a-homo-19-nor-9,10-secochola-5,7-dien-24a-on **177** als farbloser Schaum anfallen.
$^1$H-NMR ($CDCl_3$): δ= 0,07 ppm (s, 12H); 0,54 (s, 3H); 0,89 (s, 18H); 0,98 (d, 3H); 4,08 (m, 2H); 5,82 (d, 1H); 6,18 (d, 1H); 7,73 (d, 2H); 8,08 (d, 2H)

171. Man behandelt 18 mg des Ketons **177** analog 26., wobei 9 mg der Titelverbindung **178** als farbloser Schaum anfallen.
$^1$H-NMR ($CD_2Cl_2$): δ= 0,54 ppm (s, 3H); 0,95 (d, 3H); 3,96 (m, 1H); 4,03 (m, 1H); 5,85 (d, 1H); 6,26 (d, 1H); 7,73 (d, 2H); 8,05 (d, 2H)

**Beispiel 40**

**(7*E*)-(1*R*,3*R*,24a*R*)-24a-(4-Methoxyphenyl)-24a-homo-19-nor-9,10-secochola-5,7-dien-1,3,24a-triol 185a** und **(7*E*)-(1*R*,3*R*,24a*S*)-24a-(4-Methoxyphenyl)-24a-homo-19-nor-9,10-secochola-5,7-dien-1,3,24a-triol 185b**

172. Man legt 0,85 ml 4-Bromanisol in 18 ml Tetrahydrofuran vor und gibt bei -78°C 2,72 ml *n*-Butyllithium-Lösung (2,5 M in Hexan) zu. Nach 30 min bei dieser Temperatur tropft man 500 mg des Aldehydes **8** in 4 ml Tetrahydrofuran zu und rührt 1 h nach. Anschließend wird mit Natriumchlorid-Lösung gequencht, mit Essigester extrahiert, die organische Phase mit Natriumchlorid-Lösung gewaschen und über Natriumsulfat getrocknet. Das Lösungsmittel wird entfernt und der Rückstand wird an Silicagel mit Essigester/Hexan chromatographiert, wobei man 497 mg [1*R*-[1α(1*R**),3aß,4α,7aα]]-α-[4-[Octahydro-7a-methyl-4-[(triethylsilyl)oxy]-1*H*-inden-1-yl]pentyl]-4-(methoxyphenyl)methanol **179** als farbloses Öl erhält.
$^1$H-NMR ($CDCl_3$): δ= 0,54 ppm (q, 6H); 0,89 (d, 3H); 0,90 (s, 3H); 0,94 (t, 9H); 3,80 (s, 3H); 4,01 (m, 1H); 4,61 (m, 1H); 6,88 (d, 2H); 7,28 (d, 2H)

173. Man behandelt 490 mg des Alkohols **179** analog 9. und erhält 509 mg [1*R*-[1α(1*R**),3aβ,4α,7aα]]-5-[Octahydro-7a-methyl-4-[(triethylsilyl)oxy]-1*H*-inden-1-yl]-1-(4-methoxyphenyl)hexyl-acetat **180** als farbloses Öl.
$^1$H-NMR ($CDCl_3$): δ= 0,56 ppm (q, 6H); 0,88/0,89 (d, 3H); 0,90/0,91 (s, 3H); 0,95 (t, 9H); 2,06 (s, 3H); 3,80 (s, 3H); 4,02 (m, 1H); 5,69 (m, 1H); 6,88 (d, 2H); 7,28 (d, 2H)

174. Man behandelt 504 mg des Acetates **180** analog 10. und erhält 280 mg [1*R*-[1a(1*R**),3aβ,4α,7aα]]-1-[5-(Acetyloxy)-1-methyl-5-(4-methoxyphenyl)pentyl]octahydro-7a-methyl-1*H*-inden-4-ol **181** als farbloses Öl.

$^1$H-NMR ($CDCl_3$): δ= 0,87/0,88 ppm (d, 3H); 0,91/0,92 (s, 3H); 2,07 (s, 3H); 3,80 (s, 3H); 4,08 (m, 1H); 5,68 (m, 1H); 6,88 (d, 2H); 7,28 (d, 2H)

175. Man behandelt 275 mg des Alkohols **181** analog 11. und erhält 252 mg [1*R*-[1α(1*R**),3aβ,7aα]]-1-[5-(Acetyloxy)-1-methyl-5-(4-methoxyphenyl)pentyl]octahydro-7a-methyl-4*H*-inden-4-on **182** als farbloses Öl.
$^1$H-NMR ($CDCl_3$): δ= 0,61/0,62 ppm (s, 3H); 0,91/0,92 (d, 3H); 2,07 (s, 3H); 3,80 (s, 3H); 5,68 (m, 1H); 6,88 (d, 2H); 7,28 (d, 2H)

176. Man setzt 370 mg des Phosphinoxides 13, welches mit 0,30 ml *n*-Butyllithium-Lösung (2,5 M in Hexan) deprotoniert wurde, mit 130 mg des Ketons **182** analog 12. um und erhält 206 mg (7*E*)-(1*R*,3*R*)-24a-(Acetyloxy)-1,3-bis[[(1,1-dimethylethyl)dimethylsilyl]oxy]-24a-(4-methoxyphenyl)-24a-homo-19-nor-9,10-secochola-5,7-dien **183** als farblosen Schaum.
$^1$H-NMR ($CDCl_3$): δ= 0,07 ppm (s, 12H); 0,52/0,53 (s, 3H); 0,90 (s, 18H); 0,91 (d, 3H); 2,03 (s, 3H); 3,81 (s, 3H); 4,08 (m, 2H); 5,69 (m, 1H); 5,81 (d, 1H); 6,18 (d, 1H); 6,88 (d, 2H); 7,28 (d, 2H)

177. Man setzt 200 mg des Acetates **183** analog 13. um und erhält 175 mg (7*E*)-(1*R*,3*R*)-1,3-Bis[[(1,1-dimethylethyl)dimethylsilyl]oxy]-24a-(4-methoxyphenyl)-24a-homo-19-nor-9,10-secochola-5,7-dien-24a-ol **184** als farblosen Schaum.
$^1$H-NMR ($CDCl_3$): δ= 0,07 ppm (s, 12H); 0,52/0,53 (s, 3H); 0,89 (s, 18H); 0,90 (d, 3H); 3,80 (s, 3H); 4,09 (m, 2H); 4,63 (m, 1H); 5,81 (d, 1H); 6,18 (d, 1H); 6,90 (d, 2H); 7,30 (d, 2H)

178. Man behandelt 130 mg des Alkohols **184** analog 14. und erhält nach Trennung der Diastereomeren (bzgl. C-24a) über HPLC 26 mg der Titelverbindung **185a** und 22 mg der Titelverbindung **185b** als farblose Schäume.
$^1$H-NMR ($CD_2Cl_2$): **185a:** δ= 0,52 ppm (s, 3H); 0,89 (d, 3H); 3,80 (s, 3H); 3,97 (m, 1H); 4,04 (m, 1H); 4,59 (t, 1H); 5,82 (d, 1H); 6,27 (d, 1H); 6,88 (d, 2H); 7,25 (d, 2H)
**185b**: δ= 0,52 ppm (s, 3H); 0,89 (d, 3H); 3,79 (s, 3H); 3,97 (m, 1H); 4,04 (m, 1H); 4,60 (t, 1H); 5,82 (d, 1H); 6,27 (d, 1H); 6,88 (d, 2H); 7,25 (d, 2H)

## Beispiel 41

**(7*E*)-(1*R*,3*R*)-1,3-Dihydroxy-24a-(4-methoxyphenyl)-24a-homo-19-nor-9,10- secochola-5,7-dien-24a-on 187**

179. Man behandelt 44 mg des Alkohols **184** analog 25., wobei 27 mg (7*E*)-(1*R*,3*R*)-1,3-Bis[[(1,1-dimethylethyl)dimethylsilyl]oxy]-24a-(4-methoxyphenyl)-24a-homo-19-nor-9,10-secochola-5,7-dien-24a-on **186** als farbloser Schaum anfallen.
$^1$H-NMR ($CD_2Cl_2$): δ= 0,04 ppm (s, 12H); 0,51 (s, 3H); 0,84 (s, 18H); 0,94 (d, 3H); 3,82 (s, 3H); 4,08 (m, 2H); 5,81 (d, 1H); 6,15 (d, 1H); 6,91 (d, 2H); 7,90 (d, 2H)

180. Man behandelt 27 mg des Ketons **186** analog 26., wobei 16 mg der Titelverbindung **187** als farbloser Schaum anfallen.
$^1$H-NMR ($CD_2Cl_2$): δ= 0,51 ppm (s, 3H); 0,92 (d, 3H); 3,80 (s, 3H); 3,96 (m, 1H); 4,02 (m, 1H); 5,83 (d, 1H); 6,24 (d, 1H); 6,92 (d, 2H); 7,91 (d, 2H)

## Beispiel 42

**(7*E*)-(1*R*,3*R*,20*S*,24a*R*)-24a-(Thiazol-2-yl)-24a-homo-19-nor-9,10-secochola-5,7-dien-1,3,24a-trio 202a** und **(7*E*) -(1*R*,3*R*,20*S*,24a*S*)-24a-(Thiazol-2-yl)-24-a-homo-19-nor-9,10-secochola-5,7-dien-1,3,24a-triol 202b**

181. Man legt 3,89 g des Alkohols **3** in 250 ml Dichlormethan vor und gibt 7,74 g Pyridiniumchlorochromat hinzu. Es wird 3 h bei Raumtemperatur gerührt und anschließend mit Diethylether verdünnt, über Celite abgesaugt und eingeengt. Der Rückstand wird an Silicagel mit Esigester/Hexan chromatographiert, wobei man 3,19 g [1*R*-[1α (1*S**),3aβ,4α,7aα]]-2-[Octahydro-7a-methyl-4-[(triethylsilyl)oxy]-1*H*-inden-1-yl]propanal **188** als farbloses Öl erhält.
$^1$H-NMR ($CDCl_3$): δ= 0,56 ppm (q, 6H); 0,93 (s, 3H); 0,94 (t, 9H); 1,10 (t, 9H); 4,07 (m, 1H); 9,58 (s, 1H)

182. Man legt 6,23 g des Aldehydes **188** in 60 ml Toluol und 60 ml Methanol vor, gibt 4 ml Diazabicycloundecan hinzu und rührt 4 d bei Raumtemperatur. Das Rohprodukt wird eingeengt und an Silicagel mit Essigester/Hexan chromatographiert, wobei man 5,63 g des Diastereomerengemisches [1*R*-[1α(1*S**),3aβ,4α,7aα]]-2-[Octahydro-7a-methyl-4-[(triethylsilyl)-oxy]-1*H*-inden-1-yl]propanal **188** und [1*R*-[1α(1*R**),3aβ,4α,7aα]]-2-[Octahydro-7a-methyl-4-[(triethylsilyl)oxy]-1*H*-inden-1-yl]propanal **189** als farbloses Öl erhält.
$^1$H-NMR ($CDCl_3$): **189**: δ= 0,56 ppm (q, 6H); 0,93 (d, 3H); 0,94 (t, 9H); 1,02 (d, 3H); 4,07 (m, 1H) 9,53 (d, 1H)

183. Man löst 5,63 g des Epimerengemisches der Aldehyde **188/189** in 100 ml Ethanol und 10 ml Tetrahydrofuran und gibt portionsweise 377 mg Natriumborhydrid zu. Es wird 1 h bei Raumtemperatur gerührt und anschließend vorsichtig mit Ammoniumchlorid-Lösung gequencht. Man extrahiert mit Dichlormethan, wäscht die organische Phase mit Natriumchlorid-Lösung, trocknet über Natriumsulfat und engt ein. Der Rückstand wird an Silicagel mit Esigester/Hexan chromatographiert, wobei man 2,61 g [1*R*-[1α(1*R**),3aβ,4α,7aα]]-2-[Octahydro-7a-methyl-4-[(triethylsilyl)oxy]-1*H*-inden-1-yl]-1-propanol **190** neben 2,10 g der an 20 normalkonfigurierten Verbindung **3** als farblose Öle erhält.

$^1$H-NMR ($CDCl_3$): δ= 0,55 ppm (q, 6H); 0,92 (d, 3H); 0,94 (t, 9H); 0,98 (d, 3H); 3,46 (dd, 1H); 3,72 (dd, 1H); 4,03 (m, 1H)

184. Man legt 2,61 g des Alkohols **190** in 80 ml Pyridin vor, kühlt auf 0°C und fügt 3,04 g p-Toluolsulfonylchlorid hinzu. Man rührt 5 h bei dieser Temperatur nach und gießt dann das Reaktionsgemisch auf Natriumhydrogencarbonat-Lösung. Es wird mit Essigester extrahiert, die organische Phase mit Natriumchlorid-Lösung gewaschen und über Natriumsulfat getrocknet. Der Rückstand wird an Silicagel mit Esigester/Hexan chromatographiert, wobei man 3,19 g [1*R*-[1α(1*R**),3aβ,4α,7aα]]-2-[Octahydro-7a-methyl-4-[(triethylsilyl)oxy]-1*H*-inden-1-yl]propyl-(4-methylbenzensulfonat) **191** als farbloses Öl erhält.

$^1$H-NMR ($CDCl_3$): δ= 0,54 ppm (q, 6H); 0,81 (s, 3H); 0,88 (d, 3H); 0,94 (t, 9H); 2,47 (s, 3H); 3,78 (dd, 1H); 4,01 (m, 1H); 4,12 (dd, 1H); 7,34 (d, 1H); 7,79 (d, 1H)

185. Man legt 4,5 ml Propargyl-THP-Ether in 100 ml Dioxan vor und tropft 12,8 ml *n*-Butyllithium-Lösung (2,5 M in Hexan) hinzu. Nach 1 h werden 3,18 g des Tosylates **191** in 20 ml Dioxan zugetropft. Für 2 d wird nun zum Sieden erhitzt. Nach dem Abkühlen quencht man mit Natriumhydrogencarbonat-Lösung, extrahiert mit Essigester, wäscht die organische Phase mit Natriumchlorid-Lösung und engt ein. Der Rückstand wird an Silicagel mit Esigester/Hexan chromatographiert, wobei man 2,62 g [1*R*-[1α(1*S**),3aβ,4α,7aα]]-Octahydro-7a-methyl-1-[5-[(tetrahydro-2*H*-pyran-2-yl)oxy]-1-methyl-3-pentinyl]-4-[(triethylsilyl)oxy]-1*H*-inden **192** als farbloses Öl erhält.

$^1$H-NMR ($CDCl_3$): δ= 0,55 ppm (q, 6H); 0,89 (s, 3H); 0,92 (d, 3H); 0,94 (t, 9H); 4,02 (m, 1H); 4,28 (m, 2H); 4,94 (m, 1H)

186. Man legt 2,62 g des Alkins **192** in 50 ml Essigester vor, fügt 465 mg Palladium/Kohle (10%) und 1,06 g Natriumhydrogencarbonat zu und hydriert bei Normaldruck. Das Reaktionsgemisch wird anschließend über Celite filtriert und eingeengt. Das Rohprodukt (2,08 g) [1*R*-[1α(1*S**),3aβ,4α,7aα]]-Octahydro-7a-methyl-1-[5-[(tetrahydro-2*H*-pyran-2-yl)oxy]-1 -methyl-pentyl]-4-[(triethylsilyl)oxy]-1*H*-inden **193** wird direkt weiter umgesetzt.

187. Man legt 2,08 g des THP-Ethers **193** in 100 ml Dichlormethan vor und tropft 9,2 ml Diethylaluminiumchlorid-Lösung (1,8 M in Toluol) zu. Nach 2 h bei Raumtemperatur quencht man mit Isopropanol/Wasser (15:85), gibt Toluol hinzu und rührt über Nacht nach. Anschließend saugt man über Celite ab und engt ein. Der Rückstand wird an Silicagel mit Essigester/Hexan chromatographiert, wobei man 1,02 g [1*R*-[1α(1*S**),3aβ,4α,7aα]]-5-[Octahydro-7a-methyl-4-[(triethylsilyl)oxy]-1*H*-inden-1-yl]-1-hexanol **194** als farbloses Öl erhält.

$^1$H-NMR ($CDCl_3$): δ= 0,56 ppm (q, 6H); 0,82 (d, 3H); 0,92 (s, 3H); 0,96 (t, 9H); 3,64 (t, 2H); 4,02 (m, 1H)

188. Es werden 1,02 g des Alkohols **194** in 40 ml Dichlormethan gelöst und anschließend werden 835 mg Pyridiniumchlorochromat zugegeben. Man rührt 2 h bei Raumtemperatur, setzt danach Diethylether zu, filtriert über Celite und engt ein. Der Rückstand wird an Silicagel mit Essigester/Hexan chromatographiert, wobei man 810 mg [1*R*-[1α(1*S**),3aβ,4α,7aα]]-5-[Octahydro-7a-methyl-4-[(triethylsilyl)oxy]-1*H*-inden-1-yl]hexanal **195** als farbloses Öl erhält.

$^1$H-NMR ($CDCl_3$): δ= 0,56 ppm (q, 6H); 0,83 (d, 3H); 0,91 (s, 3H); 0,96 (t, 9H); 2,40 (t, 2H); 4,02 (m, 1H); 9,78 (sbr, 1H)

189. Man legt 0,99 ml 2-Bromthiazol in 20 ml Tetrahydrofuran vor und gibt bei -78°C 4,42 ml *n*-Butyllithium-Lösung (2,5 M in Hexan) zu. Nach 30 min bei dieser Temperatur tropft man 810 mg des Aldehydes **195** in 5 ml Tetrahydrofuran zu und rührt 1 h nach. Anschließend wird mit Natriumchlorid-Lösung gequencht, mit Essigester extrahiert, die organische Phase mit Natriumchlorid-Lösung gewaschen und über Natriumsulfat getrocknet. Das Lösungsmittel wird entfernt und der Rückstand wird an Silicagel mit Essigester/Hexan chromatographiert, wobei man 878 mg [1*R*-[1α(1*S**),3aβ,4α,7aα,]]-α-[4-[Octahydro-7a-methyl-4-[(triethylsilyl)oxy]-1*H*-inden-1-yl]pentyl]thiazol-2-methanol **196** als farbloses Öl erhält.

$^1$H-NMR ($CDCl_3$): δ= 0,57 ppm (q, 6H); 0,80 (d, 3H); 0,90 (s, 3H); 0,97 (t, 9H); 4,02 (m, 1H); 5,01 (m, 1H); 7,30 (d, 1H); 7,72 (d, 1H)

190. Man behandelt 878 mg des Alkohols **196** analog 9. und erhält 709 mg [1*R*-[1α(1*S**),3aβ,4α,7aα]]-5-[Octahydro-7a-methyl-4-[(triethylsilyl)oxy]-1*H*-inden-1-yl]-1-(thiazol-2-yl)hexyl-acetat **197** als farbloses Öl.

$^1$H-NMR ($CDCl_3$): δ= 0,56 ppm (q, 6H); 0,78/0,79 (d, 3H); 0,89 (s, 3H); 0,96 (t, 9H); 2,14 (s, 3H); 4,02 (m, 1H); 6,10 (t, 1H); 7,30 (d, 1H); 7,78 (d, 1H)

191. Man behandelt 704 mg des Acetates **197** analog 10. und erhält 490 mg [1*R*-[1α(1*S**),3aβ,4α,7aα]]-1-[5-(Acetyloxy)-1-methyl-5-(thiazol-2-yl)pentyl]octahydro-7amethyl-1*H*-inden-4-ol **198** als farbloses Öl.

$^1$H-NMR ($CDCl_3$): δ= 0,78/0,79 ppm (d, 3H); 0,92 (s, 3H); 2,16 (s, 3H); 4,07 (m, 1H); 6,10 (t, 1H); 7,30 (d, 1H); 7,78 (d, 1H)

192. Man behandelt 485 mg des Alkohols **198** analog 11. und erhält 480 mg [1*R*-[1α(1*S**),3aβ,7aα]]-1-[5-(Acetyloxy)-1-methyl-5-(thiazol-2-yl)pentyl]octahydro-7amethyl-4*H*-inden-4-on **199** als farbloses Öl.

$^1$H-NMR ($CDCl_3$): δ= 0,60 ppm (s, 3H); 0,81/0,82 (d, 3H); 2,13 (s, 3H); 6,10 (t, 1H); 7,31 (d, 1H); 7,78 (d, 1H)

193. Man setzt 665 mg des Phosphinoxides **13**, welches mit 0,56 ml *n*-Butyllithium-Lösung (2,5 M in Hexan) deprotoniert wurde, mit 220 mg des Ketons **199** analog 12. um und erhält 299 mg (7*E*)-(1*R*,3*R*,20*S*)-24a-(Acetyloxy)-1,3-bis[[(1,1-dimethylethyl)dimethylsilyl]oxy]-24a-(thiazol-2-yl)-24a-homo-19-nor-9,10-secochola-5,7-dien **200** als

farblosen Schaum.
$^1$H-NMR ($CDCl_3$): δ= 0,07 ppm (s, 12H); 0,53 (s, 3H); 0,83 (d, 3H); 0,89 (s, 18H); 2,18 (s, 3H); 4,08 (m, 2H); 5,82 (d, 1H); 6,10 (t, 1H); 6,18 (d, 1H); 7,31 (d, 1H); 7,78 (d, 1H)

194. Man setzt 299 mg des Acetates **200** analog 13. um und erhält 240 mg (7*E*)-(1*R*,3*R*,20*S*)-1,3-Bis[[(1,1-dimethylethyl)dimethylsilyl]oxy]-24a-(thiazol-2-yl)-24ahomo-19-nor-9,10-secochola-5,7-dien-24a-ol **201** als farblosen Schaum.
$^1$H-NMR ($CDCl_3$): δ= 0,07 ppm (s, 12H); 0,53 (s, 3H); 0,83 (d, 3H); 0,89 (s, 18H); 4,07 (m, 2H); 5,01 (m, 1H); 5,81 (d, 1H); 6,18 (d, 1H); 7,30 (d, 1H); 7,72 (d, 1H)

195. Man behandelt 192 mg des Alkohols **201** analog 14. und erhält nach Trennung der Diastereomeren (bzgl. C-24a) über HPLC 41 mg der Titelverbindung **202a** und 49 mg der Titelverbindung **202b** als farblose Schäume.
$^1$H-NMR ($CD_2Cl_2/CD_3OD$): **202a:** δ= 0,50 ppm (s, 3H); 0,80 (d, 3H); 3,93 (m, 1H); 4,01 (m, 1H); 4,90 (t, 1H); 5,82 (d, 1H); 6,24 (d, 1H); 7,29 (d, 1H); 7,68 (d, 1H)
**202b**: δ= 0,50 ppm (s, 3H); 0,80 (d, 3H); 3,93 (m, 1H); 4,01 (m, 1H); 4,91 (t, 1H); 5,82 (d, 1H); 6,24 (d, 1H); 7,30 (d, 1H); 7,68 (d, 1H)

**Beispiel 43**

**(7*E*)-(1*R*,3*R*,20*S*)-1,3-Dihydroxy-24a-(thiazol-2-yl)-24-a-homo-19-nor-9,10-secochola-5,7-dien-24a-on 204**

196. Man behandelt 43 mg des Alkohols **201** analog 25., wobei 32 mg (7*E*)-(1*R*,3*R*,20*S*)-1,3-Bis[[(1,1-dimethylethyl)dimethylsilyl]oxy]-24a-(thiazol-2-yl)-24ahomo-19-nor-9,10-secochola-5,7-dien-24a-on **203** als farbloser Schaum anfallen.
$^1$H-NMR ($CDCl_3$): δ= 0,07 ppm (s, 12H); 0,52 (s, 3H); 0,88 (s, 18H); 0,89 (d, 3H); 4,08 (m, 2H); 5,81 (d, 1H); 6,18 (d, 1H); 7,68 (d, 1H); 8,00 (d, 12H)

197. Man behandelt 31 mg des Ketons **203** analog 26., wobei 12 mg der Titeverbindung **204** als farbloser Schaum anfallen.
$^1$H-NMR ($CD_2Cl_2$): δ= 0,51 ppm (s, 3H); 0,88 (d, 3H); 3,97 (m, 1H); 4,03 (m, 1H); 5,82 (d, 1H); 6,26 (d, 1H); 7,67 (d, 1H); 7,97 (d, 1H)

**Beispiel 44**

**(5*Z*,7*E*)-(1*S*,3*R*,20*S*,24a*R*)-24a-(Thiazol-2-yl)-24a-homo-9,10-secochola-5,7,10(19)-trien-1,3,24a-triol 205a und (5*Z*,7*E*)-(1*S*,3*R*,20*S*,24a*S*)-24a-(Thiazol-2-yl)-24a-homo-9,10-secochola-5,7,10(19)-trien-1,3,24a-triol 205b**

198. Man setzt 740 mg des Phosphinoxides **17**, welches mit 0,61 ml *n*-Butyllithium-Lösung (2,5 M in Hexan) deprotoniert wurde, mit 240 mg des Ketons **199** analog 15. um und erhält 392 mg (5*Z*,7*E*)-(1*S*,3*R*,20*S*)-24a-(Acetyloxy)-1,3-bis[[(1,1-dimethylethyl)dimethylsilyl]oxy]-24a-(thiazol-2-yl)-24a-homo-9,10-secochola-5,7,10(19)-trien **205** als farblosen Schaum.
$^1$H-NMR ($CDCl_3$): δ= 0,07 ppm (s, 12H); 0,52 (s, 3H); 0,83 (d, 3H); 0,90 (s, 18H); 2,16 (s, 3H); 4,19 (m, 1H); 4,38 (m, 1H); 4,88 (s, 1H); 5,19 (s, 1H); 6,01 (d, 1H); 6,10 (t, 1H); 6,24 (d, 1H); 7,31 (d, 1H); 7,78 (d, 1H)

199. Man setzt 392 mg des Acetates **205** analog 13. um und erhält 274 mg (5*Z*,7*E*)-(1*S*,3*R*,20*S*)-1,3-Bis[[(1,1-dimethylethyl)dimethylsilyl]oxy]-24a-(thiazol-2-yl)-24ahomo-9,10-secochola-5,7,10(19)-trien-24a-ol **206** als farblosen Schaum.
$^1$H-NMR ($CDCl_3$): δ= 0,07 ppm (s, 12H); 0,52 (s, 3H); 0,83 (d, 3H); 0,90 (s, 18H); 4,19 (m, 1H); 4,38 (m, 1H); 4,88 (s, 1H); 5,01 (m, 1H); 5,18 (s, 1H); 6,01 (d, 1H); 6,23 (d, 1H); 7,30 (d, 1H); 7,72 (d, 1H)

200. Man behandelt 218 mg des Alkohols **206** analog 14. und erhält nach Trennung der Diastereomeren über HPLC 40 mg der Titelverbindung **207a** und 39 mg der Titelverbindung **207b** als farblose Schäume.
$^1$H-NMR ($CD_2Cl_2/CD_3OD$): **207a**: δ= 0,50 ppm (s, 3H); 0,79 (d, 3H); 4,13 (m, 1H); 4,34 (m, 1H); 4,90 (t, 1H); 4,93 (s, 1H); 5,28 (s, 1H); 6,00 (d, 1H); 6,32 (d, 1H); 7,30 (d, 1H); 7,69 (d, 1H)
**207b:** δ= 0,50 ppm (s, 3H); 0,80 (d, 3H); 4,13 (m, 1H); 4,34 (m, 1H); 4,91 (t, 1H); 4,93 (s, 1H); 5,28 (s, 1H); 6,00 (d, 1H); 6,32 (d, 1H); 7,30 (d, 1H); 7,69 (d, 1H)

**Beispiel 45**

**(5*Z*,7*E*)-(1*S*,3*R*,20*S*)-1,3-Dihydroxy-24a-(thiazol-2-yl)-24a-homo-9,10-secochola-5,7,10(19)-trien-24a-on 209**

201. Man behandelt 51 mg des Alkohols **206** analog 25., wobei 42 mg (5*Z*,7*E*)-(1*S*,3*R*,20*S*)-1,3-Bis[[(1,1-dimethylethyl)dimethylsilyl]oxy]-24a-(thiazol-2-yl)-24ahomo-9,10-secochola-5,7,10(19)-trien-24a-on **208** als farbloser

Schaum anfallen.
$^1$H-NMR ($CDCl_3$): δ= 0,07 ppm (s, 12H); 0,52 (s, 3H); 0,89 (s, 18H); 0,90 (d, 3H); 4,19 (m, 1H); 4,38 (m, 1H); 4,88 (s, 1H); 5,19 (s, 1H); 6,02 (d, 1H); 6,24 (d, 1H); 7,68 (d, 1H); 8,00 (d, 1H)
202. Man behandelt 41 mg des Ketons 208 analog 26., wobei 14 mg der Titelverbindung **209** als farbloser Schaum anfallen.
$^1$H-NMR ($CD_2Cl_2$): δ= 0,50 ppm (s, 3H); 0,86 (d, 3H); 4,15 (m, 1H); 4,36 (m, 1H); 4,94 (s, 1H); 5,28 (s, 1H); 6,00 (d, 1H); 6,34 (d, 1H); 7,66 (d, 1H); 7,96 (d, 1H)

**Beispiel 46**

**(5*Z*,7*E*)-(1*S*,3*R*,24*S*)-24-(Thiazol-2-yl)-9,10-secochola-5,7,10(19)-trien-1,3,24-triol 217a und (5*Z*,7*E*)-(1*S*,3*R*,24*R*)-24-(Thiazol-2-yl)-9,10-secochola-5,7,10(19)-trien-1,3,24-triol 217b**

203. Man löst 21 g (5*Z*,7*E*)-(1*S*,3*R*,20*S*)-1,3-Bis[[(1,1-dimethylethyl)dimethylsilyl]oxy]-20-methyl-9,10-secopregna-5,7,10(19)-trien-21-al **210** (Schering AG, WO 97/41096) in b70 ml Tetrahydrofuran und 140 ml Ethanol und gibt bei 0°C 715 mg Natriumborhydrid zu. Es wird 1 h bei 0°C gerührt und anschließend mit Ammoniumchlorid-Lösung gequencht. Man extrahiert mit Essigester, wäscht die organische Phase mit Natriumchlorid-Lösung, trocknet über Natriumsulfat und engt ein. Der Rückstand wird mit Essigester/Hexan an Silicagel chromatographiert, wobei man 20,54 g **211** als farblosen Schaum erhält.
$^1$H-NMR ($CDCl_3$): δ= 0,07 ppm (s, 12H); 0,56 (s, 3H); 0,89 (s, 18H); 1,08 (d, 3H); 3,40 (dd, 1H); 3,67 (dd, 1H); 4,19 (m, 1H); 4,38 (m, 1H); 4,87 (s, 1H); 5,19 (s, 1H); 6,02 (d, 1H); 6,23 (d, 1H)
204. Man löst 14,54 g des Alkohols **211** in 233 ml Pyridin, gibt 11,14 g p-Toluolsulfonylchlorid zu und rührt 4 h bei Raumtemperatur. Es wird vorsichtig mit Natriumhydrogencarbonat-Lösung gequencht, mit Essigester extrahiert, die organische Phase mit Natriumchlorid-Lösung gewaschen und über Natriumsulfat getrocknet. Der Rückstand wird mit Essigester/Hexan an Silicagel chromatographiert, wobei man 14,73 g (5*Z*,7*E*)-(1*S*,3*R*,20*S*)-1,3-Bis[[(1,1-dimethylethyl)dimethylsilyl]oxy]-20-methyl-9,10-secopregna-5,7,10(19)-trien-21-(4-methylbenzensulfonat) **212** als farblosen Schaum erhält.
$^1$H-NMR ($CDCl_3$): δ= 0,07 ppm (s, 12H); 0,51 (s, 3H); 0,89 (s, 18H); 1,00 (d, 3H); 2,47 (s, 3H); 3,71 (dd, 1H); 3,99 (dd, 1H); 4,19 (m, 1H); 4,38 (m, 1H); 4,87 (s, 1H); 5,19 (s, 1H); 6,00 (d, 1H); 6,22 (d, 1H); 7,37 (d, 2H); 7,80 (d, 2H)
205. Man löst 14,73 g des Tosylates **212** in 295 ml Dichlormethan und 295 ml Acetonitril, gibt 16,9 g Lithiumbromid und 700 mg 1,8-Bis(dimethylamino)naphthalin hinzu und rührt über Nacht bei 60°C. Man quencht danach mit Natriumchlorid-Lösung, extrahiert mit Dichlormethan, wäscht die organische Phase mit Natriumchlorid-Lösung, trocknet über Natriumsulfat und engt ein. Der Rückstand wird mit Essigester/Hexan an Silicagel chromatographiert, wobei man 9,70 g (5*Z*,7*E*)-(1*S*,3*R*,20*S*)-1,3-Bis[[(1,1-dimethylethyl)dimethylsilyl]oxy]-21-brom-20-methyl-9,10-secopregna-5,7,10(19)-trien **213** als farblosen Schaum erhält.
$^1$H-NMR ($CDCl_3$): δ= 0,07 ppm (s, 12H); 0,58 (s, 3H); 0,89 (s, 18H); 1,11 (d, 3H); 3,38 (dd, 1H); 3,52 (dd, 1H); 4,19 (m, 1H); 4,38 (m, 1H); 4,88 (s, 1H); 5,20 (s, 1H); 6,02 (d, 1H); 6,25 (d, 1H)
206. Man stellt aus 15,5 ml Diisopropylamin und 49 ml *n*-Butyllithium (2,5 M in Hexan) in 150 ml Tetrahydrofuran Lithiumdiisopropylamid her, kühlt auf -78°C und tropft 6,34 ml Acetonitril zu. Nach 30 min werden 7,8 g des Bromides **213** in 20 ml Tetrahydrofuran zugegeben. Man läßt nun das Reaktionsgemisch auf Raumtemperatur erwärmen, rührt 2 h nach und quencht dann mit Natriumchlorid-Lösung. Es wird mit Essigester extrahiert, die organische Phase mit Natriumchlorid-Lösung gewaschen, über Natriumsulfat getrocknet und eingeengt. Der Rückstand wird mit Essigester/Hexan an Silicagel chromatographiert, wobei man 5,9 g (5*Z*,7*E*)-(1*S*,3*R*)-1,3-Bis[[(1,1-dimethylethyl)dimethylsilyl]oxy]-9,10-secochola-5,7,10(19)-trien-24-nitril **214** als farblosen Schaum erhält.
$^1$H-NMR ($CDCl_3$): δ= 0,07 ppm (s, 12H); 0,57 (s, 3H); 0,89 (s, 18H); 0,97 (d, 3H); 4,19 (m, 1H); 4,38 (m, 1H); 4,88 (s, 1H); 5,19 (s, 1H); 6,02 (d, 1H); 6,23 (d, 1H)
207. Man löst 3,2 g des Nitrils **214** in 50 ml Tetrahydrofuran und kühlt auf 0°C. Bei dieser Temperatur tropft man 16,8 ml Diisobutylaluminiumhydrid (1,2 M in Toluol) hinzu und rührt 2 h nach. Anschließend setzt man Ammoniumchlorid-Lösung zu, saugt den Nierderschlag ab und extrahiert mit Essigester. Die organische Phase wird mit Natriumchlorid-Lösung gewaschen, über Natriumsulfat getrocknet und eingeengt. Der Rückstand wird mit Essigester/Hexan an Silicagel chromatographiert, wobei man 2,5 g (5*Z*,7*E*)-(1*S*,3*R*)-1,3-Bis[[(1,1-dimethylethyl)dimethyl-silyl]oxy]-9,10-secochola-5,7,10(19)-trien-24-al **215** als farblosen Schaum erhält.
$^1$H-NMR ($CDCl_3$): δ= 0,07 ppm (s, 12H); 0,54 (s, 3H); 0,89 (s, 18H); 0,94 (d, 3H); 4,19 (m, 1H); 4,38 (m, 1H); 4,88 (s, 1H); 5,19 (s, 1H); 6,01 (d, 1H); 6,23 (d, 1H); 9,79 (sbr, 1H)
208. Man legt 285 mg Thiazol in 5 ml Tetrahydrofuran vor und gibt bei -78°C 1,34 ml *n*-Butyllithium-Lösung (2,5 M in Hexan) zu. Nach 30 min bei dieser Temperatur tropft man 400 mg des Aldehydes **215** in 2 ml Tetrahydrofuran zu und rührt 1 h nach. Anschließend wird mit Natriumchlorid-Lösung gequencht, mit Essigester extrahiert, die organische Phase mit Natriumchlorid-Lösung gewaschen und über Natriumsulfat getrocknet. Das Lösungsmittel wird entfernt

und der Rückstand wird an Silicagel mit Essigester/Hexan chromatographiert, wobei man 311 mg (5*Z*,7*E*)-(1*S*,3*R*)-1,3-Bis[[(1,1-dimethylethyl)dimethylsilyl]oxy]-24-(thiazol-2-yl)-9,10-secochola-5,7,10(19)-trien-24-ol **216** als farbloses Öl erhält.
$^1$H-NMR ($CDCl_3$): δ= 0,07 ppm (s, 12H); 0,53 (s, 3H); 0,89 (s, 18H); 0,95 (d, 3H); 4,19 (m, 1H); 4,38 (m, 1H); 4,88 (s, 1H); 4,98 (m, 1H); 5,19 (s, 1H); 6,01 (d, 1H); 6,23 (d, 1H); 7,30 (d, 1H); 7,72 (d, 1H)

209. Man behandelt 130 mg des Alkohols **216** analog 14. und erhält nach Trennung der Diastereomeren (bzgl. C-24a) über HPLC 19 mg der Titelverbindung **217a** und 16 mg der Titelverbindung **217b** als farblose Schäume.
$^1$H-NMR ($CD_2Cl_2$/$CD_3OD$): **217a:** δ= 0,50 ppm (s, 3H); 0,92 (d, 3H); 4,10 (m, 1H); 4,33 (m, 1H); 4,85 (m, 1H); 4,93 (s, 1H); 5,28 (s, 1H); 6,01 (d, 1H); 6,32 (d, 1H); 7,32 (d, 1H); 7,64 (d, 1H)
**217b:** δ= 0,51 ppm (s, 3H); 0,92 (d, 3H); 4,10 (m, 1H); 4,33 (m, 1H); 4,86 (m, 1H); 4,93 (s, 1H); 5,28 (s, 1H); 6,01 (d, 1H); 6,33 (d, 1H); 7,32 (d, 1H); 7,65 (d, 1H)

### Beispiel 47

#### (5*Z*,7*E*-(1*S*,3*R*)-1,3-Dihydroxy-24-(thiazol-2-yl)-9,10-secochola-5,7,10(19)-trien-24-on 219

210. Man behandelt 40 mg des Alkohols **216** analog 25., wobei 24 mg (5*Z*,7*E*)-(1*S*,3*R*)-1,3-Bis[[(1,1-dimethylethyl)dimethylsilyl]oxy]-24-(thiazol-2-yl)-9,10-secochola-5,7,10(19)-trien-24-on **218** als farbloser Schaum anfallen.
$^1$H-NMR ($CD_2Cl_2$): δ= 0,04 ppm (s, 12H); 0,52 (s, 3H); 0,84 (s, 18H); 0,97 (d, 3H); 4,17 (m, 1H); 4,36 (m, 1H); 4,84 (s, 1H); 5,16 (s, 1H); 6,01 (d, 1H); 6,23 (d, 1H); 7,65 (d, 1H); 7,97 (d, 1H)

211. Man behandelt 24 mg des Ketons **218** analog 26., wobei 14 mg der Titelverbindung **219** als farbloser Schaum anfallen.
$^1$H-NMR ($CD_2Cl_2$): δ= 0,55 ppm (s, 3H); 0,99 (d, 3H); 4,15 (m, 1H); 4,36 (m, 1H); 4,93 (s, 1H); 5,28 (s, 1H); 6,00 (d, 1H); 6,33 (d, 1H); 7,65 (d, 1H); 7,98 (d, 1H)

### Beispiel 48

#### (5*Z*,7*E*)-(1*S*,3*R*,24*S*)-24-(4-Methylthiazol-2-yl)-9,10-secochola-5,7,10(19)-trien-1,3,24-triol 221a und (5*Z*,7*E*)-(1*S*,3*R*,24*R*)-24-(4-Methylthiazol-2-yl)-9,10-secochola-5,7,10(19)-trien-1,3,24-triol 221b

212. Man legt 991 mg 4-Methylthiazol in 15 ml Tetrahydrofuran vor und gibt bei -78°C 4 ml *n*-Butyllithium-Lösung (2,5 M in Hexan) zu. Nach 30 min bei dieser Temperatur tropft man 1,2 g des Aldehydes **215** in 8 ml Tetrahydrofuran zu und rührt 1 h nach. Anschließend wird mit Natriumchlorid-Lösung gequencht, mit Essigester extrahiert, die organische Phase mit Natriumchlorid-Lösung gewaschen und über Natriumsulfat getrocknet. Das Lösungsmittel wird entfernt und der Rückstand wird an Silicagel mit Essigester/Hexan chromatographiert, wobei man 1,03 g (5*Z*,7*E*)-(1*S*,3*R*)-1,3-Bis[[(1,1-dimethylethyl)dimethylsilyl] oxy]-24-(4-methylthiazol-2-yl)-9,10-secochola-5,7,10(19)-trien-24-ol **220** als farbloses Öl erhält.
$^1$H-NMR ($CD_2Cl_2$): δ= 0,06 ppm (s, 12H); 0,53 (s, 3H); 0,87 (s, 18H); 0,94 (d, 3H); 2,35 (s, 3H); 4,17 (m, 1H); 4,36 (m, 1H); 4,85 (s, 1H); 4,86 (m, 1H); 5,17 (s, 1H); 6,01 (d, 1H); 6,23 (d, 1H); 6,83 (s, 1H)

213. Man behandelt 250 mg des Alkohols **220** analog 14. und erhält nach Trennung der Diastereomeren (bzgl. C-24) über HPLC 57 mg der Titelverbindung **221a** und 63 mg der Titelverbindung **221b** als farblose Schäume.
$^1$H-NMR ($CD_2Cl_2$): **221a:** δ= 0,51 ppm (s, 3H); 0,93 (d, 3H); 2,36 (s, 3H); 4,16 (m, 1H); 4,36 (m, 1H); 4,85 (m, 1H); 4,93 (s, 1H); 5,28 (s, 1H); 6,00 (d, 1H); 6,33 (d, 1H); 6,81 (s, 1H)
**221b:** δ= 0,51 ppm (s, 3H); 0,93 (d, 3H); 2,36 (s, 3H); 4,16 (m, 1H); 4,35 (m, 1H); 4,85 (m, 1H); 4,92 (s, 1H); 5,27 (s, 1H); 6,00 (d, 1H); 6,33 (d, 1H); 6,82 (s, 1H)

### Beispiel 49

#### (5*Z*,7*E*)-(1*S*,3*R*)-1,3-Dihydroxy-24-(4-methylthiazol-2-yl)-9,10-secochola-5,7,10(19)-trien-24-on 223

214. Man behandelt 70 mg des Alkohols **220** analog 25., wobei 56 mg (5*Z*,7*E*)-(1*S*,3*R*)-1,3-Bis[[(1,1-dimethylethyl)dimethylsilyl]oxy]-24-(4-methylthiazol-2-yl)-9,10-secochola-5,7,10(19)-trien-24-on **222** als farbloser Schaum anfallen.
$^1$H-NMR ($CD_2Cl_2$): δ= 0,04 ppm (s, 12H); 0,51 (s, 3H); 0,82 (s, 18H); 0,95 (d, 3H); 2,44 (s, 3H); 4,16 (m, 1H); 4,35 (m, 1H); 4,82 (s, 1H); 5,15 (s, 1H); 5,99 (d, 1H); 6,22 (d, 1H); 7,18 (s,1H)

215. Man behandelt 56 mg des Ketons **222** analog 26., wobei 25 mg der Titelverbindung **223** als farbloser Schaum anfallen.
$^1$H-NMR ($CD_2Cl_2$): δ= 0,54 ppm (s, 3H); 0,99 (d, 3H); 2,49 (s, 3H); 4,16 (m, 1H); 4,38 (m, 1H); 4,94 (s, 1H); 5,28 (s,

1H); 6,00 (d, 1H); 6,34 (d, 1H); 7,24 (d, 1H)

**Beispiel 50**

**(5*Z*,7*E*)-(1*S*,3*R*,24*S*)-24-(Thien-2-yl)-9,10-secochola-5,7,10(19)-trien-1,3,24-triol 225a** und **(5*Z*,7*E*)-(1*S*,3*R*,24*R*)-24-(Thien-2-yl)-9,10-secochola-5,7,10(19)-trien-1,3,24-triol 225b**

216. Man legt 450 mg Thiophen in 10 ml Tetrahydrofuran vor und gibt bei -78°C 2 ml *n*-Butyllithium-Lösung (2,5 M in Hexan) zu. Nach 30 min bei dieser Temperatur tropft man 600 mg des Aldehydes **215** in 4 ml Tetrahydrofuran zu und rührt 1 h nach. Anschließend wird mit Natriumchlorid-Lösung gequencht, mit Essigester extrahiert, die organische Phase mit Natriumchlorid-Lösung gewaschen und über Natriumsulfat getrocknet. Das Lösungsmittel wird entfernt und der Rückstand wird an Silicagel mit Essigester/Hexan chromatographiert, wobei man 398 mg (5*Z*,7*E*)-(1*S*,3*R*)-1,3-Bis[[(1,1-dimethylethyl)dimethylsilyl]oxy]-24-(thien-2-yl)-9,10-secochola-5,7,10(19)-trien-24-ol **224** als farbloses Öl erhält.
$^1$H-NMR ($CDCl_3$): δ= 0,06 ppm (s, 12H); 0,53 (s, 3H); 0,89 (s, 18H); 0,94 (d, 3H); 4,18 (m, 1H); 4,38 (m, 1H); 4,86 (s, 1H); 4,86 (m, 1H); 5,17 (s, 1H); 6,01 (d, 1H); 6,23 (d, 1H); 6,98 (m, 2H); 7,24 (m, 1H)
217. Man behandelt 92 mg des Alkohols **224** analog 14. und erhält nach Trennung der Diastereomeren (bzgl. C-24) über HPLC 21 mg der Titelverbindung **225a** und 19 mg der Titelverbindung **225b** als farblose Schäume.
$^1$H-NMR ($CD_2Cl_2$): **225a:** δ= 0,51 ppm (s, 3H); 0,92 (d, 3H); 4,14 (m, 1H); 4,34 (m, 1H); 4,83 (dd, 1H); 4,93 (s, 1H); 5,28 (s, 1H); 6,00 (d, 1H); 6,33 (d, 1H); 6,92 (m, 2H); 7,22 (dd, 1H)
**225b**: δ= 0,51 ppm (s, 3H); 0,92 (d, 3H); 4,15 (m, 1H); 4,35 (m, 1H); 4,84 (t, 1H); 4,92 (s, 1H); 5,27 (s, 1H); 6,00 (d, 1H); 6,33 (d, 1H); 6,92 (m, 1H); 7,21 (dd, 1H)

**Beispiel 51**

**(5*Z*,7*E*)-(1*S*,3*R*)-1,3-Dihydroxy-24-(thien-2-yl)-9,10-secochola-5,7,10(19)-trien-24-on 227**

218. Man behandelt 70 mg des Alkohols **224** analog 25., wobei 28 mg (5*Z*,7*E*)-(1*S*,3*R*)-1,3-Bis[[(1,1-dimethylethyl)dimethylsilyl]oxy]-24-(thien-2-yl)-9,10-secochola-5,7,10(19)-trien-24-on **226** als farbloser Schaum anfallen.
$^1$H-NMR ($CD_2Cl_2$): δ= 0,05 ppm (s, 12H); 0,52 (s, 3H); 0,84 (s, 18H); 0,95 (d, 3H); 4,16 (m, 1H); 4,35 (m, 1H); 4,83 (s, 1H); 5,15 (s, 1H); 6,00 (d, 1H); 6,23 (d, 1H); 7,13 (t, 1H); 7,63 (d, 1H); 7,70 (d, 1H)
219. Man behandelt 28 mg des Ketons **226** analog 26., wobei 11 mg der Titelverbindung **227** als farbloser Schaum anfallen.
$^1$H-NMR ($CD_2Cl_2$): δ= 0,54 ppm (s, 3H); 0,99 (d, 3H); 4,16 (m, 1H); 4,38 (m, 1H); 4,95 (s, 1H); 5,28 (s, 1H); 6,00 (d, 1H); 6,35 (d, 1H); 7,12 (dd, 1H); 7,62 (d, 1H); 7,71 (d, 1H)

**Beispiel 52**

**(5*Z*,7*E*)-(1*S*,3*R*,24*S*)-24-(4-Methylthien-2-yl)-9,10-secochola-5,7,10(19)-trien-1,3,24-triol 229a** und **(5*Z*,7*E*)-(1*S*,3*R*,24*R*)-24-(4-Methylthien-2-yl)-9,10-secochola-5,7,10(19)-trien-1,3,24-triol 229b**

220. Man legt 500 mg 4-Methylthiophen in 10 ml Tetrahydrofuran vor und gibt bei -78°C 2 ml *n*-Butyllithium-Lösung (2,5 M in Hexan) zu. Nach 30 min bei dieser Temperatur tropft man 600 mg des Aldehydes **215** in 4 ml Tetrahydrofuran zu und rührt 1 h nach. Anschließend wird mit Natriumchlorid-Lösung gequencht, mit Essigester extrahiert, die organische Phase mit Natriumchlorid-Lösung gewaschen und über Natriumsulfat getrocknet. Das Lösungsmittel wird entfernt und der Rückstand wird an Silicagel mit Essigester/Hexan chromatographiert, wobei man 402 mg (5*Z*,7*E*)-(1*S*,3*R*)-1,3-Bis[[(1,1-dimethylethyl)dimethylsilyl]oxy]-24-(4-methylthien-2-yl)-9,10-secochola-5,7,10(19)-trien-24-ol **228** als farbloses Öl erhält.
$^1$H-NMR ($CD_2Cl_2$): δ= 0,06 ppm (s, 12H); 0,55 (s, 3H); 0,86 (s, 18H); 0,94 (d, 3H); 2,21 (s, 3H); 4,16 (m, 1H); 4,36 (m, 1H); 4,84 (s, 1H); 4,86 (m, 1H); 5,16 (s, 1H); 6,01 (d, 1H); 6,23 (d, 1H); 6,74 (m, 1H); 7,78 (m, 1H)
221. Man behandelt 98 mg des Alkohols **228** analog 14. und erhält nach Trennung der Diastereomeren (bzgl. C-24) über HPLC 22 mg der Titelverbindung **229a** und 17 mg der Titelverbindung **229b** als farblose Schäume.
$^1$H-NMR ($CD_2Cl_2$): **229a:** δ= 0,52 ppm (s, 3H); 0,92 (d, 3H); 2,21 (s, 3H); 4,15 (m, 1H); 4,35 (m, 1H); 4,86 (dd, 1H); 4,93 (s, 1H); 5,28 (s, 1H); 5,99 (d, 1H); 6,33 (d, 1H); 6,84 (m, 1H); 6,89 (m, 1H)
**229b:** δ= 0,52 ppm (s, 3H); 0,92 (d, 3H); 2,22 (s, 3H); 4,15 (m, 1H); 4,35 (m, 1H); 4,85 (t, 1H); 4,92 (s, 1H); 5,27 (s, 1H); 6,00 (d, 1H); 6,33 (d, 1H); 6,84 (m, 1H); 6,88 (m, 1H)

**Beispiel 53**

**(5*Z*,7*E*)-(1*S*,3*R*)-1,3-Dihydroxy-24-(4-methylthien-2-yl)-9,10-secochola-5,7,10(19)-trien-24-on 231**

222. Man behandelt 65 mg des Alkohols **228** analog 25., wobei 34 mg (5*Z*,7*E*)-(1*S*,3*R*)-1,3-Bis[[(1,1-dimethylethyl)dimethylsilyl]oxy]-24-(4-methylthien-2-yl)-9,10-secochola-5,7,10(19)-trien-24-on **230** als farbloser Schaum anfallen.
$^1$H-NMR ($CD_2Cl_2$): δ= 0,05 ppm (s, 12H); 0,53 (s, 3H); 0,84 (s, 18H); 0,94 (d, 3H); 2,33 (s, 3H); 4,16 (m, 1H); 4,35 (m, 1H); 4,83 (s, 1H); 5,15 (s, 1H); 6,01 (d, 1H); 6,23 (d, 1H); 7,65 (s, 1H); 7,70 (s, 1H)

223. Man behandelt 34 mg des Ketons **230** analog 26., wobei 13 mg der Titelverbindung **231** als farbloser Schaum anfallen.
$^1$H-NMR ($CD_2Cl_2$): δ= 0,54 ppm (s, 3H); 0,98 (d, 3H); 2,32 (s, 3H); 4,16 (m, 1H); 4,37 (m, 1H); 4,95 (s, 1H); 5,28 (s, 1H); 6,00 (d, 1H); 6,34 (d, 1H); 7,63 (s, 1H); 7,71 (s, 1H)

**Beispiel 54**

**(7*E*)-(1*R*,3*R*,24a*R*)-24a-Fluor-24a-(thiazol-2-yl)-24a-homo-19-nor-9,10-secochola-5,7-dien-1,3-diol 236a** und **(7*E*)-(1*R*,3*R*,24a*S*)-24a-Fluor-24a-(thiazol-2-yl)-24a-homo-19-nor-9,10-secochola-5,7-dien-1,3-diol 236b**

224. Man legt 1,0 g des Alkohols **21** bei -78°C in 80 ml Dichlormethan vor, gibt 0,044 ml Diethylaminoschwefeltrifluorid (DAST) zu und rührt 15 min nach. Es wird mit Natriumhydrogencarbonat gequencht, mit Dichlormethan extrahiert und über Natriumsulfat getrocknet. Nach dem Einengen und der Chromatographie an Silicagel mit Essigester/Hexan erhält man 765 mg [1*R*-[1α(1*R**),3aβ,4α,7aα]]-1-[5-Fluor-1-methyl-5-(thiazol-2-yl)pentyl]octahydro-7a-methyl-4-[(triethylsilyl)oxy]-1*H*-inden **232** als farbloses Öl.
$^1$H-NMR ($CDCl_3$): δ= 0,55 ppm (q, 6H); 0,90 (d, 3H); 0,91 (s, 3H); 0,96 (t, 9H); 4,02 (m, 1H); 5,24 (dbr, 1H); 7,35 (d, 1H); 7,80 (t, 1H)

225. Man behandelt 765 mg der Verbindung **232** analog 10. und erhält 505 mg [1*R*-[1α(1*R**),3aβ,4α,7aα]]-1-[5-Fluor-1-methyl-5-(thiazol-2-yl)pentyl]octahydro-7amethyl-1*H*-inden-4-ol **233** als farbloses Öl.
$^1$H-NMR ($CDCl_3$): δ= 0,90 ppm (d, 3H); 0,92 (s, 3H); 4,08 (m, 1H); 5,75 (dbr, 1H); 7,38 (d, 1H); 7,80 (t, 1H)

226. Man behandelt 505 mg des Alkohols **233** analog 11. und erhält 468 mg [1*R*-[1α(1*R**),3aβ,7aα]]-1-[5-Fluor-1-methyl-5-(thiazol-2-yl)pentyl]octahydro-7a-methyl-4*H*-inden-4-on **234** als farbloses Öl.
$^1$H-NMR ($CDCl_3$): δ= 0,62 ppm (s, 3H); 0,98 (d, 3H); 2,45 (dd, 1H); 5,75 (dbr, 1H); 7,38 (d, 1H); 7,80 (t, 1H)

227. Man setzt 676 mg des Phosphinoxides **13**, welches mit 0,57 ml *n*-Butyllithium-Lösung (2,5 M in Hexan) deprotoniert wurde, mit 200 mg des Ketons **234** analog 12. um und erhält 341 mg (7*E*)-(1*R*,3*R*)-1,3-Bis[[(1,1-dimethylethyl)dimethylsilyl]oxy]-24afluor-24a-(thiazol-2-yl)-24a-homo-19-nor-9,10-secochola-5,7-dien **235** als farblosen Schaum.
$^1$H-NMR ($CDCl_3$): δ= 0,06 ppm (s, 12H); 0,53 (s, 3H); 0,89 (s, 18H); 0,93 (d, 3H); 4,09 (m, 2H); 5,75 (dbr, 1H); 5,82 (d, 1H); 6,18 (d, 1H); 7,39 (d, 1H); 7,80 (t, 1H)

228. Man behandelt 331 mg des Fluorides **235** analog 14. und erhält nach Trennung der Diastereomeren (bzgl. C-24a) über HPLC 67 mg der Titelverbindung **236a** und 69 mg der Titelverbindung **236b** als farblose Schäume.
$^1$H-NMR ($CD_2Cl_2$): **236a:** δ= 0,52 ppm (s, 3H); 0,91 (d, 3H); 3,98 (m, 1H); 4,05 (m, 1H); 5,73 (dbr, 1H); 5,85 (d, 1H); 6,27 (d, 1H); 7,40 (d, 1H); 7,78 (t, 1H)
**236b**: δ= 0,52 ppm (s, 3H); 0,91 (d, 3H); 3,97 (m, 1H); 4,04 (m, 1H); 5,74 (dbr, 1H); 5,85 (d, 1H); 6,27 (d, 1H); 7,40 (d, 1H); 7,78 (t, 1H)

**Beispiel 55**

**(5*Z*,7*E*)-(1*S*,3*R*,24a*R*)-24a-Fluor-24a-(thiazol-2-yl)-24a-homo-9,10-secochola-5,7,10(19)-trien-1,3-diol 238a** und **(5*Z*,7*E*)-(1*S*,3*R*,24a*S*)-24a-Fluor-24a-(thiazol-2-yl)-24a-homo-9,10-secochola-5,7,10(19)-trien-1,3-diol 238b**

229. Man setzt 517 mg des Phosphinoxides **17**, welches mit 0,42 ml *n*-Butyllithium-Lösung (2,5 M in Hexan) deprotoniert wurde, mit 150 mg des Ketons **234** analog 15. um und erhält 293 mg (5Z,7*E*)-(1*S*,3*R*)-1,3-Bis[[(1,1-dimethylethyl)dimethylsilyl]oxy]-24afluor-24a-(thiazol-2-yl)-24a-homo-9,10-secochola-5,7,10(19)-trien **237** als farblosen Schaum.
$^1$H-NMR ($CDCl_3$): δ= 0,07 ppm (s, 12H); 0,54 (s, 3H); 0,92 (s, 18H); 0,96 (d, 3H); 4,20 (m, 1H); 4,39 (m, 1H); 4,89 (s, 1H); 5,20 (s, 1H); 5,77 (dbr, 1H); 6,05 (d, 1H); 6,25 (d, 1H); 7,40 (d, 1H); 7,80 (t, 1H)

230. Man behandelt 283 mg des Fluorides **237** analog 14. und erhält nach Trennung der Diastereomeren (bzgl. C-24a) über HPLC 67 mg der Titelverbindung **238a** und 59 mg der Titelverbindung **238b** als farblose Schäume.

$^1H$-NMR ($CD_2Cl_2$): **238a:** δ= 0,52 ppm (s, 3H); 0,92 (d, 3H); 4,18 (m, 1H); 4,38 (m, 1H); 4,95 (s, 1H); 5,28 (s, 1H); 5,72 (dbr, 1H); 6,00 (d, 1H); 6,36 (d, 1H); 7,40 (d, 1H); 7,80 (t, 1H)
**238b**: δ= 0,51 ppm (s, 3H); 0,89 (d, 3H); 4,11 (m, 1H); 4,33 (m, 1H); 4,90 (t, 1H); 4,92 (s, 1H); 5,27 (s, 1H); 6,00 (d, 1H); 6,32 (d, 1H); 7,29 (d, 1H); 7,68 (d, 1H)

**Beispiel 56**

**(7*E*)-(1*R*,3*R*,24a*R*)-24a-(Acetyloxy)-24a-(thiazol-2-yl)-24a-homo-19-nor-9,10-secochola-5,7-dien-1,3,24a-triol 239a** und **(7*E*)-(1*R*,3*R*,24a*S*)-24a-(Acetyloxy)-24a-(thiazol-2-yl)-24a-homo-19-nor-9,10-secochola-5,7-dien-1,3,24a-triol 239b**

231. Man legt 117 mg des Acetates **25** in 6 ml Tetrahydrofuran vor, gibt 0,9 ml Fluorwasserstoff-Pyridin-Komplex hinzu und rührt 4 h bei 25°C. Das Gemisch wird mit Natriumhydrogencarbonat-Lösung versetzt, mit Essigester extrahiert und die organische Phase wird mit Natriumchlorid-Lösung gewaschen. Nach dem Trocknen über Natriumsulfat wird das Lösungsmittel entfernt und der Rückstand wird an Silicagel mit Essigester/Hexan chromatographiert, wobei man 72 mg der Titelverbindungen **239a** und **239b** als farbloses Öl erhält. Die Trennung der Diastereomeren erfolgt über HPLC und liefert 31 mg der Titelverbindung **239a** und 26 mg der Titelverbindung **239b** als farblose Schäume.
$^1H$-NMR ($CD_2Cl_2$): **239a:** δ= 0,51 ppm (s, 3H); 0,88 (d, 3H); 2,11 (s, 3H); 3,98 (m, 1H); 4,05 (m, 1H); 5,84 (d, 1H); 6,04 (dd, 1H); 6,37 (d, 1H); 7,31 (d, 1H); 7,71 (d, 1H)
**239b:** δ= 0,51 ppm (s, 3H); 0,89 (d, 3H); 2,12 (s, 3H); 3,98 (m, 1H); 4,06 (m, 1H); 5,84 (d, 1H); 6,05 (dd, 1H); 6,37 (d, 1H); 7,31 (d, 1H); 7,71 (d, 1H)

**Beispiel 57**

**(7*E*)-(1*R*,3*R*,24a*R*)-24a-(2,2-Dimethyl-1-oxopropyl)oxy-24a-(thiazol-2-yl)-24ahomo-19-nor-9,10-secochola-5,7-dien-1,3,24a-triol 241a und (7*E*)-(1*R*,3*R*,24a*S*)-24a-(2,2-Dimethyl-1-oxopropyl)oxy-24a-(thiazol-2-yl)-24a-homo-19-nor-9,10-secochola-5,7-dien-1,3,24a-triol 241b**

232. Man legt 200 mg des Alkohols **26** in 6 ml Pyridin vor und rührt 5 h bei 25°C. Anschließend quencht man mit Natriumhydrogencarbonat-Lösung, extrahiert mit Essigester und wäscht die organische Phase mit Natriumchlorid-Lösung. Nach dem Trocknen über Natriumsulfat wird das Lösungsmittel entfernt und der Rückstand wird an Silicagel mit Essigester/Hexan chromatographiert, wobei man 1120 mg (7*E*)-(1*R*,3*R*)-1,3-Bis[[(1,1-dimethylethyl)dimethylsilyl]oxy]-24a-(2,2-dimethyl-1-oxopropyl)oxy-24a-(thiazol-2-yl)-24a-homo-19-nor-9,10-secochola-5,7-dien **240** als farblosen Schaum erhält.
$^1H$-NMR ($CD_2Cl_2$): δ= 0,07 ppm (s, 3H); 0,53 (s, 3H); 0,89 (s, 18H); 0,92 (d, 3H); 1,23 (s, 9H); 3,98 (m, 1H); 4,05 (m, 1H); 5,84 (d, 1H); 6,03 (dd, 1H); 6,37 (d, 1H); 7,31 (d, 1H);7,71(d,1H)
233. Man legt 120 mg des Pivalates **240** in 7 ml Tetrahydrofuran vor, gibt 0,4 ml Fluorwasserstoff-Pyridin-Komplex hinzu und rührt 4 h bei 25°C. Das Gemisch wird mit Natriumhydrogencarbonat-Lösung versetzt, mit Essigester extrahiert und die organische Phase wird mit Natriumchlorid-Lösung gewaschen. Nach dem Trocknen über Natriumsulfat wird das Lösungsmittel entfernt und der Rückstand wird an Silicagel mit Essigester/Hexan chromatographiert, wobei man 74 mg der Titelverbindungen **241a** und **241b** als farbloses Öl erhält. Die Trennung der Diastereomeren erfolgt über HPLC und liefert 28 mg der Titelverbindung **241a** und 27 mg der Titelverbindung **241b** als farblose Schäume.
$^1H$-NMR ($CD_2Cl_2$): **241a:** δ= 0,51 ppm (s, 3H); 0,88 (d, 3H); 1,24 (s, 9H); 3,98 (m, 1H); 4,05 (m, 1H); 5,84 (d, 1H); 6,05 (dd, 1H); 6,37 (d, 1H); 7,30 (d, 1H); 7,72 (d, 1H)
**241b:** δ= 0,51 ppm (s, 3H); 0,88 (d, 3H); 1,23 (s, 9H); 3,98 (m, 1H); 4,06 (m, 1H); 5,84 (d, 1H); 6,05 (dd, 1H); 6,37 (d, 1H); 7,31 (d, 1H); 7,72 (d, 1H)

**Beispiel 58**

**(3R,5R)-3,5-Bis(benzoyloxy)-4-brom-cyclohexan-1-on**

234.

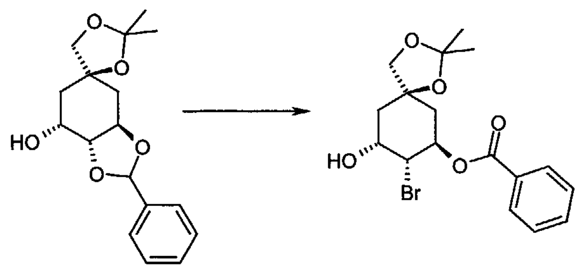

XXIX XXXb

17,9 g der Ausgangsverbindung XXIX (synthetisiert nach J.-L. Montchamp, J.W. Frost *J. Am. Chem. Soc.* **113**, 6296 (1991)) werden in 500ml Benzol gelöst. Anschließend werden 11.26 g N-Bromsuccinimid und eine Spatelspitze AIBN zugegeben und 1,5 Stunden gerührt. Die fast entfärbte Lösung wird mit Essigester extrahiert, mit $Na_2S_2O_3$-Lösung und anschließend mit Natriumchloridlösung gewaschen, getrocknet und eingeengt. Säulenchromatographie mit Hexan/Essigester (1:1) ergibt 14,7 g des bromierten Produktes XXXb.

235.

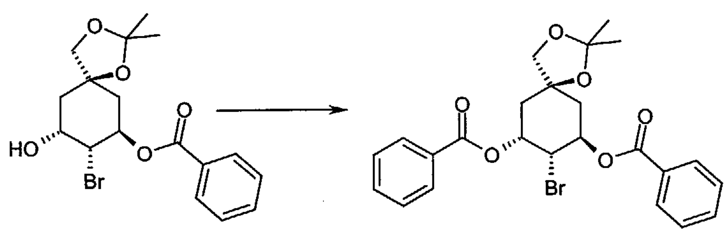

XXXb XXXIb

14,7 g des Alkohols XXXb werden in 350 ml Pyridin vorgelegt und mit 12,8 ml Benzoylchlorid und einer Spatelspitze DMAP (4-Dimethylaminopyridin) versetzt und 12 Stunden bei Raumtemperatur gerührt. Zum Reaktionsgemisch wird vorsichtig Natriumhydrogencarbonat zugesetzt, 30 min gerührt, mit Methylenchlorid extrahiert, über Natriumsulfat getrocknet und eingeengt. Säulenchromatographie mit Essigester/Hexan (1:9) ergibt 15,4 g Diester XXXIb.

236.

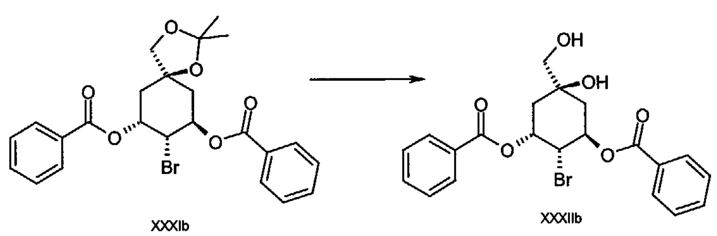

XXXIb XXXIIb

15,4 g Diester XXXIb aus 235 werden in 300 ml Methanol gelöst und mit 5,9 g p-Toluolsulfonsäure versetzt. Der Ansatz wird 12 Stunden bei Raumtemperatur gerührt. Nach Zugabe von 11 Essigester wird viermal mit Natriumchloridlösung gewaschen, über Natriumsulfat getrocknet und eingeengt. Säulenchromatographie mit Hexan/Essigester ergibt 9,2 g Diol XXXIIb.

237.

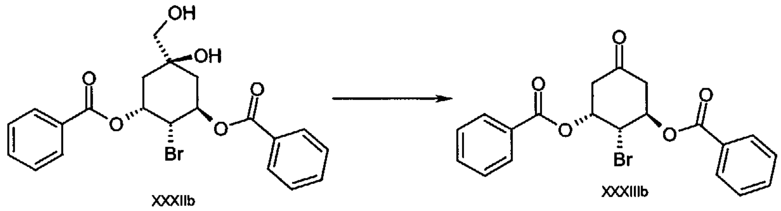

XXXIIb

XXXIIIb

5 g des aus 236 erhaltenen Diols XXXIIb werden in 200 ml Methanol vorgelegt. 8,31 g Natriumperiodat in 60 ml Wasser werden zugetropft und anschließend eine Stunde bei 0°C gerührt. Es wird mit Natriumchloridlösung gequencht, mit Essigester extrahiert, getrocknet und eingeengt. Es werden 4,68 g Rohprodukt XXXIIIb erhalten, das ohne weitere Reinigung in die Wittig-Reaktion (Beispiel 59, Vorschrift 243 und 60, Vorschrift 249) eingesetzt wird.

**Beispiel 59**

**(7E)-(1R,3R)-2-Brom-24a-(thiazol-2-yl)-24a-homo-19-nor-9,10-secochola-5,7-dien-1,3,24a-triol**

238. **[1*R*-[1α(1*R**),3aβ,7aα]]-1-[5-(Hydroxy)-1-methyl-5-(thiazol-2-yl)pentyl]octahydro-7a-methyl-4*H*-inden-4-on**

Man legt 500mg [1*R*-[1α(1*R**),3aβ,7aα]]-1-[5-(Acetyloxy)-1-methyl-5-(thiazol-2-yl)pentyl]octahydro-7a-methyl-4*H*-inden-4-on **12**, erhältlich nach Beispiel 1, in 30ml Methanol vor und versetzt mit 800mg Kaliumcarbonat. Nach 24h Rühren bei Raumtemperatur wird mit Methylenchlorid versetzt und mit Natriumchloridlösung gewaschen. Nach Chromatographie über Kieselgel mit Essigester/Hexan werden 440mg [1*R*-[1α(1*R**),3aβ,7aα]]-1-[5-(Hydroxy)-1-methyl-5-(oxazol-4-yl)pentyl]octahydro-7a-methyl-4*H*-inden-4-on erhalten.

239. **[1*R*-[1α(1*R**),3aβ,7aα]]-Octahydro-7a-methyl-1-[1-methyl-5-(thiazol-2-yl)-5-[(triethylsilyl)oxy]pentyl]-4H-inden-4-on**

435mg [1*R*-[1α(1*R**),3aβ,7aα]]-1-[5-(Hydroxy)-1-methyl-5-(thiazol-2-yl)pentyl]octahydro-7a-methyl-4*H*-inden-4-on werden in 7ml DMF mit 0,31ml Chlortriethylsilan und 151mg Imidazol versetzt und drei Tage bei Raumtemperatur gerührt. Nach Extrahieren mit Essigester, Waschen mit Natriumchloridlösung und Trocknen wird mit Essigester/Hexan chromatographiert. Es werden 584mg [1*R*-[1α(1*R**),3aβ,7aα]]-Octahydro-7a-methyl-1-[1-methyl-5-(thiazol-2-yl)-5-[(triethylsilyl)oxy]pentyl]-4H-inden-4-on erhalten.

240. **[[1*R*-[1α(1*R**),3aβ,7aα]]-1-[5-[(Triethylsilyl)oxy]-1-methyl-5-(thiazol-2-yl)pentyl]octahydro-7a-methyl-1H-inden-4-yliden]essigsäureethylester**

Man stellt aus 0,7 ml Diisopropylamin und 2 ml *n*-Butyllithium (2,5 M in Hexan) in 14 ml Tetrahydrofuran Lithiumdiisopropylamid her, kühlt auf -78°C und tropft 0,95 ml Trimethylesigsäureethylester in 1ml Tetrahydrofuran zu. Nach 20 min. Rühren bei -78°C werden 584 mg [1*R*-[1α(1*R**),3aβ,7aα]]-Octahydro-7a-methyl-1-[1-methyl-5-(thiazol-2-yl)-5-[(triethylsilyl)oxy]pentyl]-4H-inden-4-on in 2ml Tetrahydrofuran zugegeben. Die Reaktionsmischung wird bei -78°C solange gerührt bis die Reaktion nach DC-Kontrolle (Essigester/Hexan 2:8) beendet ist. Nach Erwärmen auf Raumtemperatur, wird mit Natriumchlorid-Lösung gequencht und mit Essigester extrahiert, die organische Phase mit Natriumchlorid-Lösung gewaschen, über Natriumsulfat getrocknet und eingeengt. Der Rückstand wird mit Essigester/Hexan an Silicagel chromatographiert, wobei 700 mg [[1*R*-[1α(1*R**),3aβ,7aα]]-1-[5-[(Triethylsilyl)oxy]-1-methyl-5-(thiazol-2-yl)pentyl]octahydro-7a-methyl-1*H*-inden-4-yliden]essigsäureethylester erhalten werden.

241. **2-[[1*R*-[1α(1*R**),3aβ,7aα]]-1-[5-[(Triethylsilyl)oxy]-1-methyl-5-(thiazol-2- yl)pentyl]octahydro-7a-methyl-1H-inden-4-yliden]ethanol**

Es werden 700mg [[1*R*-[1α(1*R**),3aβ,7aα]]-1-[5-[(Triethylsilyl)oxy]-1-methyl-5-(thiazol-2-yl)pentyl]octahydro-7a-methyl-1H-inden-4-yliden]essigsäureethylester in 30 ml Tetrahydrofuran bei -20°C vorgelegt und 4,3 ml Diisobutylaluminiumhydridlösung zugetropft. Es wird 3 Stunden bei -20°C gerührt und dann langsam auf 0°C erwärmt, nochmals 0,6 ml Diisobutylalumiumhydridlösung zugegeben. Nach 1 Stunde Rühren bei 0°C wird Toluol zugegeben, mit 1,5 ml Isopropanol/Wasser (1:9) gequencht und über Celite abgesaugt. Der Rückstand wird eingeengt und über Kieselgel (Essigester/Hexan) chromatographiert. Es werden 385 mg 2-[[1*R*-[1α(1*R**),3aβ,7aα]]-1-[5-[(Triethylsilyl)oxy]-1-methyl-5-(thiazol-2-yl)pentyl]octahydro-7a-methyl-1 1H-inden-4-yliden]-ethanol erhalten.

242. **[2-[1*R*-[1α(1*R**),3aβ,7aα]]-1-[1-Methyl-5-(thiazol-2-yl)-5-[(Triethylsily-l)oxy]pentyl]octahydro-7a-methyl-1*H*-indenyliden]ethyl]diphenylphosphinoxid**

a) 385 mg des aus D erhaltenen Alkohols 2-[[1*R*-[1α(1*R**),3aβ,7aα]]-1-[5-[(Triethylsilyl)oxy]-1-methyl-5-(thiazol-2-yl)pentyl]octahydro-7a-methyl-1 1H-inden-4-yliden]-ethanol werden in 2,5 ml Tetrahydrofuran bei 0°C vorgelegt und bei 0°C tropfenweise mit 0,35 ml n-Butyllithium versetzt. Anschließend werden 166 mg Tosylchlorid in 0,5 ml Tetrahydrofuran zugetropft und mindestens 5 min. bei 0°C nachgerührt.

b) In einem zweiten Kolben werden 0,3 ml Diphenylphosophin in 2 ml Tetrahydrofuran bei 0°C vorgelegt. Bei Zutropfen von 0,7 ml n-Butyllithium färbt sich die Lösung orange.

c) Bei 0°C wird nun Lösung b) zu Lösung a) langsam zugetropft und 30 min. bei 0°C nachgerührt. Anschließend wird mit Wasser gequencht, die Lösung eingeengt und mit wenig Methylenchlorid aufgenommen. Nach Kühlen auf 0°C werden 0,3 ml 10%ige Wasserstoffperoxidlösung zugegeben und eine Stunde bei 0°C nachgerührt. Anschließend wird mit Natriumthiosulfatlösung gequencht, mit Natriumchloridlösung gewaschen und getrocknet. Es werden nach Säulenchromatographie 471 mg [2-[1*R*-[1α(1*R**),3aβ,7aα]]-1-[1-Methyl-5-(thiazol-2-yl)-5-[(Triethylsilyl)oxy]pentyl]octahydro-7a-methyl-1*H*-indenyliden]ethyl]diphenylphosphinoxid erhalten.

243. **(7*E*)-(1*R*,3*R*)-1,3-Bis-(benzoyloxy)-2-brom-24a-(thiazol-2-yl)-24a-[(Triethylsilyl)oxy]-24a-homo-19-nor-9,10-secochola-5,7-dien**

471 mg [2-[1*R*-[1α(1*R**),3aβ,7aα]]-1-[1-Methyl-5-(thiazol-2-yl)-5-[(Triethylsilyl)oxy]-pentyl]octahydro-7a-methyl-1*H*-indenyliden]ethyl]diphenylphosphinoxid werden in 7 ml Tetrahydrofuran vorgelegt und auf -78°C gekühlt. Bei dieser Temperatur werden 0,26 ml n-Butyllithium-Lösung zugegeben und 10 min bei -30°C nachgerührt. Anschließend werden 446 mg des aus Beispiel 57,Vorschrift 235 erhaltenen Ketons als Rohprodukt in 3 ml Tetrahydrofuran zugegeben und 5 Stunden bei -30°C nachgerührt (DC-Kontrolle Hexan/Essigester 6:4). Es wird mit Natriumchloridlösung gequencht, mit Essigester extrahiert, mit Natriumchloridlösung gewaschen, über Natriumsulfat bgetrocknet und eingeengt. Es werden 913 mg Rohprodukt (7E)-(1R, 3R)-1,3-Bis(benzoyloxy)-2-brom-24a-(thiazol-2-yl)-24a-[(Triethylsilyl)oxy]-24a-homo-19-nor-9,10-secochola-5,7-dien erhalten, das ohne Reinigung weiter umgesetzt wird.

**244. (7E)-(1R, 3R)-1,3-Bis-(benzoyloxy)-2-brom-24a-(thiazol-2-yl)- 24a-homo-19-nor-9,10-secochola-5,7-dien-24a-ol**

Es werden 913mg Rohprodukt aus Beispiel 58F in 20 ml Tetrahydrofuran vorgelegt und mit 878 mg Tetrabutylammoniumfluorid-Hydrat versetzt. Nach 2 Stunden Rühren bei Raumtemperatur wird Natriumhydrogencarbonatlösung zugegeben, mit Essigester extrahiert, die organische Phase mit Natriumchloridlösung gewaschen und über Natriumsulfat getrocknet. Nach Einengen wird der Rückstand als Rohprodukt weiter umgesetzt. Es werden 134 mg (7E) -(1R, 3R)-1,3-Bis-(benzoyloxy)-2-brom-24a-(thiazol-2-yl)- 24a-homo-19-nor-9,10-secochola-5,7-dien-24a-ol als Rohprodukt erhalten.

**245. (7E)-(1R,3R)-2-Brom-24a-(thiazol-2-yl)-24a-homo-19-nor-9,10-secochola-5,7-dien-1,3,24a-triol**

134 mg (7E)-(1R, 3R)-1,3-Bis-(benzoyloxy)-2-brom-24a-(thiazol-2-yl)- 24a-homo-19-nor-9,10-secochola-5,7-dien-24a-ol werden in 5ml Tetrahydrofuran bei 78°C vorgelegt, mit 0,44 ml DIBAH/Tetrahydrofuran versetzt, auf 0°C erwärmen gelassen und nach nochmaliger Zugabe von 3ml DIBAH nachgerührt. Es wird mit Natriumchlorid gequencht, mit Essigester extrahiert, über Natriumsulfat getrocknet und eingeengt. Das Rohprodukt wird über Silicagel chromatographiert. Es werden 24 mg (7E)-(1R,3R)-2-Brom-24a-(thiazol-2-yl)-24a-homo-19-nor-9,10-secochola-5,7-dien-1,3,24a-triol erhalten.

**Beispiel 60**

**(7E)-(1R,3R)-2-Brom-19-nor-9,10-secocholesta-5,7-dien-1,3,25-triol**

246. **[[1*R*-[1α(1*R**),3aβ,7aα]]-1-[1,5,5-trimethyl-5-[(trimethylsilyl)oxy]pentyl]octahydro-7a-methyl-1*H*-inden-4-yliden]essigsäureethylester**

1,0 g der literaturbekannten Verbindung [[1*R*-[1α(1*R**),3aβ,7aα]]-1-[1,5,5-trimethyl-5-[(trimethylsilyl)oxy]pentyl]octahydro-7a-methyl-4*H*-inden-4-on werden analog zu Beispiel 59, Vorschrift 240 umgesetzt und 1,03 g [[1*R*-[1α(1*R**),3aβ,7aα]]-1-[1,5,5-trimethyl-5-[(trimethylsilyl)oxy]pentyl]octahydro-7a-methyl-1*H*-inden-4-yliden]essigsäureethylester erhalten.
$^1$H-NMR ($CDCl_3$): δ = 0,1 ppm (s, 9H); 0,59 ppm (s, 3H); 0,95 (d, 3H); 1,19 (s, 6H); 1,29 (t, 3H); 4,1 (q, 2H); 5,45 (s, 1H)

247. 2-[[1*R*-[1α(1*R**),3aβ,7aα]]-1-[1,5,5-trimethyl-5-[(trimethylsilyl)oxy]pentyl]-**octahydro-7a-methyl**-1*H*-inden**-4-yliden]ethanol**

1,03 g der aus Beispiel 60, Vorschrift 246 erhaltenen Verbindung werden analog Beispiel 59, Vorschrift 241 umgesetzt und 535 mg 2-[[1R-[1α(1R*),3aβ,7aα]]-1-[1,5,5-trimethyl-5-[(trimethylsilyl)oxy]pentyl]octahydro-7a-methyl-1*H*-inden-4-yliden]ethanol erhalten.
$^1$H-NMR ($CDCl_3$): δ = 0,1 ppm (s, 9H); 0,57 (s, 3H); 0,91 (d, 3H); 1,2 (s, 6H); 4,21 (d, 2H); 5,23 (t, 1H)

248. **[1*R*-[1α(1*R**),3aβ,7aα]]-1-[5-[(Triethylsilyl)oxy]-1,5,5-trimethyl-5-(trimethylsilyloxy)pentyl]octahydro-7a-methyl-1*H*-indenyliden]ethyl]diphenylphosphinoxid**

535 mg 2-[[1*R*-[1α(1*R**),3aβ,7aα]]-1-[1,5,5-trimethyl-5-[(trimethylsilyl)oxy]pentyl]-octahydro-7a-methyl-1*H*-inden-4-yliden]ethanol werden analog zu Beispiel 59, Vorschrift 242 umgesetzt und 318 mg [1*R*-[1α(1*R**),3aβ,7aα]]-1-[5-[(Triethylsilyl)oxy]-1,5,5-trimethyl-5-(trimethylsilyloxy)pentyl]octahydro-7a-methyl-1*H*-indenyliden]ethyl]-diphenylphosphinoxid erhalten.
$^1$H-NMR ($CDCl_3$): δ = 0,1 ppm (s, 9H); 0,3 (s, 3H); 0,88 (d, 3H); 1,2 (s, 6H); 3,05-3,34 (m, 2H); 5,0 (m, 1H); 7,45 (m, 6H); 7,73 (m, 4H)

249. **(7E)-(1R,3R)-1,3-Bis(benzoyloxy)-2-brom-25-[(trimethylsilyl)oxy]-19-nor-9,10-secocholesta-5,7-dien**

Aus 210 mg [1*R*-[1α(1*R**),3aβ,7aα]]-1-[5-[(Triethylsilyl)oxy]-1,5,5-trimethyl-5-(trimethylsilyloxy)pentyl]octahydro-7a-methyl-1*H*-indenyliden]ethyl]diphenylphosphinoxid werden analog Beispiel 59, Vorschrift 243 445 mg (7E) -(1R,3R)-1,3-Bis(benzoyloxy)-2-brom-25-[(trimethylsilyl)oxy]-19-nor-9,10-secocholesta-5,7-dien erhalten.
$^1$H-NMR ($CDCl_3$): δ = 0,1 ppm (s, 9H); 0,24 (s, 3H); 0,89 (d, 3H); 1,18 (s, 6H)

250. **(7E)-(1R,3R)-1,3-Bis(benzoyloxy)-2-brom-19-nor-9,10-secocholesta-5,7-dien-25-ol**

Aus 445 mg (7E)-(1R,3R)-1,3-Bis(benzoyloxy)-2-brom-25-[(trimethylsilyl)oxy]-19-nor-9,10-secocholesta-5,7-dien werden analog Beispiel 59, Vorschrift 244 170 mg (7E)-(1R,3R)- 1,3-Bis(benzoyloxy)-2-brom-19-nor-9,10-secocholesta-5,7-dien-25-ol erhalten, die ohne Reinigung weiter umgesetzt werden.

251. **(7E)-(1R,3R)-2-Brom-19-nor-9,10-secocholesta-5,7-dien-1,3,25-triol**

Aus 151 mg (7E)-(1R,3R)-1,3-Bis(benzoyloxy)-2-brom-25-[(trimethylsilyl)oxy]-19-nor-9,10-secocholesta-5,7-dien werden analog zu Beispiel 59, Vorschrift 245 35 mg (7E)-(1R,3R)-2-Brom-19-nor-9,10-secocholesta-5,7-dien-1,3,25-triol erhalten.
$^1$H-NMR ($CDCl_3$): δ= 0,52 ppm (s, 3H);0,92 (d, 3H); 1,14 (s, 6H); 3,8 (d, 1H); 3,95-4,08 (2x m, 2H); 4,19 (dd, 1H); 6,27 (d, 1H)

**Patentansprüche**

**1.** Verfahren zur Herstellung der Verbindungen der allgemeinen Formel Iia

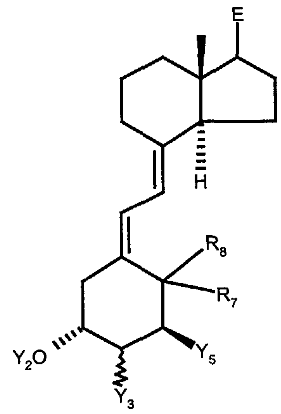

**IIa**

worin
E eine beliebige Seitenkette bedeutet,

$R_7$, $R_8$ unabhängig voneinander ein Wasserstoffatom, eine Methylgruppe, oder gemeinsam eine exocyclische Methylengruppe oder einen Cyclopropylring bedeuten,

$Y_2$ ein Wasserstoffatom oder eine Gruppe -(CO)$R_5$ bedeutet,

$Y_3$ ein Wasserstoffatom oder eine Hydroxygruppe, ein Halogenatom, eine Gruppe -O(CO)$R_5$ oder eine $OR_5$-Gruppe bedeutet,
wobei

$R_5$ für einen aliphatischen $C_1$-$C_{12}$ Alkylrest, der gegebenenfalls unterbrochen ist von 1-2 Sauerstoffatomen, 1-2 Schwefelatomen und/oder 1-2 NH-Gruppen und/oder gegebenenfalls substituiert ist durch 1-2 Hydroxygruppen, 1-2 Aminogruppen, 1-2 SH-Gruppen, 1-2 COOH-Gruppen und/oder 1-2 Phenylgruppen, oder für einen aromatischen Rest mit 5 bis 12-C-Atomen steht,

$Y_5$ ein Fluoratom, eine $(CH_2)_n$-OH Gruppe oder eine $(CH_2)_n$-O(CO)$R_5$Gruppe bedeutet,
wobei n = 0 bis 4 bedeutet.

und gegebenenfalls vorhandene Hydroxygruppen gegebenenfalls geschützt vorliegen,
**dadurch gekennzeichnet, daß**
ein Keton der allgemeinen Formel XIIIc

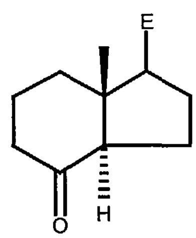

**XIIIc**

worin
E eine beliebige Seitenkette bedeutet,
und gegebenenfalls vorhandene Ketogruppen und/oder Hydroxygruppen geschützt vorliegen,
durch Umsetzung mit Trimethylsilylessigester in Gegenwart einer Base wie z.B. n-Butyllitium oder Lithiumaluminiumhydrid oder mit einem geeigneten Wittigreagenz in einem aprotischen Lösungsmittel wie Toluol, Tetrahydrofuran, Diethylether oder Dioxan in eine Verbindung der allgemeinen Formel XXXIVc

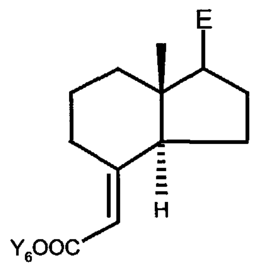

**XXXIVc**

worin

$Y_6$ eine $C_1$-$C_6$-Alkylgruppe, eine Benzylgruppe, oder eine Phenylgruppe bedeutet, überführt,

die Estergruppe durch Reaktion mit einem Reduktionsmittel wie Dibah, Lithiumaluminiumhydrid, Diboran oder RedAl in Hexan, Toluol, Tetrahydrofuran, Diethylether oder Dioxan in den Allylalkohol der allgemeinen Formel XXXVc

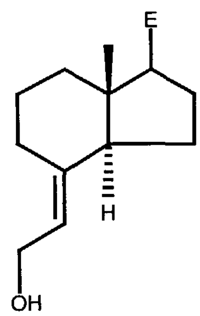

**XXXVc**

überführt,

den Allylalkohol in an sich bekannter Weise in eine Verbindung der allgemeinen Formel XXXVc

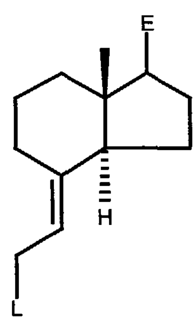

**XXXVc**

worin

L jede beliebige Abgangsgruppe bedeutet (Halogen, Mesylat, Tosylat, Triflat, Nonaflat), überführt,

die isoliert oder gegebenenfalls in situ erzeugt und sofort weiter umgesetzt wird zu einem Wittig Reagenz der allgemeinen Formel XXXVIc,

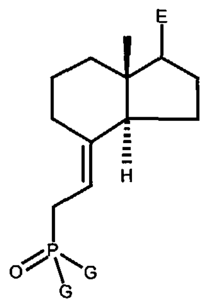

XXXVIc

worin
G eine $C_1$-$C_{10}$-Alkylgruppe, eine $C_1$-$C_{10}$ Alkoxygruppe, einen Phenylrest oder einen Phenoxyrest bedeutet, das dann anschließend unter bekannten Bedingungen mit einem Keton der allgemeinen Formel XXXIIIc

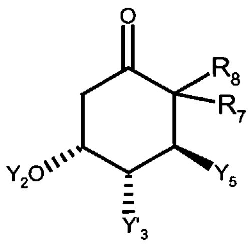

XXXIIIc

worin
$Y'_3$ ein Wasserstoffatom, ein Halogenatom oder eine geschützte Hydroxygruppe , eine Gruppe —O(CO)$R_5$ oder eine $OR_5$-Gruppe bedeutet, und
$Y_5$, $R_7$ und $R_8$ die oben angegebene Bedeutung haben,
umgesetzt wird und falls gewünscht, Schutzgruppen abgespalten werden.

**2.** Zwischenprodukte des Verfahrens gemäß Anspruch 8 der allgemeinen Formeln XXXIVc,

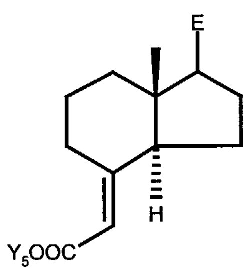

XXXIVc

XXXVc

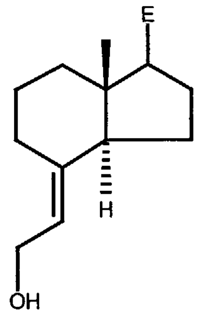

**XXXVc**

und XXXVIc

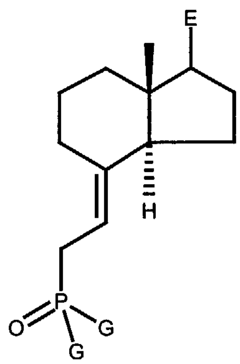

**XXXVIc**

worin E und G die oben angegebenen Bedeutungen haben.

Europäisches Patentamt

# EUROPÄISCHER RECHERCHENBERICHT

Nummer der Anmeldung
EP 03 09 0212

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | HATAKEYAMA, S. ET AL.: "A Novel Convergent Synthesis of (+)-1alpha,25-Dihyroxyvitamin D3 Using a Chromium(II)-Mediated Coupling Reaction" JOURNAL OF ORGANIC CHEMISTRY, Bd. 56, Nr. 1, 1991, Seiten 461-463, XP000973200 * Seite 462; Abbildung I; Beispiel 10 * ----- | 2 | C07C401/00 |
| X | DURAISAMY, M. & WALBORSKY, H.M.: "Circular Dichroism of Isomeric 10-19-Dihydrovitamin D1" JOURNAL OF THE AMERICAN CHEMICAL SOCIETY, Bd. 105, Nr. 10, 1983, Seiten 3270-3273, XP000973256 * Seite 3271, Spalte 1, Zeile 8 - Zeile 9; Abbildung II; Beispiele 11,12 * ----- | 2 | |
| P,X | DODO, K. ET AL.: "Synthesis of a Novel Class of cdc25A Inhibitors from Vitamin D3" BIOORGANIC & MEDICINAL CHEMISTRY LETTERS, Bd. 10, Nr. 7, 2000, Seiten 615-617, XP004191838 * Seite 616; Abbildung 1; Beispiel 3A * ----- | 2 | RECHERCHIERTE SACHGEBIETE (Int.Cl.7) C07C |
| A | EP 0 717 034 A (DUPHAR INT RES) 19. Juni 1996 (1996-06-19) * Seite 5 * ----- | 1 | |
| A | WO 97 41096 A (WIESINGER HERBERT ;KIRSCH GERALD (DE); SCHERING AG (DE); HABEREY M) 6. November 1997 (1997-11-06) * Anspruch 1 * ----- | 1 | |
| A | EP 0 854 138 A (TEIJIN LTD) 22. Juli 1998 (1998-07-22) * Seite 5, Zeile 35 - Zeile 45 * ----- -/-- | 1 | |

Der vorliegende Recherchenbericht wurde für alle Patentansprüche erstellt

| Recherchenort | Abschlußdatum der Recherche | Prüfer |
|---|---|---|
| München | 9. September 2003 | Janus, S. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches Patentamt

# EUROPÄISCHER RECHERCHENBERICHT

Nummer der Anmeldung
EP 03 09 0212

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
| A | WO 98 47866 A (HOFFMANN LA ROCHE) 29. Oktober 1998 (1998-10-29) * Zusammenfassung * ----- | 1 | |
| A | EP 0 927 721 A (SCHERING AG) 7. Juli 1999 (1999-07-07) * Anspruch 1 * ----- | 1 | |
| | | | RECHERCHIERTE SACHGEBIETE (Int.Cl.7) |

Der vorliegende Recherchenbericht wurde für alle Patentansprüche erstellt

| Recherchenort | Abschlußdatum der Recherche | Prüfer |
|---|---|---|
| München | 9. September 2003 | Janus, S. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 03 09 0212

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

09-09-2003

| Im Recherchenbericht angeführtes Patentdokument | Datum der Veröffentlichung | Mitglied(er) der Patentfamilie | Datum der Veröffentlichung |
|---|---|---|---|
| EP 0717034 A | 19-06-1996 | EP 0717034 A1 | 19-06-1996 |
| | | AT 178596 T | 15-04-1999 |
| | | CA 2164916 A1 | 15-06-1996 |
| | | DE 69508895 D1 | 12-05-1999 |
| | | DE 69508895 T2 | 21-10-1999 |
| | | DK 717034 T3 | 18-10-1999 |
| | | ES 2130522 T3 | 01-07-1999 |
| | | IL 116326 A | 13-08-2000 |
| | | JP 8319268 A | 03-12-1996 |
| | | US 5929056 A | 27-07-1999 |
| WO 9741096 A | 06-11-1997 | DE 19619036 A1 | 13-11-1997 |
| | | AT 234280 T | 15-03-2003 |
| | | AU 730394 B2 | 08-03-2001 |
| | | AU 2766697 A | 19-11-1997 |
| | | CN 1216978 A | 19-05-1999 |
| | | CZ 9803465 A3 | 17-02-1999 |
| | | DE 59709509 D1 | 17-04-2003 |
| | | DK 900198 T3 | 23-06-2003 |
| | | WO 9741096 A1 | 06-11-1997 |
| | | EP 0900198 A1 | 10-03-1999 |
| | | HU 9901534 A2 | 30-08-1999 |
| | | JP 2000510826 T | 22-08-2000 |
| | | NO 985038 A | 23-12-1998 |
| | | NZ 332488 A | 27-03-2000 |
| | | PL 329597 A1 | 29-03-1999 |
| | | PT 900198 T | 30-06-2003 |
| | | SK 146498 A3 | 13-04-1999 |
| | | US 2002049344 A1 | 25-04-2002 |
| | | US 6600058 B1 | 29-07-2003 |
| | | ZA 9703757 A | 20-08-1998 |
| EP 0854138 A | 22-07-1998 | EP 0854138 A2 | 22-07-1998 |
| | | EP 0854139 A2 | 22-07-1998 |
| | | AT 186721 T | 15-12-1999 |
| | | AT 201397 T | 15-06-2001 |
| | | AT 205190 T | 15-09-2001 |
| | | CA 2168728 A1 | 14-12-1995 |
| | | DE 69513367 D1 | 23-12-1999 |
| | | DE 69513367 T2 | 08-06-2000 |
| | | DE 69521065 D1 | 28-06-2001 |
| | | DE 69521065 T2 | 25-10-2001 |
| | | DE 69522589 D1 | 11-10-2001 |
| | | DE 69522589 T2 | 18-04-2002 |
| | | EP 0712843 A1 | 22-05-1996 |
| | | ES 2138212 T3 | 01-01-2000 |

EPO FORM P0461

Für nähere Einzelheiten zu diesem Anhang : siehe Amtsblatt des Europäischen Patentamts, Nr.12/82

## ANHANG ZUM EUROPÄISCHEN RECHERCHENBERICHT ÜBER DIE EUROPÄISCHE PATENTANMELDUNG NR. EP 03 09 0212

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

09-09-2003

| Im Recherchenbericht angeführtes Patentdokument | Datum der Veröffentlichung | Mitglied(er) der Patentfamilie | Datum der Veröffentlichung |
|---|---|---|---|
| EP 0854138 A | | WO 9533716 A1 | 14-12-1995 |
| | | JP 8053411 A | 27-02-1996 |
| | | US 6177586 B1 | 23-01-2001 |
| | | US 5719297 A | 17-02-1998 |
| | | US 5986112 A | 16-11-1999 |
| | | JP 8134052 A | 28-05-1996 |
| | | JP 8143541 A | 04-06-1996 |
| WO 9847866 A | 29-10-1998 | AU 7523798 A | 13-11-1998 |
| | | BR 9809266 A | 27-06-2000 |
| | | CN 1252791 T | 10-05-2000 |
| | | WO 9847866 A2 | 29-10-1998 |
| | | EP 0977734 A2 | 09-02-2000 |
| | | HR 980214 A1 | 30-04-1999 |
| | | JP 2000511937 T | 12-09-2000 |
| | | TR 9902487 T2 | 22-01-2001 |
| | | US 6153605 A | 28-11-2000 |
| | | ZA 9803116 A | 21-10-1998 |
| EP 0927721 A | 07-07-1999 | EP 0927721 A1 | 07-07-1999 |
| | | AU 2413499 A | 05-07-1999 |
| | | WO 9931112 A1 | 24-06-1999 |
| | | EP 1042335 A1 | 11-10-2000 |
| | | JP 2002508383 T | 19-03-2002 |
| | | US 6531459 B1 | 11-03-2003 |

EPO FORM P0461

Für nähere Einzelheiten zu diesem Anhang : siehe Amtsblatt des Europäischen Patentamts, Nr.12/82